# EUROPEAN PATENT APPLICATION

(11) **EP 4 822 018 A1**
(43) Date of publication of application: **14.10.2026**
(21) Application number: 25169382.6
(22) Date of filing: 09.04.2025
(51) Int. Cl.: C12N 9/04, C12P 7/24

(54) **PERIPLASMIC OXIDATION OF 5-HYDROXYMETHYL-2-FUROIC ACID INTO 5-FORMYL-2-FUROIC ACID AND/OR CONVERTING 5-FORMYL-2-FUROIC ACID INTO 2,5 FURANDICARBOXYLIC ACID USING ALCOHOL OXIDOREDUCTASE MUTANT VARIANTS**

(71) Applicant: Forschungszentrum Jülich GmbH, 52428 Jülich (DE); Philipps-Universität Marburg, 35032 Marburg (DE); Max-Planck-Gesellschaft zur Förderung der Wissenschaften e.V., 80539 München (DE)
(72) Inventor: Wierckx, Nick Johannes Petrus, 6268NV Bemelen (NL); Lechtenberg, Thorsten, 40221 Düsseldorf (DE); Wynands, Benedikt Niklas Daniel, 50859 Köln (DE); Elsayed, Eslam Mohsen Ahmed Gabr, 35039 Marburg (DE); Bange, Gert, 35043 Marburg (DE); Lepak, Alexander, 35043 Marburg (DE)
(74) Representative: Schiweck Weinzierl Koch Patentanwälte Partnerschaft mbB

(57) **Abstract**

The present invention provides a alcohol oxidoreductase polypeptide converting 5-hydroxymethyl-2-furoic acid (HMFA) to 5-formyl-2-furoic acid (FFA) with a Km value of less than 15 mM selected from the group consisting of a polypeptide having the amino acid sequence shown in SEQ ID NO: 1, wherein (a) in said polypeptide amino acids at positions F412, F459 and W561 are V, W or R and A, respectively; or (d) in said polypeptide amino acids at positions F412, L560 and W561 are V, R or W and A, respectively; or (g) in said polypeptide amino acids at positions F412, L455 and W561 are V, W or R and A, respectively; preferably in the polypeptide (a) or (d) amino acids at positions F412, F459, L560 and W561 are V, W or R, R or W and A, respectively and this polypeptide converts FFA to 2,5 furandicarboxylic acid (FDCA).

## Description

### TECHNICAL FIELD OF THE INVENTION

The invention provides a alcohol oxidoreductase polypeptide converting 5-hydroxymethyl-2-furoic acid (HMFA) to 5-formyl-2-furoic acid (FFA) with a Km value of less than 15 mM selected from the group consisting of (a) a polypeptide having the amino acid sequence shown in SEQ ID NO: 1, wherein in said polypeptide amino acids at positions corresponding to positions F412, F459 and W561 are V, W or R and A, respectively; optionally having the amino acid sequence shown in SEQ ID NO: 3, 4, 5 or 6; (b) a polypeptide fragment of the polypeptide of (a), wherein in said polypeptide fragment amino acids at positions corresponding to positions F412, F459 and W561 are V, W or R and A, respectively; (c) a polypeptide having an amino acid sequence which is at least 70% identical to the amino acid sequence of the polypeptide of (a) or (b), wherein in said polypeptide amino acids at positions corresponding to positions F412, F459 and W561 are V, W or R and A, respectively; (d) a polypeptide having the amino acid sequence shown in SEQ ID NO: 1, wherein in said polypeptide amino acids at positions corresponding to positions F412, L560 and W561 are V, R or W and A, respectively; optionally having the amino acid sequence shown in SEQ ID NO: 7, 8, 9, or 10; (e) a polypeptide fragment of the polypeptide of (d), wherein in said polypeptide fragment amino acids at positions corresponding to positions F412, L560 and W561 are V, R or W and A, respectively; (f) a polypeptide having an amino acid sequence which is at least 70% identical to the amino acid sequence of the polypeptide of (d) or (e), wherein in said polypeptide amino acids at positions corresponding to positions F412, L560 and W561 are V, R or W and A, respectively; (g) a polypeptide having the amino acid sequence shown in SEQ ID NO: 1, wherein in said polypeptide amino acids at positions corresponding to positions F412, L455 and W561 are V, W or R and A, respectively; optionally having the amino acid sequence shown in SEQ ID NO: 11, 12, 13, or 14; (h) a polypeptide fragment of the polypeptide of (g), wherein in said polypeptide fragment amino acids at positions corresponding to positions F412, L455 and W561 are V, W or R and A, respectively; (i) a polypeptide having an amino acid sequence which is at least 70% identical to the amino acid sequence of the polypeptide of (g) or (h), wherein in said polypeptide amino acids at positions corresponding to positions F412, L455 and W561 are V, W or R and A, respectively. In one embodiment in said polypeptides or polypeptide fragments (a) to (f) amino acids at positions corresponding to positions F412, F459, L560 and W561 are V, W or R, R or W and A, respectively, said polypeptide or polypeptide fragment further converting FFA to 2,5 furandicarboxylic acid (FDCA). In one embodiment in said polypeptides or polypeptide fragments (g) to (i) amino acids at positions corresponding to positions F412, L455, L560 and W561 are V, W or R, R or W and A, respectively, said polypeptide or polypeptide fragment further converting FFA to FDCA. Further aspects of the invention are directed to polynucleotides encoding the polypeptides mentioned above, vectors comprising these polynucleotides, host cells comprising the vector or the polynucleotide and uses of the host cells to produce FFA from HMFA and/or producing FDCA from FFA.

### BACKGROUND

Plastics have firmly integrated into modern society, yet there is a pressing need to transition their production to renewable resources and sustainable processes (1). This requires the replacement of building blocks based on petroleum with comparable biomass-derived chemicals, such as 2,5-furandicarboxylic acid (FDCA), which has the potential to replace terephthalic acid to give rise to the PET alternative polyethylene furanoate (PEF) (2). Using bio-sourced ethylene glycol as the second monomer results in a fully bio-based material that even features improved properties like a higher gas diffusion barrier, higher tensile strength, and a higher glass transition temperature (3, 4). Given its role as promising polymer building block, FDCA is ranked as one of 12 priority chemicals for establishing a future green chemistry (5). The dicarboxylic acid can be synthesized through the oxidation of the sugar-based precursor 5-(hydroxymethyl) furfural (HMF), either homo- or heterogeneously catalyzed by (noble) metals, or biocatalytically (6). While chemical synthesis still needs elevated temperatures and pressures causing a high energy demand, enzyme-based processes operate at very mild conditions and are considered attractive sustainable alternatives (7). Accordingly, the number of published biocatalytic routes, either founded on whole cells or on isolated enzymes leading to FDCA is rapidly increasing (8,9-11). Particularly concerning redox-reactions, whole cells offer the advantage of efficiently regenerating necessary cofactors and allowing for multi-step reactions in a single strain (12). However, FDCA synthesis comprises highly reactive aldehydes which are toxic to living systems, necessitating the use of robust microorganisms such as Pseudomonads as production hosts (13).

Whole-cell biocatalytic FDCA production with *Pseudomonas* is based on the organism's innate ability to efficiently oxidize both the substrate aldehyde HMF and the intermediate 5-formyl-2-furoic acid (FFA). These reactions predominantly involve the periplasmic aldehyde oxidoreductase PaoEFG, as well as the two cytoplasmic aldehyde dehydrogenases AldB-I and AldB-II (14). In order to produce FDCA, a gene encoding an additional oxidoreductase, typically *hmfH,* must be expressed heterologous, enabling the transformation from 5-hydroxymethyl-2-furoic acid (HMFA) to FFA (9, 15). This has two major disadvantages: First, the enzyme belongs to the glucose-methanol-choline (GMC) oxidoreductase protein family and uses FAD as a redox cofactor leading to the production of stoichiometric amounts of toxic H₂O₂ upon oxidation by molecular oxygen (16, 17). Although *Pseudomonas* exhibits a notable resilience to oxidative stress, the constant high-rate production of peroxide will inevitably cause cellular damage (18). Furthermore, the availability of oxygen can be a limiting factor in high-density cell cultures and low intracellular oxygen level can affect enzyme kinetics (19). Second, HmfH is a cytoplasmic enzyme and its substrate HMFA needs to be transported from the periplasm, where it is primarily formed, into the cell. The cell membrane as a mass transport barrier is a main drawback of whole-cell biocatalysis (20). Despite the additional implementation of a transporter, HmfT, cellular uptake of HMFA was recently shown to be rate-limiting in aldehyde oxidation-optimized BOX mutants (14).

Theoretically, these issues could be bypassed by a completely periplasmic production route from HMF to FDCA. For this, a substitute for HmfH catalyzing oxidation of the primary alcohol of HMFA in the periplasm is required. *P. putida* KT2440 possesses an interesting set of pyrroloquinoline quinone (PQQ)-dependent alcohol dehydrogenases (ADHs) encoded in the ped cluster allowing metabolism of volatile alcoholic compounds like 2-phenylethanol without formation of hydrogen peroxide (21, 22). Instead the electrons released by the oxidation reaction are conveyed via cytochrome c to the respiratory chain conserving electrochemical energy in the form of the proton motive force (23). Influenced by the availability of metal ions, the bacterium can switch between a calcium-dependent dehydrogenase PedE and a preferred lanthanide-dependent isoenzyme PedH which benefits from a higher Lewis acidity of Ln³⁺ ions compared to Ca²⁺ (21). Several rare earth elements (REEs) are accepted as cofactor, but the early elements of the class such as lanthanum (La³⁺), praseodymium (Pr³⁺) or neodymium (Nd³⁺) are favoured (21). However, neither PedE nor PedH naturally display activity for HMFA. This was addressed by Wehrmann *et al.* through protein engineering resulting in the improved variant PedH^{F412V/W561A}. Supported by structural analyses, it was shown that replacement of F412 and W561 by smaller amino acids widens and opens the substrate channel of the enzyme enabling bulkier substrates like HMFA to access the active site (24). Obtaining any level of activity with HMFA was a significant achievement, but an *in vivo* whole-cell conversion could not be shown, likely due to the high Kₘ value of PedH^{F412V/W561A} (18.8 ± 2.6 mM) (24). However, the double mutant of Wehrmann *et al.* is not active *in vivo.* Since the HMFA concentration in the periplasm would be lower, enzymes with a lower km would be needed for using a variant PedH *in vivo.*

Thus, PedH variants are needed which are active in vivo, have a better Kₘ value and which could enable production of FDCA entirely in the periplasm of *Pseudomonas.*

### SUMMARY OF INVENTION

The present invention provides, in a first aspect, alcohol oxidoreductase polypeptide converting 5-hydroxymethyl-2-furoic acid (HMFA) to 5-formyl-2-furoic acid (FFA) with a Km value of less than 15 mM selected from the group consisting of
(a) a polypeptide having the amino acid sequence shown in SEQ ID NO: 1, wherein in said polypeptide amino acids at positions corresponding to positions F412, F459 and W561 are V, W or R and A, respectively;
(b) a polypeptide fragment of the polypeptide of (a), wherein in said polypeptide fragment amino acids at positions corresponding to positions F412, F459 and W561 are V, W or R and A, respectively;
(c) a polypeptide having an amino acid sequence which is at least 70% identical to the amino acid sequence of the polypeptide of (a) or (b), wherein in said polypeptide amino acids at positions corresponding to positions F412, F459 and W561 are V, W or R and A, respectively;
(d) a polypeptide having the amino acid sequence shown in SEQ ID NO: 1, wherein in said polypeptide amino acids at positions corresponding to positions F412, L560 and W561 are V, R or W and A, respectively;
(e) a polypeptide fragment of the polypeptide of (d), wherein in said polypeptide fragment amino acids at positions corresponding to positions F412, L560 and W561 are V, R or W and A, respectively;
(f) a polypeptide having an amino acid sequence which is at least 70% identical to the amino acid sequence of the polypeptide of (d) or (e), wherein in said polypeptide amino acids at positions corresponding to positions F412, L560 and W561 are V, R or W and A, respectively;
(g) a polypeptide having the amino acid sequence shown in SEQ ID NO: 1, wherein in said polypeptide amino acids at positions corresponding to positions F412, L455 and W561 are V, W or R and A, respectively;
(h) a polypeptide fragment of the polypeptide of (g), wherein in said polypeptide fragment amino acids at positions corresponding to positions F412, L455 and W561 are V, W or R and A, respectively;
(i) a polypeptide having an amino acid sequence which is at least 70% identical to the amino acid sequence of the polypeptide of (g) or (h), wherein in said polypeptide amino acids at positions corresponding to positions F412, L455 and W561 are V, W or R and A, respectively.

In one embodiment, said alcohol oxidoreductase is a alcohol dehydrogenase, preferably the alcohol dehydrogenase is a co factor dependent alcohol dehydrogenase, wherein the co factor is a co factor located in the periplasm, preferably the co factor is cytochrome C, pyrroloquinoline quinone (PQQ), or a quinone similar to PQQ, more preferably the co factor dependent alcohol dehydrogenase is the pyrroloquinoline quinone (PQQ) dependent alcohol dehydrogenase, most preferably the alcohol oxidoreductase is PedE or PedH, exceptionally preferred is PedH.

In one embodiment, in said polypeptide or polypeptide fragment amino acids of (a) to (f) of the first aspect at positions corresponding to positions F412, F459, L560 and W561 are V, W or R, R or W and A, respectively, said polypeptide or polypeptide fragment further converting FFA to 2,5 furandicarboxylic acid (FDCA).

In one embodiment of the previous embodiment said polypeptide is selected from the group consisting of
(a) a polypeptide having the amino acid sequence shown in SEQ ID NO: 1, wherein in said polypeptide amino acids at positions corresponding to positions F412, F459, L560 and W561 are V, W or R, R or W and A, respectively;
(b) a polypeptide fragment of the polypeptide of (a), wherein in said polypeptide fragment amino acids at positions corresponding to positions F412, F459, L560 and W561 are V, W or R, R or W, and A, respectively;
(c) a polypeptide having an amino acid sequence which is at least 70% identical to the amino acid sequence of the polypeptide of (a) or (b), wherein in said polypeptide amino acids at positions corresponding to positions F412, F459, L560 and W561 are V, W or R, R or W and A, respectively.

In one embodiment, the polypeptide (a) of the embodiment before has the amino acid sequence shown in SEQ ID NO: 15, 16, 17, 18, 19, 20, 21, or 22.

In one embodiment of the first aspect, in the polypeptide or polypeptide fragment of (g) to (i) of the first aspect amino acids at positions corresponding to positions F412, L455, L560 and W561 are V, W or R, R or W and A, respectively, said polypeptide or polypeptide fragment further converting FFA to FDCA.

In one embodiment of the embodiment before, said polypeptide is selected from the group consisting of
(a) a polypeptide having the amino acid sequence shown in SEQ ID NO: 1, wherein in said polypeptide amino acids at positions corresponding to positions F412, L455, L560 and W561 are V, W or R, R or W and A, respectively;
(b) a polypeptide fragment of the polypeptide of (a), wherein in said polypeptide fragment amino acids at positions corresponding to positions F412, L455, L560 and W561 are V, W or R, R or W, and A, respectively;
(c) a polypeptide having an amino acid sequence which is at least 70% identical to the amino acid sequence of the polypeptide of (a) or b), wherein in said polypeptide amino acids at positions corresponding to positions F412, L455, L560 and W561 are V, W or R, R or W and A, respectively.

In one embodiment, the polypeptide (a) of the embodiment before has the amino acid sequence shown in SEQ ID NO: 23, 24, 25, 26, 27, 28, 29, and 30.

In one embodiment, the polypeptide (a) of the first aspect has the amino acid sequence shown in SEQ ID NO : 3, 4, 5 or 6.

In one embodiment, the polypeptide (d) of the first aspect has the amino acid sequence shown in SEQ ID NO: 7, 8, 9, or 10.

In one embodiment, the polypeptide (g) of the first aspect has the amino acid sequence shown in SEQ ID NO: 11, 12, 13, or 14.

In a second aspect, the invention provides a polynucleotide encoding any one of the polypeptides of the first aspect.

In one embodiment, the polynucleotide has the nucleotide sequence shown in SEQ ID NO: 35, 36, 37, 38, 39, 40, 41, 42, 43, 44, 45, 46, 47, 48, 49, 50, 51, 52, 53, 54, 55, 56, 57, 58, 59, 60, 61, or 62.

In a third aspect, the invention provides a vector comprising one of the polynucleotides of the second aspect.

In one embodiment, the said vector is an expression vector.

In a fourth aspect, the invention provides a host cell comprising one of the polynucleotides of the second aspect or the vector of the third aspect.

In one embodiment, said host cell is a gram-negative bacterial host cell.

In one embodiment, said gram-negative bacterial host cell is a *Pseudomonas* species, preferably *Pseudomonas putida.*

In one further embodiment, said host cell lacks a functional hmft1 gene or a functional hmfH gene.

In a fifth aspect, the invention provides a method for converting HMFA to FFA, comprising culturing the host cell of the fourth aspect expressing the polypeptide as defined the first aspect.

In one embodiment, FFA is further converted to FDCA by the host cell of the fourth aspect expressing the polypeptide of the embodiment of the first aspect, in which in the polypeptide or polypeptide fragment amino acids of (a) to (f) of the first aspect at positions corresponding to positions F412, F459, L560 and W561 are V, W or R, R or W and A, respectively, or in which in the polypeptide or polypeptide fragment of (g) to (i) of the first aspect amino acids at positions corresponding to positions F412, L455, L560 and W561 are V, W or R, R or W and A, respectively.

In one embodiment, said host cell is further capable of converting 5-(hydroxymethyl)furfural (HMF) to HMFA.

In one embodiment, the conversion takes place in the periplasm of said host cell.

In a sixth aspect, the invention provides a method for the production of FDCA, comprising culturing a host cell expressing a polypeptide of the embodiment of the first aspect, in which in the polypeptide or polypeptide fragment amino acids of (a) to (f) of the first aspect at positions corresponding to positions F412, F459, L560 and W561 are V, W or R, R or W and A, respectively, or in which in the polypeptide or polypeptide fragment of (g) to (i) of the first aspect amino acids at positions corresponding to positions F412, L455, L560 and W561 are V, W or R, R or W and A, respectively.

In one embodiment, the conversion takes place in the periplasm of said host cell.

In a seventh aspect, the invention provides a use of the host cell of the fourth aspect for converting HMFA to FFA, said host cell expressing the polypeptide as defined in the first aspect.

In one embodiment, said host cell is further capable of converting HMF to HMFA.

In a eight aspect, the invention provides a use of the host cell of the fourth aspect for converting FFA to FDCA, said host cell expressing the polypeptide of the embodiment of the first aspect, in which in the polypeptide or polypeptide fragment amino acids of (a) to (f) of the first aspect at positions corresponding to positions F412, F459, L560 and W561 are V, W or R, R or W and A, respectively, or in which in the polypeptide or polypeptide fragment of (g) to (i) of the first aspect amino acids at positions corresponding to positions F412, L455, L560 and W561 are V, W or R, R or W and A, respectively.

In one embodiment, said host cell is further capable of converting HMF to HMFA.

In a ninth aspect, the invention provides a use of the host cell of the fourth aspect for expressing the polypeptide of the embodiment of the first aspect, in which in the polypeptide or polypeptide fragment amino acids of (a) to (f) of the first aspect at positions corresponding to positions F412, F459, L560 and W561 are V, W or R, R or W and A, respectively, or in which in the polypeptide or polypeptide fragment of (g) to (i) of the first aspect amino acids at positions corresponding to positions F412, L455, L560 and W561 are V, W or R, R or W and A, respectively, for the production of FDCA.

In a tenth aspect, the invention provides a crystal structure of the polypeptide having the amino acid shown in SEQ ID NO: 15 characterized as having (a) a space group of P4₃2₁2 and (b) unit cell dimensions of a = 105.23 Å, b = 105.23 Å and c = 186.52 Å, α, β, γ = 90°.

In an eleventh aspect, the invention provides a use of the crystal structure as defined in the tenth aspect for obtaining atomic spatial relationship data.

In a twelfth aspect, the invention provides a use of the crystal structure of the tenth aspect for identifying amino acids which aid in the conversion of FFA to FDCA.

### BRIEF DESCRIPTION OF FIGURES

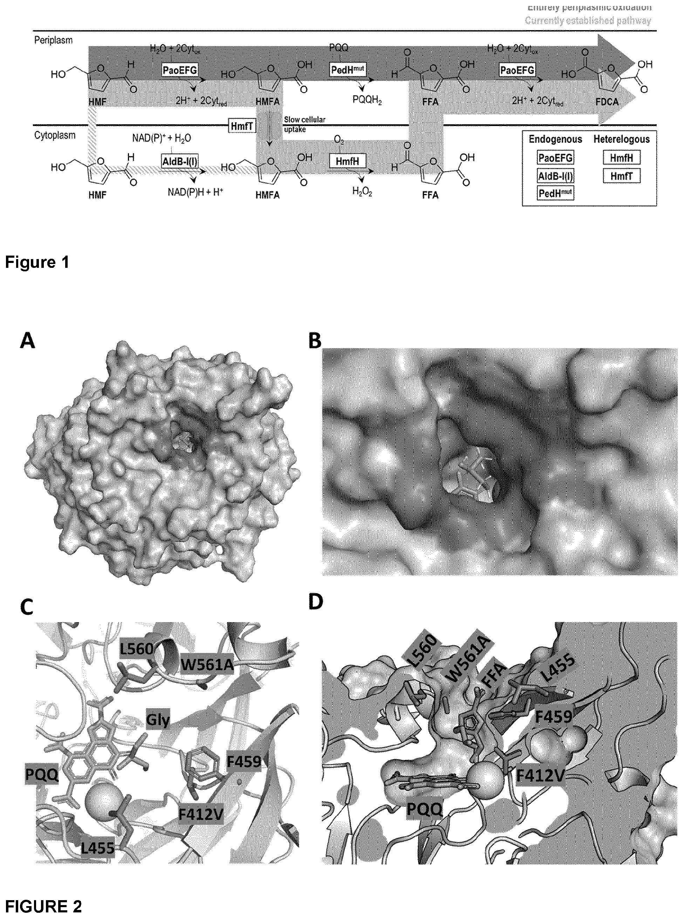

**Figure 1****.** Comparison between the currently established pathway used for whole-cell biocatalytic FDCA production with *P. putida* KT2440 or closely related bacteria (light grey) and an envisioned entirely periplasmic alternative (dark grey) enabled by a rationally designed PedH mutant.
**Figure 2****.** Optimization of the PQQ-dependent alcohol dehydrogenase PedH for carboxylated furanic substrates. **A, B**) surface overview of the double mutant PedH^{F412V/W561A} variant (PDB ID: 6ZCV) showing the active site and the substrate channel of the enzyme formed by the sidechains of C131, E199, W285, V412, L413, A453, L455, F459, A557 and L560 residues. The double mutant PedHF412V/W561A variant is shown in light grey, the residues involved to form the substrate channel shown in darker grey. In the active site, a glycerol molecule, and the cofactor PQQ is coordinating the Pr³⁺. **C**) View of the active site of the double mutant PedH^{F412V/W561A} variant with glycerol (Gly) as a substrate. **D**) Cut-away of the active site, where a molecule of carboxylated furanic substrates (for example FFA or HMFA) is docked onto the glycerol substrate defining the closest residues (L455, F459, and L560) pointing to the carboxylic group of the FFA as an attractive target for further engineering.

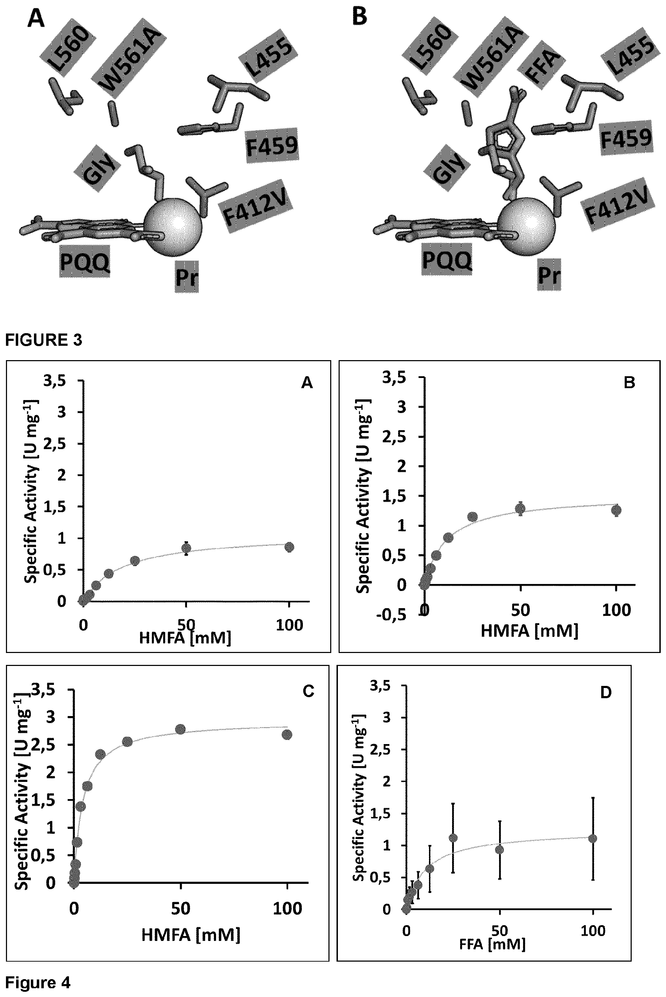

**Figure 3****.** Superimposition of a carboxylated furanic substrate (HMFA or FFA) onto the glycerol, defining L455, F459, and L560 as the closest residues pointing to the carboxylic group of the carboxylated furanic substrate. **A**) The active site of the double mutant PedH^{F412V/W561A} variant with glycerol (Gly) as a substrate. **B**) The active site of the double mutant PedH^{F412V/W561A} variant, where a molecule of carboxylated furanic substrates (for example FFA or HMFA) is docked onto the glycerol substrate.
**Figure 4****.** Kinetic parameters determination of the designed variants. (**A-E**) Michaelis-Menten plot showing the specific enzyme activities of (**A**) PedH^{F412V/W561A}, (**B**) PedH^{F412V/F459W/W561A}, (**C**) PedH^{F412V/L560R/W561A}, (**D**) PedH^{F412V/F459W/L560R/W561A} with different concentrations of HMFA, and (**E**) PedH^{F412V/F459W/L560R/W561A} with different concentrations of FFA.

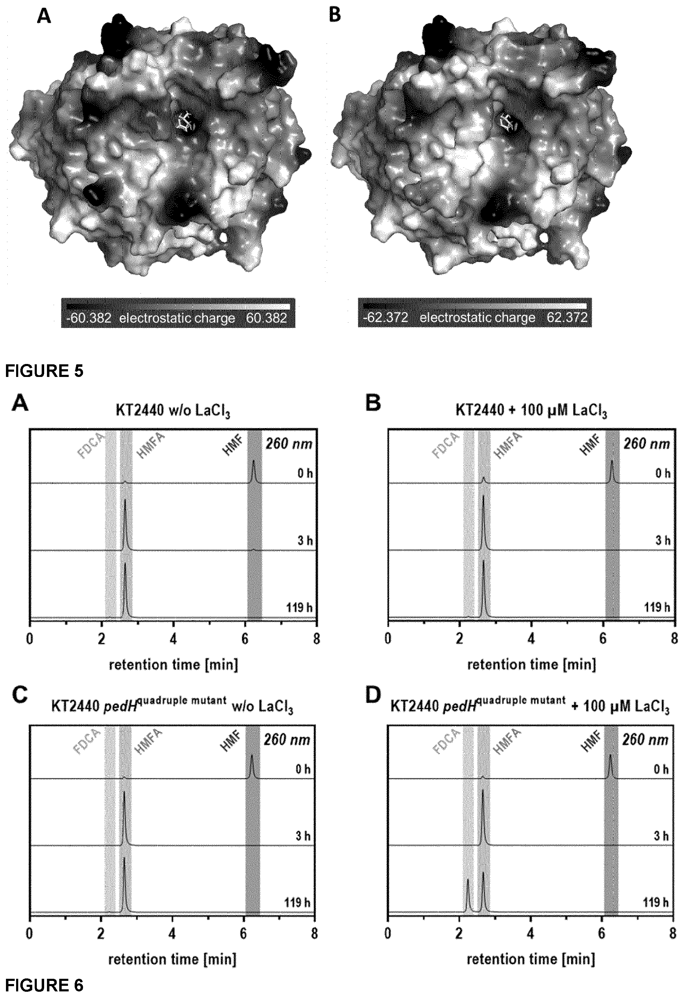

**Figure 5****.** Comparison of the electrostatic charge, ranging from negative (black) to positive (white), of both **A**) the double mutant PedH^{F412V/W561A} variant and **B**) the quadruple mutant PedH^{F412V/F459W/L560R/W561A} variant, showing a clear difference in the electrostatic charge around the substrate channel between both variants.
**Figure 6****.** The rationally engineered PedH quadruple mutant PedH^{F412V/F459W/L560R/W561A} empowers *P. putida* KT2440 to produce FDCA. Selected HPLC chromatograms (absorption at 260 nm) depicting HMF conversion by *P. putida* KT2440 (**A/B**) and *P*. *putida* KT2440 *pedH*^{F412V/F459W/L560R/W561A} (**C/D**) without (**A/C**) and with addition of 100 µM LaCl₃ (**B/D**). Only the mutant strain produced significant amounts of FDCA in case LaCl₃ was present. Experiments were carried out in four-fold buffered MSM with 40 mM glycerol, 2 mM glucose, and 10 mM HMF using shake flasks. A single dataset chosen from duplicates is shown.

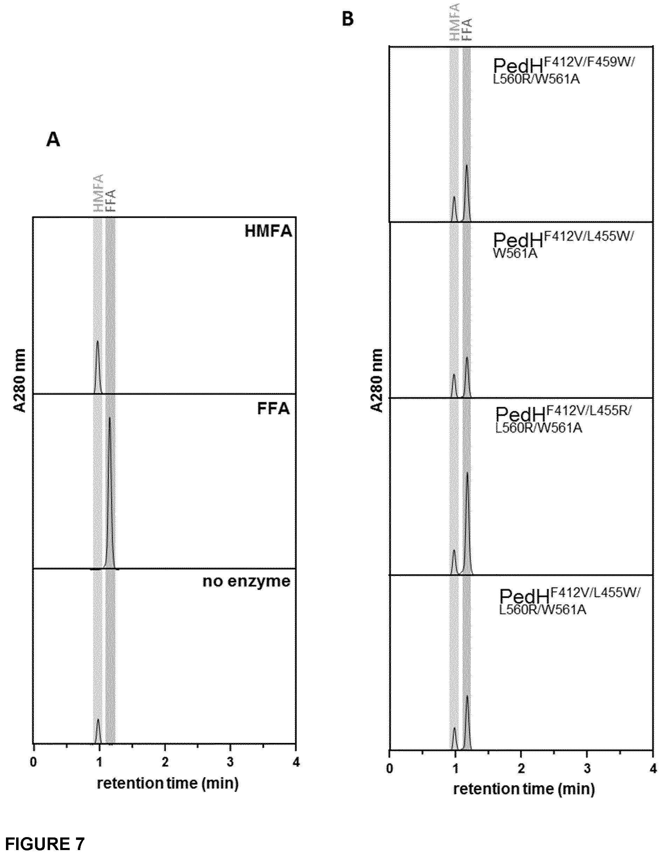

**Figure 7****.** Verification of *in vitro* oxidation of HMFA. Shortly, 200µL of assay solution were prepared containing 10 mM HMFA, 0.4 µM of enzyme, 20 mM HEPES-Na (pH 8), 500 µM phenazine methosulfate (PMS), 150 µM 2,6-dichlorophenol indophenol (DCPIP), 25 mM imidazole, 50 µM PrCl₃, and 5µM PQQ. This mix was incubated at 30°C in a microtiter plate for 6 hours. The plate was covered with a lid to minimize evaporation. The graphs show HPLC chromatograms (absorption at 280 nm) of **A:** analytical standards (HMFA, FFA) and a control reaction without added enzyme, and **B:** reactions with different enzyme mutants (PedH^{F412V/L455W/W561A} PedH^{F412V/F459W/L560R/W561A}, PedH^{F412V/L455R/L560R/W561A}, and PedH^{F412V/L455W/L560R/W561A}). All four tested mutants convert HMFA into FFA, confirming their activity.

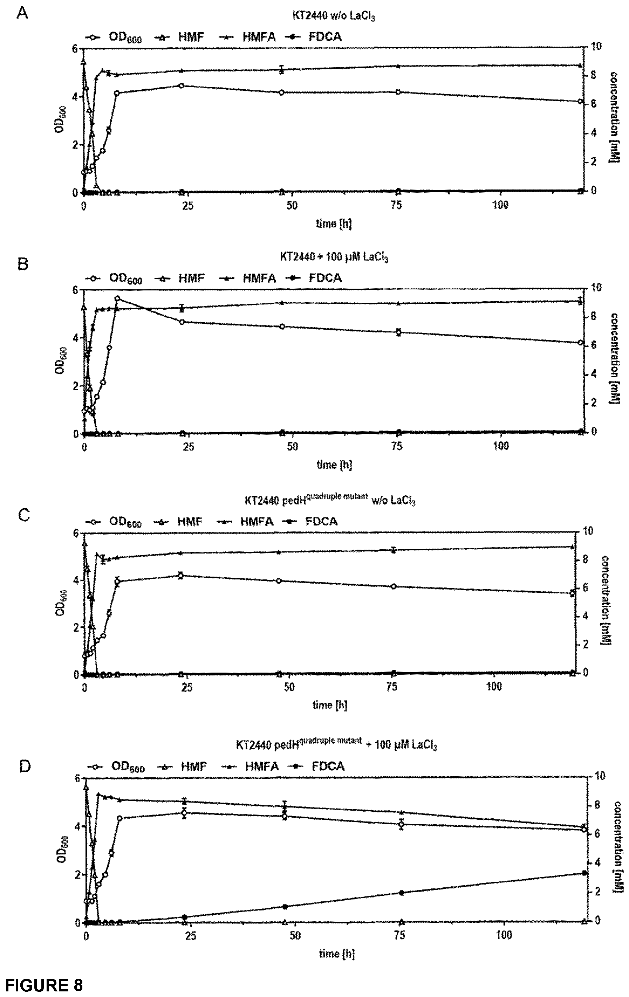

**Figure 8****.** HMF conversion by *P. putida* KT2440 (**A/B**) and *P. putida* KT2440 *pedH*^{F412V/F459W/L560R/W561A} (**C/D**) without (**A/C**) and with addition of 100 µM LaCl₃ (**B/D**) over time. Only the mutant strain produced significant amounts of FDCA in case LaCl₃ is present. Experiments were carried in four-fold buffered MSM with 40 mM glycerol, 2 mM glucose, and 10 mM HMF using shake flasks. The mean and standard deviation of duplicates is shown.

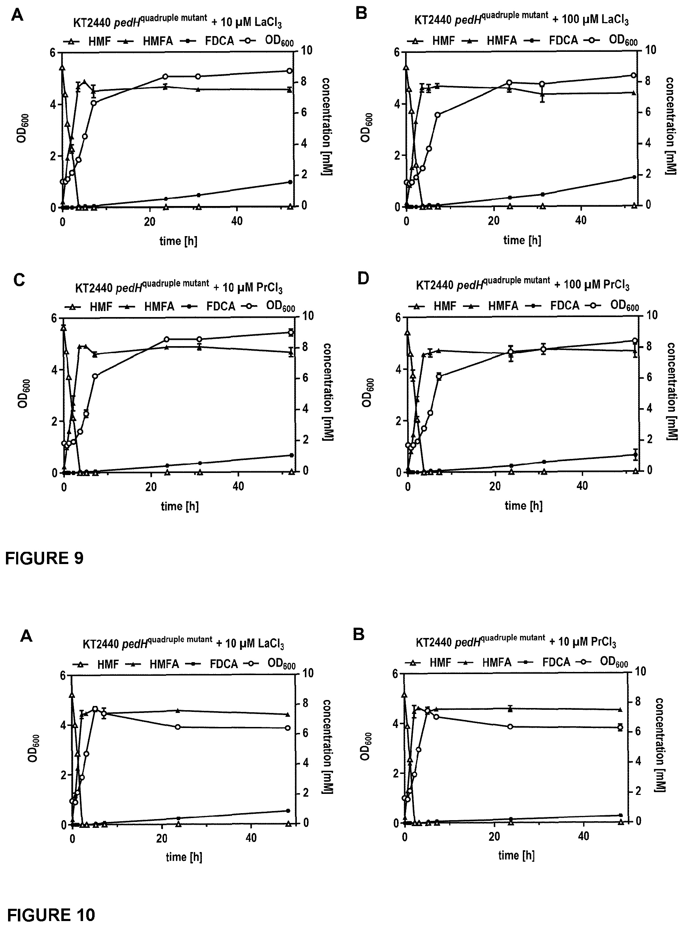

**Figure 9****.** HMF conversion by *P. putida* KT2440 *pedH*^{F412V/F459W/L560R/W561A} with addition of varying lanthanide salts concentrations. (**A**) 10 µM LaCl₃. (**B**) 100 µM LaCl₃. (**C**) 10 µM PrCl₃. (**D**) 100 µM PrCl₃. Experiments were carried out in four-fold buffered MSM with 40 mM glycerol, 2 mM glucose, and 10 mM HMF using shake flasks. The mean and standard deviation of duplicates is shown. According to previous studies, PedH promotor activity in *P. putida* KT2440 is highest in the presence of lanthanum ions and can vary considerably depending on the metal added and its concentration. In contrast, enzyme activity is maximal when praseodymium is used as cofactor (29). As efficient whole-cell biocatalysis benefits from high gene expression and high enzymatic activity, FDCA production experiments were repeated with both REE. Due to a low FDCA production in general, no large differences could be observed. However, there was the trend that dicarboxylic acid formation was a little bit faster in the presence of lanthanum. Furthermore, a higher concentration of the corresponding metal ion seemed to be slightly beneficial.
**Figure 10****.** HMF conversion by *P. putida* KT2440 *pedH*^{F412V/F459W/L560R/W561A} with glucose as carbon source and addition of 10 µM LaCl₃ (**A**) or 10 µM PrCl₃ (**B**). Experiments were carried out in four-fold buffered MSM with 20 mM glucose and 10 mM HMF using shake flasks. The mean and standard deviation of duplicates is shown. As a glycerol molecule was found in the active site of the crystal structure of PedH^{F412V/W561A}, it was speculated that glycerol used as a carbon source could putatively block the active site of the engineered enzyme. To exclude any problem related to glycerol, a control experiment with glucose as carbon source was performed, but no obvious differences to cultivations on glycerol were observed (24).

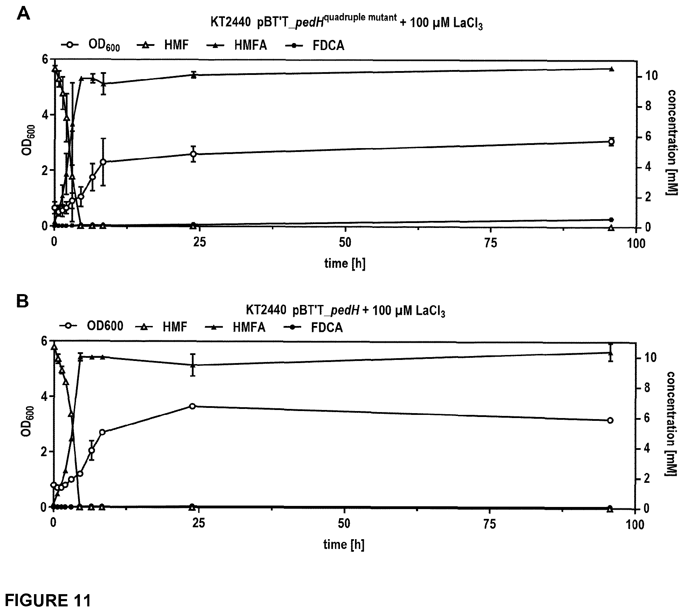

**Figure 11****.** HMF conversion by plasmid-harboring *P*. *putida* KT2440 pBT'T_*pedH*^{F412V/F459W/L560R/W561A} (**A**) and the *P. putida* KT2440 pBT'T_*pedH* control (**B**) with addition of 100 µM LaCl₃. Experiments were carried out in four-fold buffered MSM with 40 mM glycerol, 2 mM glucose, and 10 mM HMF using shake flasks. The mean and standard deviation of duplicates is shown. Although it seemed unlikely, because previous studies showed high *pedH* expression levels even on glucose as carbon source in the absence of lanthanides (39), strong and constitutive plasmid-based expression should exclude a putatively weak natural gene expression as a problem causing low efficiency of the whole-cell biocatalyst. To allow a clean comparison between HMFA oxidation by PedH^{F412V/F459W/L560R/W561A} and HmfH the mutated *pedH* was cloned into pBT'T vector (constitutive *P_{tac}* promotor) ensuring equal expression levels of both proteins. *P. putida* KT2440 was transformed with the resulting plasmid as well as a control harboring the wild type enzyme by electroporation. As expected, only the strain with the plasmid encoding the mutant could produce FDCA. However, HMFA oxidation was even worse than for the strain possessing the genomically modified *pedH.* A reason for that, could be that *P. putida* KT2440 pBT'T_*pedH*^{F412V/F459W/L560R/W561A} possessed a mixture of the mutant, expressed from the plasmid, and the wildtype enzyme, expressed from the genome, so that final protein levels of PedH^{F412V/F459W/L560R/W561A} could be lower than for the strain having the mutations introduced into the genome.

### DETAILED DESCRIPTION OF THE INVENTION

The aim of the present invention is to bypass the intracellular production of FFA by a completely periplasmic production route from HMF to FDCA. The pathway for the complete periplasmic production compared to the known partly intracellular pathway from MF to FDCA is shown in **Figure 1****.**

Herein, it is shown that with a variant of a pyrroloquinoline quinone (PQQ)-dependent alcohol dehydrogenase PedH, a mutated PedH, it is possible to produce 5-formyl-2-furoic acid (FFA) from 5-hydroxymethyl-2-furoic acid (HMFA). Furthermore, it may be possible to produce 2,5 furandicarboxylic acid (FDCA) from FFA by the same PedH variant. With this the yield of FDCA can be increased, since the PaoEFG still produce FDCA from the intermediary molecule FFA. Thus, the heterologous intracellular enzyme HmfH and the transporter HmfT are no longer necessary and the cells must no longer stressed by transforming the cells with the nucleic acid coding for HmfH and HmfT and by expressing these heterologous proteins. Furthermore, by avoiding the heterologous enzymes HmfH and HmfT the cell would contain no foreign DNA, because the variants are point mutations of physiological enzymes of the *Pseudomonas putida.* Thus, these variants may face lower regulatory requirements regarding biosafety.

In general, a low Km value shows a high affinity of the PedH variants to their substrate, e.g. the HMFA. Further, a low Km value also show that the reaction speed is higher, here the reaction from HMFA to FFA and/or from FFA to FDCA.

The inventors surprisingly found and produced PedH variants with three mutations which have a lower Kₘ value for transforming HMFA to FFA, compared to the double mutant of Wehrmann *et al.* (24). Furthermore, they surprisingly found and produced PedH variants with four mutations having not only a lower Kₘ value for transforming HMFA to FFA but further could transform FFA to FDCA using *P. putida* KT2440 as a chassis. This finding is even more surprisingly because in Wehrmann *et al.* the active site of PedH was widened by exchanging at position 420 the phenylalanine with valine (F420V) and at position 561 the tryptophan with alanine (W561A). In the inventive mutation variants, described herein, the active site is narrowed e.g. by exchange of phenylalanine at position 459 with tryptophan (F495W) and exchanging leucine at position 560 by arginine (L560R), wherein with the L560R exchange a hydrophobic amino acid is exchanged to a charged amino acid. Since the active site of the PedH was not widened as in Wehrmann *et al.* but narrowed and modified in the terms of charges, the results, the possibility of the variants to convert HMFA to FFA and, for the quadruple variants, to FDCA is a surprising and not obvious result.

Thus, the inventors showed herein for the first time that FDCA production with *P. putida* KT2440 is achievable without introducing foreign DNA. Instead of heterologously expressed cytoplasmic HmfH, primary alcohol oxidation of HMFA to FFA can also be catalyzed by a rationally-improved quadruple mutant of PQQ- and lanthanide-dependent ADH PedH. This marks the first demonstration of a fully periplasmic oxidation route from HMF to FDCA which: i) circumvents the formation of H₂O₂ as toxic byproduct, ii) conserves the energy released during the oxidation steps, and iii) avoids transport limitation issues. While currently still experiencing low catalytic efficiency *in vivo,* a foundation has been established for further improvement through methods such as growth-coupled or directed enzyme evolution. Overall, the specific refinement of the previously developed mutant PedH^{F412V/W561A} and the first successful application *in vivo* underline the potential of alcohol oxidoreductases in whole-cell biocatalysis and represents an important step forward on the way towards sustainable production of bio-based plastic monomer FDCA.

Thus, the present invention provides in the first aspect a alcohol oxidoreductase polypeptide converting HMFA to FFA with a Km value of less than 15 mM selected from the group consisting of
(a) a polypeptide having the amino acid sequence shown in SEQ ID NO: 1, wherein in said polypeptide amino acids at positions corresponding to positions F412, F459 and W561 are V, W or R and A, respectively;
(b) a polypeptide fragment of the polypeptide of (a), wherein in said polypeptide fragment amino acids at positions corresponding to positions F412, F459 and W561 are V, W or R and A, respectively;
(c) a polypeptide having an amino acid sequence which is at least 70% identical to the amino acid sequence of the polypeptide of (a) or (b), wherein in said polypeptide amino acids at positions corresponding to positions F412, F459 and W561 are V, W or R and A, respectively;
(d) a polypeptide having the amino acid sequence shown in SEQ ID NO: 1, wherein in said polypeptide amino acids at positions corresponding to positions F412, L560 and W561 are V, R or W and A, respectively;
(e) a polypeptide fragment of the polypeptide of (d), wherein in said polypeptide fragment amino acids at positions corresponding to positions F412, L560 and W561 are V, R or W and A, respectively;
(f) a polypeptide having an amino acid sequence which is at least 70% identical to the amino acid sequence of the polypeptide of (d) or (e), wherein in said polypeptide amino acids at positions corresponding to positions F412, L560 and W561 are V, R or W and A, respectively;
(g) a polypeptide having the amino acid sequence shown in SEQ ID NO: 1, wherein in said polypeptide amino acids at positions corresponding to positions F412, L455 and W561 are V, W or R and A, respectively;
(h) a polypeptide fragment of the polypeptide of (g), wherein in said polypeptide fragment amino acids at positions corresponding to positions F412, L455 and W561 are V, W or R and A, respectively;
(i) a polypeptide having an amino acid sequence which is at least 70% identical to the amino acid sequence of the polypeptide of (g) or (h), wherein in said polypeptide amino acids at positions corresponding to positions F412, L455 and W561 are V, W or R and A, respectively.

In one embodiment, said alcohol oxidoreductase is a alcohol dehydrogenase, preferably the alcohol dehydrogenase is a co factor dependent alcohol dehydrogenase, wherein the co factor is a co factor located in the periplasm, preferably the co factor is cytochrome C, pyrroloquinoline quinone (PQQ), or a quinone similar to PQQ, more preferably the co factor dependent alcohol dehydrogenase is the pyrroloquinoline quinone (PQQ) dependent alcohol dehydrogenase, most preferably the co factor dependent alcohol dehydrogenase is PedE or PedH, exceptionally preferred is PedH.

The terms "polypeptide", "polypeptide fragment", or "protein" are used herein synonymously and includes natural and non-natural occurring amino acids bonded to each other with a peptide bond. The natural occurring amino acids are alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, wherein if applicable the amino acid is a L amino acid. Non-natural occurring amino acids includes all enantiomers of natural occurring amino acids and every other amino acid useable as replacement of the natural occurring amino acid.

The position of the mutated amino acids mentioned herein refer to the wildtype (wt) sequence of PedH of *P. putida* KT2440 including the secretion signal for periplasmic targeting. This wt sequence is mentioned herein as SEQ ID NO: 1.

Thus, the present invention includes not only the sequence of wt PedH of *P. putida* KT2440, but also sequences homologous, to some extent identical, to this sequence e.g. without the secretion signal having the same mutated amino acids in a position corresponding to that of the wt PedH sequence. For example, the first triple PedH variant (a) mentioned above has amino acid mutations at the positions 412, 459, 561 of the wt sequence SEQ ID NO: 1 as shown in SEQ ID NO: 4. As an example, due to the secretion signal peptide for targeting the periplasm (SEQ ID NO: 31) at the N-terminus of the wt sequence (SEQ ID NO: 1), the polypeptide will be secreted in the periplasm. During secretion the signal peptide is cleaved of the polypeptide, resulting in the mature PedH with SEQ ID NO: 2. The signal peptide (SEQ ID NO: 31) has a length of 27 amino acid. Therefore, the amino acid positions of the wt sequence SEQ ID NO: 1 shift by -27. For example, the positions of the amino acid mutations mentioned before are shifted from position 412 to position 385 (412-27), from position 459 to 432, and from position 561 to 534, respectively, in the wt sequence without signal peptide (SEQ ID NO: 2.), as shown in SEQ ID NO: 3. Thus, e.g. the amino acid position 385 in SEQ ID NO: 3 corresponds to amino acid position 412 in SEQ ID NO: 4.

The term "position" when used in accordance with the present invention means the position of either an amino acid within an amino acid sequence depicted herein or the position of a nucleotide within a nucleic acid sequence depicted herein. The term "corresponding" as used herein also includes that a position is not only determined by the number of the preceding nucleotides/amino acids. Accordingly, the position of a given amino acid in accordance with the present invention which may vary due to deletion or addition of amino acids, e.g. by deleting the signal peptide. Similarly, the position of a given nucleotide in accordance with the present invention may vary due to deletions or additional nucleotides.

Thus, under a "corresponding position" in accordance with the invention it is preferably to be understood that nucleotides/amino acids may differ in the indicated number but may still have similar neighbouring nucleotides/amino acids. Said nucleotides/amino acids which may be exchanged, deleted or added are also comprised by the term "corresponding position". When used herein "at a position corresponding to a position" a position in a "query" amino acid (or nucleotide) sequence is meant that corresponds to a position in a "subject" amino acid (or nucleotide) sequence. The skilled person will be able to determine "a position corresponding to a position" within an amino acid or nucleic acid sequence, e.g. by sequence alignments, either manually or automatically, e.g. as described herein.

Thus, the invention also includes variants of the sequence of wt PedH of *P. putida* KT2440, resulting in that the position of the amino acid mutations corresponds to the amino acid position in the wt sequence.

Therefore, the invention comprises polypeptides having an amino acid sequence which is at least 70% identical to the amino acid sequence of SEQ ID NO: 1, and wherein amino acids at positions corresponding to the positions in the wt sequence (SEQ ID NO: 1) are mutated.

Part of the inventions are therefore polypeptides and/or polypeptide fragments having at least 70% identity, preferably 80 % identity, more preferably 90 % identity, even more preferably 95 % identity to the wt sequence of PedH (SEQ ID NO: 1) including the respective amino acid mutations at positions corresponding the respective amino acid position in the wt sequence. The comparison between a given polypeptide sequence and the wt sequence of PedH (Seq ID NO: 1) or a fragment thereof to determine "% identity" can be conducted by eye, or more usually, with the aid of readily available sequence comparison programs. These commercially available computer programs can calculate % identity or % homology between two or more sequences.

% identity or homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues (for example, less than 50 contiguous amino acids).

Thus, in a first triple mutation variant of the alcohol oxidoreductase, e.g. PedH, the amino acids at positions 412, 459, and 561 of the SEQ ID NO: 1, the wildtype (wt) sequence of PedH of *P. putida* KT2440, have been mutated in that the amino acids at positions corresponding to positions F412, F459, and W561 (of SEQ ID NO: 1) has been changed (or mutated) to V, W or R and A, respectively, in e.g. the PedH variant (a). This first triple mutation variant includes polypeptide fragments (b) and polypeptides having at least 70% sequence identity to the polypeptide of variants (a) or (b) ((c)), in which the wt sequence has been mutated, given that the polypeptide fragment (b) and polypeptide having at least 70% identity (c) have also the amino acid mutations mentioned in variant (a) at positions corresponding to the wt sequence. The first triple mutation variant includes variants (a) to (c) mentioned above.

In one embodiment, the first triple variant (a) has the sequences shown in SEQ ID NO: 3, 4, 5 or 6. Therein, the SEQ ID NOs: 3 and 4 shows the amino acid sequence of the variant PedH^{F412V, F459W, W561A} with (SEQ ID NO: 4) and without (SEQ ID NO: 3) the signal peptide for secretion into the periplasm; the SEQ ID NOs 5 and 6 shows the amino acid sequence of the variant PedH^{F412V, F459R, W561A} with (SEQ ID NO: 6) and without (SEQ ID NO: 5) the signal peptide for secretion into the periplasm as example amino acid sequences of the first triple mutation variant.

In a second triple mutation variant of the alcohol oxidoreductase, e.g. PedH, the amino acids at positions 412, 560, and 561 of the SEQ ID NO: 1, the wildtype (wt) sequence of PedH of *P. putida* KT2440, have been mutated in that the amino acids at positions corresponding to positions F412, L560, and W561 (of SEQ ID NO: 1) has been changed (or mutated) to V, R or W and A, respectively, in e.g. the PedH variant (d). This second triple mutation variant includes polypeptide fragments (e) and polypeptides having at least 70% sequence identity to the polypeptide of variants (d) or (e) ((f)), in which the wt sequence has been mutated, given that also the polypeptide fragment (e) and the polypeptide having at least 70% identity (f) have also the amino acid mutations mentioned in variant (d) at positions corresponding to the wt sequence. The second triple mutation variant includes variants (d) to (f) mentioned above.

In one embodiment, the second triple variant (d) has the sequences shown in SEQ ID NO: 7, 8, 9, or 10. Therein, the SEQ ID NOs: 7 and 8 shows the amino acid sequence of the variant PedH^{F412V, L560R, W561A} with (SEQ ID NO: 8) and without (SEQ ID NO: 7) the signal peptide for secretion into the periplasm; the SEQ ID NOs 9 and 10 shows the amino acid sequence of the variant PedH^{F412V, L560W, W561A} with (SEQ ID NO: 10) and without (SEQ ID NO: 9) the signal peptide for secretion into the periplasm as example amino acid sequences of the second triple mutation variant.

In a third triple mutation variant of the alcohol oxidoreductase, e.g. PedH, the amino acids at positions 412, 455, and 561 of the SEQ ID NO: 1, the wildtype (wt) sequence of PedH of *P. putida* KT2440, have been mutated in that the amino acids at positions corresponding to positions F412, L455, and W561 (of SEQ ID NO: 1) has been changed (or mutated) to V, W or R and A, respectively, in e.g. the PedH variant. This triple mutation variant includes polypeptide fragments (h) and polypeptides having at least 70% sequence identity to the polypeptide of variants (g) or (h) ((i)), in which the wt sequence has been mutated, given that also the polypeptide fragment (h) and the polypeptide having at least 70% identity (i) have also the amino acid mutations mentioned before at positions corresponding to the wt sequence. The third triple mutation variant includes variants (g) to (i) mentioned above.

In one embodiment, the third triple variant (g) has the sequences shown in SEQ ID NO: 11, 12, 13, or 14. Therein, the SEQ ID NOs: 11 and 12 shows the amino acid sequence of the variant PedH^{F412V,L455W, W561A} with (SEQ ID NO: 12) and without (SEQ ID NO: 11) the signal peptide for secretion into the periplasm; the SEQ ID NOs 13 and 14 shows the amino acid sequence of the variant PedH^{F412V, L455R, W561A} with (SEQ ID NO: 14) and without (SEQ ID NO: 13) the signal peptide for secretion into the periplasm as example amino acid sequences of the third triple mutation variant.

All the triple mutation variants, the first, second and third triple mutation variant, are able to convert HMFA to FFA with a Km value of less than 15 mM.

In one embodiment of the invention, in the polypeptide or polypeptide fragment of the first and/or second triple mutation variants ((a) to (f)) mentioned above, the amino acids at positions corresponding to positions F412, F459, L560 and W561 (of the wt sequence SEQ ID NO: 1) are V, W or R, R or W and A, respectively, and these polypeptides or polypeptide fragments further converting FFA to FDCA.

In a further embodiment, this polypeptide, having four amino acid mutations, is selected from the group consisting of
(a) a polypeptide having the amino acid sequence shown in SEQ ID NO: 1, wherein in said polypeptide amino acids at positions corresponding to positions F412, F459, L560 and W561 are V, W or R, R or W and A, respectively;
(b) a polypeptide fragment of the polypeptide of (a), wherein in said polypeptide fragment amino acids at positions corresponding to positions F412, F459, L560 and W561 are V, W or R, R or W, and A, respectively;
(c) a polypeptide having an amino acid sequence which is at least 70% identical to the amino acid sequence of the polypeptide of (a) or (b), wherein in said polypeptide amino acids at positions corresponding to positions F412, F459, L560 and W561 are V, W or R, R or W and A, respectively.

Thus, in this, first, quadruple mutation variant of the alcohol oxidoreductase, e.g. PedH, the amino acids corresponding to the positions 412, 459, 560, and 561 of the wt sequence, SEQ ID NO: 1, have been mutated. As explained above, "corresponding to position(s)" means that the amino acid position in the wt sequence (Seq ID NO: 1) is given and in the case of e.g. an amino acid sequence being shorter than the wt sequence the position of the respective amino acids changed accordingly.

In this first quadruple mutation variant the amino acids at positions 412, 459, 560, and 561 of SEQ ID NO: 1, the wildtype (wt) sequence of PedH of *P. putida* KT2440, have been mutated in that the amino acids at positions corresponding to positions F412, F459, L560, and W561 (of SEQ ID NO: 1) has been changed (or mutated) to V, W or R, R or W and A, respectively, in e.g. the first quadruple PedH variant (a). This quadruple mutation variant includes polypeptide fragments (b) and polypeptides having at least 70% sequence identity to the polypeptide of variants (a) or (b) ((c)), in which the wt sequence has been mutated, given that also these polypeptide fragment (b) and polypeptides having at least 70% identity (c) have also the amino acid mutations mentioned before at positions corresponding to the wt sequence. The first quadruple mutation variant includes variants (a) to (c) mentioned above.

In one embodiment, the first quadruple variant (a) has the sequences shown in SEQ ID NO: 15, 16, 17, 18, 19, 20, 21, or 22. Therein, the SEQ ID NOs: 15 and 16 shows the amino acid sequence of the variant PedH^{F412V, F459W, L560R, W561A} with (SEQ ID NO: 16) and without (SEQ ID NO: 15) the signal peptide for secretion into the periplasm; the SEQ ID NOs 17 and 18 shows the amino acid sequence of the variant PedH^{F412V, F459R, L560W, W561A} with (SEQ ID NO: 18) and without (SEQ ID NO: 17) the signal peptide for secretion into the periplasm; the SEQ ID NOs 19 and 20 shows the amino acid sequence of the variant PedH^{F412V, F459W, L560W, W561A} with (SEQ ID NO: 20) and without (SEQ ID NO: 19) the signal peptide for secretion into the periplasm; and the SEQ ID NOs 21 and 22 shows the amino acid sequence of the variant PedH^{F412V, F459R, L560R, W561A} with (SEQ ID NO: 22) and without (SEQ ID NO: 21) the signal peptide for secretion into the periplasm as example amino acid sequences of the first quadruple mutation variant.

This first quadruple mutation variant is able to convert HMFA to FFA with a Km value of less than 15 mM and additionally to convert FFA to FDCA.

In one embodiment of the invention, in the polypeptide or polypeptide fragment of the third triple mutation variant ((g) to (i)) mentioned above, the amino acids at positions corresponding to positions F412, L455, L560 and W561 (of the wt sequence SEQ ID NO: 1) are V, W or R, R or W and A, respectively, and these polypeptides or polypeptide fragments further converting FFA to FDCA.

In a further embodiment, this polypeptide, having four amino acid mutations, is selected from the group consisting of
(a) a polypeptide having the amino acid sequence shown in SEQ ID NO: 1, wherein in said polypeptide amino acids at positions corresponding to positions F412, L455, L560 and W561 are V, W or R, R or W and A, respectively;
(b) a polypeptide fragment of the polypeptide of (a), wherein in said polypeptide fragment amino acids at positions corresponding to positions F412, L455, L560 and W561 are V, W or R, R or W, and A, respectively;
(c) a polypeptide having an amino acid sequence which is at least 70% identical to the amino acid sequence of the polypeptide of (a) or b), wherein in said polypeptide amino acids at positions corresponding to positions F412, L455, L560 and W561 are V, W or R, R or W and A, respectively.

Thus, in this, second, quadruple mutation variant of the alcohol oxidoreductase, e.g. PedH, the amino acids corresponding to the positions 412, 455, 560, and 561 of the wt sequence, SEQ ID NO: 1, have been mutated. As explained above, "corresponding to position(s)" means that the amino acid position in the wt sequence (Seq ID NO: 1) is given and in the case of e.g. an amino acid sequence been shorter than the wt sequence the position of the respective amino acids changed accordingly. In this second quadruple mutation variant the amino acids at positions 412, 455, 560, and 561 of SEQ ID NO: 1, the wildtype (wt) sequence of PedH of *P. putida* KT2440, have been mutated in that the amino acids at positions corresponding to positions F412, L455, L560 and W561 (of SEQ ID NO: 1) has been changed (or mutated) to V, W or R, R or W and A, respectively, in e.g. the second quadruple PedH variant (a). This second quadruple mutation variant includes polypeptide fragments (b) and polypeptides having at least 70% sequence identity to the polypeptide of variants (a) or (b) ((c)), in which the wt sequence has been mutated, given that also this polypeptide fragment (b) and polypeptide having at least 70% identity (c) have also the amino acid mutations mentioned before at positions corresponding to the wt sequence.

In one embodiment, the first quadruple variant (a) has the sequences shown in SEQ ID NO: 23, 24, 25, 26, 27, 28, 29, and 30. Therein, the SEQ ID NOs: 23 and 24 shows the amino acid sequence of the variant PedH^{F412V, L455W, L560R, W561A} with (SEQ ID NO: 24) and without (SEQ ID NO: 23) the signal peptide for secretion into the periplasm; the SEQ ID NOs 25 and 26 shows the amino acid sequence of the variant PedH^{F412V, L455R, L560W, W561A} with (SEQ ID NO: 26) and without (SEQ ID NO: 25) the signal peptide for secretion into the periplasm; the SEQ ID NOs 27 and 28 shows the amino acid sequence of the variant PedH^{F412V, L455W, L560W, W561A} with (SEQ ID NO: 28) and without (SEQ ID NO: 27) the signal peptide for secretion into the periplasm; and the SEQ ID NOs 29 and 30 shows the amino acid sequence of the variant Ped^{F412V, L455R, L560R, W561A} with (SEQ ID NO: 30) and without (SEQ ID NO: 29) the signal peptide for secretion into the periplasm as example amino acid sequences of the second quadruple mutation variant.

This second quadruple mutation variant is also able to convert HMFA to FFA with a Km value of less than 15 mM and additionally to convert FFA to FDCA.

In a second aspect the invention provides a polynucleotide encoding the polypeptides of the first triple mutation variant ((a) to (c)), the second triple mutation variant ((d) to (f)), and/or the third triple mutation variant ((g) to (i)), including polynucleotides encoding the polypeptides having one of the sequences of SEQ ID NO: 3 to 14. Further, the second aspect provides a polynucleotide encoding the polypeptides of the first quadruple mutation variant ((a) to (c)) and/or the second quadruple mutation variant ((a) to (c)), including polynucleotides encoding the polypeptides having one of the sequences of SEQ ID NO: 15 to 30.

The term polynucleotide includes every nucleic acid, e.g. deoxyribonucleic acid (DNA) or ribonucleic acid (RNA), with any possible composition including natural occurring nucleobases or non-natural occurring nucleobases e.g. universal or degenerated bases as known by the skilled person.

In one embodiment of the second aspect, the polynucleotide mentioned above has the nucleotide sequence shown in SEQ ID NO: 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 67, 68, 69, 70, 71, 72, 73, 74, 75, 76, 77, 78, 79, 80, 81, 82, 83, 84, 85, 86, 87, 88, 89, 90, 91, 92 or 93. The SEQ ID NO: 35 to SEQ ID NO: 62 are DNA sequences and the SEQ ID NO: 63 to SEQ ID NO: 93 are RNA sequences.

In a third aspect, the invention provides a vector comprising the polynucleotide of one embodiment of the second aspect. For expression in *P. putida* especially preferred are one of the polynucleotides having the nucleotide sequence shown in SEQ ID NO: 36, 38, 40, 42, 44, 46, 48, 50, 52, 54, 56, 58, 60, or 62; each comprising the nucleotide sequence encoding the signal peptide for targeting the periplasm. Exemplified vectors could be found in the Example section of this application.

In an embodiment of the third aspect, the vector is an expression vector.

The skilled person knows how to construct an expression vector and which genetic elements are necessary to create a vector which is able to express a gene of interest. Further, expression vectors are commercially available to the skilled person. Of note in this context is, that the alcohol oxidoreductase, e.g. PedH, mutation variants mentioned herein may be expressed in the periplasmic space of a bacterium. Therefore, the polynucleotide inserted as the gene of interest in the vector should comprise a polynucleotide sequence coding for the respective signal peptide for targeting the expressed polypeptide to the periplasm. The sequence of one such signal peptide is shown in SEQ ID NO: 128.

In a fourth aspect the invention provides a host cell comprising the polynucleotide of the second aspect or the vector of the third aspect.

In general, the host cell could be any cell, e.g. a bacterial cell, a fungal cell, a plant cell, or a eukaryotic cell. Especially preferred is the bacterial cell.

In one embodiment the host cell is a gram-negative bacterial host cell.

The gram-negative bacterial cell is preferred since it has two membranes, an inner cytoplasmic membrane and an outer cell membrane, and the periplasm is the space between these two membranes. Whereas gram-positive bacterial cells have the cytoplasmic membrane and a peptidoglycan layer on the cytoplasmic membrane and a small periplasm space between them.

In one further embodiment the gram-negative bacterial host cell is a *Pseudomonas* species, preferably *Pseudomonas putida.*

In an embodiment of the gram-negative bacterial host cell, the host cell lacks a functional hmft1 gene, which encodes HMFA transporter and /or a functional hmfH gene.

As explained above and shown in figure 1, by the use of the inventive triple and/or quadruple mutant variants of the alcohol oxidoreductase, e.g. PedH, described herein there is no more need of transporting HMFA in the cytoplasm, since at least the converting of HMFA to FFA happens in the periplasm and not, as mentioned in the prior art, in the cytoplasm, by HfmH. Therefore, there is no need for the hmft1 gene coding for the HmfT transporter which transports HFMA in the cytoplasm of the bacterial cell. Furthermore, since the inventive PedH variants described herein convert HMFA to FFA, there is also no need for HfmH to catalyse this conversion in the cytoplasm of the bacterial cell and the heterologous hfmH gene could be omitted.

In a fifth aspect the invention provides a method for converting HMFA to FFA, comprising culturing the host cell of the fourth aspect expressing the polypeptide as defined in the first aspect, namely the polypeptides of the first triple mutation variant ((a) to (c)), the second triple mutation variant ((d) to (f)), and/or the third triple mutation variant ((g) to (i)), including polynucleotides encoding the polypeptides having one of the sequences of SEQ ID NO: 3 to 14, and further, the second aspect provides a polynucleotide encoding the polypeptides of the first quadruple mutation variant ((a) to (c)) and/or the second quadruple mutation variant ((a) to (c)), including polynucleotides encoding the polypeptides having one of the sequences of SEQ ID NO: 15 to 30.

As shown above, the inventive triple and quadruple mutant variants are able to convert HMFA to FFA.

In the case of PedE mutant variants calcium has to be added and in the case of PedH variants a lanthanide has to be added.

In one embodiment, in the method of converting HMFA to FFA, the FFA is further converted to FDCA by the host cell of the fourth aspect expressing the polypeptide of the first or second quadruple mutation variant as defined above.

As mentioned above, the first and second quadruple mutation variants are able to convert HMFA to FFA and also to convert FFA to FDCA.

In one embodiment of the fifth aspect, the host cell used in the inventive method is further capable of converting HMF to HMFA.

Thus, there complete pathway from HMF to FDCA could be located at the periplasm as shown in figure 1.

In a sixth aspect, the invention provides a method for the production of FDCA, comprising culturing a host cell expressing a polypeptide of the first or second quadruple mutation variant as defined above.

As mentioned above, the first and second quadruple mutation variants are able to convert FFA to FDCA.

In one embodiment of the fifth and sixth aspect, the conversion of HMFA to FFA and/or of FFA to FDCA takes place in the periplasm of said host cell.

With this a total periplasm-based production of FDCA is possible. This is accomplished by two possible alternatives. First, using the inventive triple mutant variants, the HMFA in the periplasm is converted to FFA by the periplasm located inventive triple mutant variants and the FFA is converted to FDCA by other, possibly endogenous enzymes, like PaoEFG. Second, using the inventive quadruple mutant variants, the HMFA in the periplasm is converted to FFA by the periplasm located inventive quadruple mutant variants and the FFA is converted to FDCA by the inventive quadruple mutant variants and endogenous PaoEFG.

In a seventh aspect the invention provides a use of the host cell of the fourth aspect for converting HMFA to FFA, and said host cell expressing the polypeptide as defined in the first aspect, namely one of the inventive triple or quadruple mutant variants.

As shown above, the inventive triple and quadruple mutant variants are able to convert HMFA to FFA.

In an eight aspect, the invention provides a use of the host cell of the fourth aspect for converting FFA to FDCA, and said host cell expressing the polypeptide of the first or second quadruple variant as defined in the first aspect.

As mentioned above, the first and second quadruple mutation variants are able to convert FFA to FDCA.

In one embodiment of the seventh and eight aspect said host cell is further capable of converting HMF to HMFA.

Thus, there complete pathway from HMF to FDCA could be located at the periplasm as shown in figure 1.

In a ninth aspect, the invention provides a use of the host cell of the fourth aspect for expressing the polypeptide of the first or second quadruple variant as defined in the first aspect for the production of FDCA.

As mentioned above, the first and second quadruple mutation variants are able to convert HMFA to FFA and also to convert FFA to FDCA enabling a total periplasm-based production of FDCA.

In a tenth aspect, the invention provides a crystal structure of the polypeptide having the amino acid shown in SEQ ID NO: 15 characterized as having (a) a space group of P4₃2₁2 and (b) unit cell dimensions of a = 105.23 Å, b = 105.23 Å and c = 186.52 Å, α, β, γ = 90°.

The crystal structure as e.g. shown in the examples, could be used to identify further amino acids which helps by the conversion of FFA to FDCA, especially if the atomic spatial relationship data are considered.

Therefore, in an eleventh aspect, the invention provides for a use of the crystal structure of the tenth aspect for obtaining atomic spatial relationship data.

Therefore, in a twelfth aspect, the invention provides a use of the crystal structure of the tenth aspect for identifying amino acids which aid in the conversion of FFA to FDCA.

The description of the sequences, shown in the sequence listing and as used in the context of the present invention, is as follows:

| | |
|---|---|
| SEQ ID NO: 1 | shows the amino acid sequence of wildtype PedH with the signal peptide |
| SEQ ID NO: 2 | shows the amino acid sequence of wildtype PedH without the signal peptide |
| SEQ ID NO: 3 | shows the amino acid sequence of PedH F412V, F459W, W561A mutant without the signal peptide |
| SEQ ID NO: 4 | shows the amino acid sequence of PedH F412V, F459W, W561A mutant with the signal peptide |
| SEQ ID NO: 5 | shows the amino acid sequence of PedH F412V, F459R, W561A mutant without the signal peptide |
| SEQ ID NO: 6 | shows the amino acid sequence of PedH F412V, F459R, W561A mutant with the signal peptide |
| SEQ ID NO: 7 | shows the amino acid sequence of PedH F412V, L560R, W561A mutant without the signal peptide |
| SEQ ID NO: 8 | shows the amino acid sequence of PedH F412V, L560R, W561A mutant with the signal peptide |
| SEQ ID NO: 9 | shows the amino acid sequence of PedH F412V, L560W, W561A mutant without the signal peptide |
| SEQ ID NO: 10 | shows the amino acid sequence of PedH F412V, L560W, W561A mutant with the signal peptide |
| SEQ ID NO: 11 | shows the amino acid sequence of PedH F412V, L455W, W561A mutant without the signal peptide |
| SEQ ID NO: 12 | shows the amino acid sequence of PedH F412V, L455W, W561A mutant with the signal peptide |
| SEQ ID NO: 13 | shows the amino acid sequence of PedH F412V, L455R, W561A mutant without the signal peptide |
| SEQ ID NO: 14 | shows the amino acid sequence of PedH F412V, L455R, W561A mutant with the signal peptide |
| SEQ ID NO: 15 | shows the amino acid sequence of PedH F412V, F459W, L560R, W561A mutant without the signal peptide |
| SEQ ID NO: 16 | shows the amino acid sequence of PedH F412V, F459W, L560R, W561A mutant with the signal peptide |
| SEQ ID NO: 17 | shows the amino acid sequence of PedH F412V, F459R, L560W, W561A mutant without the signal peptide |
| SEQ ID NO: 18 | shows the amino acid sequence of PedH F412V, F459R, L560W, W561A mutant with the signal peptide |
| SEQ ID NO: 19 | shows the amino acid sequence of PedH F412V, F459W, L560W, W561A mutant without the signal peptide |
| SEQ ID NO: 20 | shows the amino acid sequence of PedH F412V, F459W, L560W, W561A mutant with the signal peptide |
| SEQ ID NO: 21 | shows the amino acid sequence of PedH F412V, F459R, L560R, W561A mutant without the signal peptide |
| SEQ ID NO: 22 | shows the amino acid sequence of PedH F412V, F459R, L560R, W561A mutant with the signal peptide |
| SEQ ID NO: 23 | shows the amino acid sequence of PedH F412V, L455W, L560R, W561A mutant without the signal peptide |
| SEQ ID NO: 24 | shows the amino acid sequence of PedH F412V, L455W, L560R, W561A mutant with the signal peptide |
| SEQ ID NO:25 | shows the amino acid sequence of PedH F412V, L455R, L560W, W561A mutant without the signal peptide |
| SEQ ID NO: 26 | shows the amino acid sequence of PedH F412V, L455R, L560W, W561A mutant with the signal peptide |
| SEQ ID NO: 27 | shows the amino acid sequence of PedH F412V, L455R, L560W, W561A mutant without the signal peptide |
| SEQ ID NO: 28 | shows the amino acid sequence of PedH F412V, L455R, L560W, W561A mutant with the signal peptide |
| SEQ ID NO: 29 | shows the amino acid sequence of PedH F412V, L455R, L560R, W561A mutant without the signal peptide |
| SEQ ID NO: 30 | shows the amino acid sequence of PedH F412V, L455R, L560R, W561A mutant with the signal peptide |
| SEQ ID NO: 31 | shows the amino acid sequence of the secretion signal peptide for targeting the periplasm (part of e.g. SEQ ID NO: 1) |
| SEQ ID NO: 32 | shows the amino acid sequence of PedH F412V, F459W, W561A mutant, with an N-terminal Met and a C-terminal 6xHis-tag, without the signal peptide |
| SEQ ID NO: 33 | shows the DNA sequence of wild type PedH (DNA sequence encoding amino acid sequence of SEQ ID NO: 1) |
| SEQ ID NO: 34 | shows the DNA sequence of wild type PedH (DNA sequence encoding amino acid sequence of SEQ ID NO: 2) |
| SEQ ID NO: 35 | shows the DNA sequence of PedH F412V, F459W, W561A mutant (DNA sequence encoding amino acid sequence of SEQ ID NO: 3) |
| SEQ ID NO: 36 | shows the DNA sequence of PedH F412V, F459W, W561A mutant (DNA sequence encoding amino acid sequence of SEQ ID NO: 4) |
| SEQ ID NO: 37 | shows the DNA sequence of PedH F412V, F459R, W561A mutant (DNA sequence encoding amino acid sequence of SEQ ID NO: 5) |
| SEQ ID NO: 38 | shows the DNA sequence of PedH F412V, F459R, W561A mutant (DNA sequence encoding amino acid sequence of SEQ ID NO: 6) |
| SEQ ID NO: 39 | shows the DNA sequence of PedH F412V, L560R, W561A mutant (DNA sequence encoding amino acid sequence of SEQ ID NO: 7) |
| SEQ ID NO: 40 | shows the DNA sequence of PedH F412V, L560R, W561A mutant (DNA sequence encoding amino acid sequence of SEQ ID NO: 8) |
| SEQ ID NO: 41 | shows the DNA sequence of PedH F412V, L560W, W561A mutant (DNA sequence encoding amino acid sequence of SEQ ID NO: 9) |
| SEQ ID NO: 42 | shows the DNA sequence of PedH F412V, L560W, W561A mutant (DNA sequence encoding amino acid sequence of SEQ ID NO: 10) |
| SEQ ID NO: 43 | shows the DNA sequence of PedH F412V, L455W, W561A mutant (DNA sequence encoding amino acid sequence of SEQ ID NO: 11) |
| SEQ ID NO: 44 | shows the DNA sequence of PedH F412V, L455W, W561A mutant (DNA sequence encoding amino acid sequence of SEQ ID NO: 12) |
| SEQ ID NO: 45 | shows the DNA sequence of PedH F412V, L455R, W561A mutant (DNA sequence encoding amino acid sequence of SEQ ID NO: 13) |
| SEQ ID NO: 46 | shows the DNA sequence of PedH F412V, L455R, W561A mutant (DNA sequence encoding amino acid sequence of SEQ ID NO: 14) |
| SEQ ID NO: 47 | shows the DNA sequence of PedH F412V, F459W, L560R, W561A mutant (DNA sequence encoding amino acid sequence of SEQ ID NO: 15) |
| SEQ ID NO: 48 | shows the DNA sequence of PedH F412V, F459W, L560R, W561A mutant (DNA sequence encoding amino acid sequence of SEQ ID NO: 16) |
| SEQ ID NO: 49 | shows the DNA sequence of PedH F412V, F459R, L560W, W561A mutant (DNA sequence encoding amino acid sequence of SEQ ID NO: 17) |
| SEQ ID NO: 50 | shows the DNA sequence of PedH F412V, F459R, L560W, W561A mutant (DNA sequence encoding amino acid sequence of SEQ ID NO: 18) |
| SEQ ID NO: 51 | shows the DNA sequence of PedH F412V, F459W, L560W, W561A mutant (DNA sequence encoding amino acid sequence of SEQ ID NO: 19) |
| SEQ ID NO: 52 | shows the DNA sequence of PedH F412V, F459W, L560W, W561A mutant (DNA sequence encoding amino acid sequence of SEQ ID NO: 20) |
| SEQ ID NO: 53 | shows the DNA sequence of PedH F412V, F459R, L560R, W561A mutant (DNA sequence encoding amino acid sequence of SEQ ID NO: 21) |
| SEQ ID NO: 54 | shows the DNA sequence of PedH F412V, F459R, L560R, W561A mutant (DNA sequence encoding amino acid sequence of SEQ ID NO: 22) |
| SEQ ID NO: 55 | shows the DNA sequence of PedH F412V, L455W, L560R, W561A mutant (DNA sequence encoding amino acid sequence of SEQ ID NO: 23) |
| SEQ ID NO: 56 | shows the DNA sequence of PedH F412V, L455W, L560R, W561A mutant (DNA sequence encoding amino acid sequence of SEQ ID NO: 24) |
| SEQ ID NO: 57 | shows the DNA sequence of PedH F412V, L455R, L560W, W561A mutant (DNA sequence encoding amino acid sequence of SEQ ID NO: 25) |
| SEQ ID NO: 58 | shows the DNA sequence of PedH F412V, L455R, L560W, W561A mutant (DNA sequence encoding amino acid sequence of SEQ ID NO: 26) |
| SEQ ID NO: 59 | shows the DNA sequence of PedH F412V, L455W, L560W, W561A mutant (DNA sequence encoding amino acid sequence of SEQ ID NO: 27) |
| SEQ ID NO: 60 | shows the DNA sequence of PedH F412V, L455W, L560W, W561A mutant (DNA sequence encoding amino acid sequence of SEQ ID NO: 28) |
| SEQ ID NO: 61 | shows the DNA sequence of PedH F412V, L455R, L560R, W561A mutant (DNA sequence encoding amino acid sequence of SEQ ID NO: 29) |
| SEQ ID NO: 62 | shows the DNA sequence of PedH F412V, L455R, L560R, W561A mutant (DNA sequence encoding amino acid sequence of SEQ ID NO: 30) |
| SEQ ID NO: 63 | shows the DNA sequence of PedH F412V, F459W, W561A mutant (DNA sequence encoding amino acid sequence of SEQ ID NO: 32) |
| SEQ ID NO: 64 | shows the RNA sequence of wild type PedH (RNA sequence encoding amino acid sequence of SEQ ID NO: 1) |
| SEQ ID NO: 65 | shows the RNA sequence of wild type PedH (RNA sequence encoding amino acid sequence of SEQ ID NO: 2) |
| SEQ ID NO: 66 | shows the RNA sequence of PedH F412V, F459W, W561A mutant (RNA sequence encoding amino acid sequence of SEQ ID NO: 3) |
| SEQ ID NO: 67 | shows the RNA sequence of PedH F412V, F459W, W561A mutant (RNA sequence encoding amino acid sequence of SEQ ID NO: 4) |
| SEQ ID NO: 68 | shows the RNA sequence of PedH F412V, F459R, W561A mutant (RNA sequence encoding amino acid sequence of SEQ ID NO: 5) |
| SEQ ID NO: 69 | shows the RNA sequence of PedH F412V, F459R, W561A mutant (RNA sequence encoding amino acid sequence of SEQ ID NO: 6) |
| SEQ ID NO: 70 | shows the RNA sequence of PedH F412V, L560R, W561A mutant (RNA sequence encoding amino acid sequence of SEQ ID NO: 7) |
| SEQ ID NO: 71 | shows the RNA sequence of PedH F412V, L560R, W561A mutant (RNA sequence encoding amino acid sequence of SEQ ID NO: 8) |
| SEQ ID NO: 72 | shows the RNA sequence of PedH F412V, L560W, W561A mutant (RNA sequence encoding amino acid sequence of SEQ ID NO: 9) |
| SEQ ID NO: 73 | shows the RNA sequence of PedH F412V, L560W, W561A mutant (RNA sequence encoding amino acid sequence of SEQ ID NO: 10) |
| SEQ ID NO:74 | shows the RNA sequence of PedH F412V, L455W, W561A mutant (RNA sequence encoding amino acid sequence of SEQ ID NO: 11) |
| SEQ ID NO: 75 | shows the RNA sequence of PedH F412V, L455W, W561A mutant (RNA sequence encoding amino acid sequence of SEQ ID NO: 12) |
| SEQ ID NO: 76 | shows the RNA sequence of PedH F412V, L455R, W561A mutant (RNA sequence encoding amino acid sequence of SEQ ID NO: 13) |
| SEQ ID NO: 77 | shows the RNA sequence of PedH F412V, L455R, W561A mutant (RNA sequence encoding amino acid sequence of SEQ ID NO: 14) |
| SEQ ID NO: 78 | shows the RNA sequence of PedH F412V, F459W, L560R, W561A mutant (RNA sequence encoding amino acid sequence of SEQ ID NO: 15) |
| SEQ ID NO: 79 | shows the RNA sequence of PedH F412V, F459W, L560R, W561A mutant (RNA sequence encoding amino acid sequence of SEQ ID NO: 16) |
| SEQ ID NO: 80 | shows the RNA sequence of PedH F412V, F459R, L560W, W561A mutant (RNA sequence encoding amino acid sequence of SEQ ID NO: 17) |
| SEQ ID NO: 81 | shows the RNA sequence of PedH F412V, F459R, L560W, W561A mutant (RNA sequence encoding amino acid sequence of SEQ ID NO: 18) |
| SEQ ID NO: 82 | shows the RNA sequence of PedH F412V, F459W, L560W, W561A mutant (RNA sequence encoding amino acid sequence of SEQ ID NO: 19) |
| SEQ ID NO: 83 | shows the RNA sequence of PedH F412V, F459W, L560W, W561A mutant (RNA sequence encoding amino acid sequence of SEQ ID NO: 20) |
| SEQ ID NO: 84 | shows the RNA sequence of PedH F412V, F459R, L560R, W561A mutant (RNA sequence encoding amino acid sequence of SEQ ID NO: 21) |
| SEQ ID NO: 85 | shows the RNA sequence of PedH F412V, F459R, L560R, W561A mutant (RNA sequence encoding amino acid sequence of SEQ ID NO: 22) |
| SEQ ID NO: 86 | shows the RNA sequence of PedH F412V, L455W, L560R, W561A mutant (RNA sequence encoding amino acid sequence of SEQ ID NO: 23) |
| SEQ ID NO: 87 | shows the RNA sequence of PedH F412V, L455W, L560R, W561A mutant (RNA sequence encoding amino acid sequence of SEQ ID NO: 24) |
| SEQ ID NO: 88 | shows the RNA sequence of PedH F412V, L455R, L560W, W561A mutant (RNA sequence encoding amino acid sequence of SEQ ID NO: 25) |
| SEQ ID NO: 89 | shows the RNA sequence of PedH F412V, L455R, L560W, W561A mutant (RNA sequence encoding amino acid sequence of SEQ ID NO: 26) |
| SEQ ID NO: 90 | shows the RNA sequence of PedH F412V, L455W, L560W, W561A mutant (RNA sequence encoding amino acid sequence of SEQ ID NO: 27) |
| SEQ ID NO: 91 | shows the RNA sequence of PedH F412V, L455W, L560W, W561A mutant (RNA sequence encoding amino acid sequence of SEQ ID NO: 28) |
| SEQ ID NO: 92 | shows the RNA sequence of PedH F412V, L455R, L560R, W561A mutant (RNA sequence encoding amino acid sequence of SEQ ID NO: 29) |
| SEQ ID NO: 93 | shows the RNA sequence of PedH F412V, L455R, L560R, W561A mutant (RNA sequence encoding amino acid sequence of SEQ ID NO: 30 |
| SEQ ID NO: 94 | shows the DNA sequence of oligonucleotide TL_016 as described in Table 4 below |
| SEQ ID NO: 95 | shows the DNA sequence of oligonucleotide TL_017 as described in Table 4 below |
| SEQ ID NO: 96 | shows the DNA sequence of oligonucleotide TL_018 as described in Table 4 below |
| SEQ ID NO: 97 | shows the DNA sequence of oligonucleotide TL_019 as described in Table 4 below |
| SEQ ID NO: 98 | shows the DNA sequence of oligonucleotide TL_020 as described in Table 4 below |
| SEQ ID NO: 99 | shows the DNA sequence of oligonucleotide TL_021 as described in Table 4 below |
| SEQ ID NO: 100 | shows the DNA sequence of oligonucleotide TL_023 as described in Table 4 below |
| SEQ ID NO: 101 | shows the DNA sequence of oligonucleotide TL_022 as described in Table 4 below |
| SEQ ID NO: 102 | shows the DNA sequence of oligonucleotide TL_024 as described in Table 4 below |
| SEQ ID NO: 103 | shows the DNA sequence of oligonucleotide TL_025 as described in Table 4 below |
| SEQ ID NO: 104 | shows the DNA sequence of oligonucleotide TL_001 as described in Table 4 below |
| SEQ ID NO: 105 | shows the DNA sequence of oligonucleotide TL_002 as described in Table 4 below |
| SEQ ID NO: 106 | shows the DNA sequence of oligonucleotide TL_028 as described in Table 4 below |
| SEQ ID NO: 107 | shows the DNA sequence of oligonucleotide TL_027 as described in Table 4 below |
| SEQ ID NO: 108 | shows the DNA sequence of oligonucleotide oES_92 as described in Table 4 below |
| SEQ ID NO: 109 | shows the DNA sequence of oligonucleotide oES_93 as described in Table 4 below |
| SEQ ID NO: 110 | shows the DNA sequence of oligonucleotide oES_110 as described in Table 4 below |
| SEQ ID NO: 111 | shows the DNA sequence of oligonucleotide oES_111 as described in Table 4 below |
| SEQ ID NO: 112 | shows the DNA sequence of oligonucleotide oES_114 as described in Table 4 below |
| SEQ ID NO: 113 | shows the DNA sequence of oligonucleotide oES_115 as described in Table 4 below |
| SEQ ID NO: 114 | shows the DNA sequence of oligonucleotide oES_134 as described in Table 4 below |
| SEQ ID NO: 115 | shows the DNA sequence of oligonucleotide oES_135 as described in Table 4 below |
| SEQ ID NO: 116 | shows the DNA sequence of oligonucleotide oES_136 as described in Table 4 below |
| SEQ ID NO: 117 | shows the DNA sequence of oligonucleotide oES_137 as described in Table 4 below |
| SEQ ID NO: 118 | shows the DNA sequence of oligonucleotide oES_138 as described in Table 4 below |
| SEQ ID NO: 119 | shows the DNA sequence of oligonucleotide oES_139 as described in Table 4 below |
| SEQ ID NO: 120 | shows the DNA sequence of oligonucleotide oES_140 as described in Table 4 below |
| SEQ ID NO: 121 | shows the DNA sequence of oligonucleotide oES_141 as described in Table 4 below |
| SEQ ID NO: 122 | shows the DNA sequence of oligonucleotide oES_142 as described in Table 4 below |
| SEQ ID NO: 123 | shows the DNA sequence of oligonucleotide oES_143 as described in Table 4 below |
| SEQ ID NO: 124 | shows the DNA sequence of oligonucleotide oES_144 as described in Table 4 below |
| SEQ ID NO: 125 | shows the DNA sequence of oligonucleotide oES_145 as described in Table 4 below |
| SEQ ID NO: 126 | shows the DNA sequence of oligonucleotide oES_146 as described in Table 4 below |
| SEQ ID NO: 127 | shows the DNA sequence of oligonucleotide oES_147 as described in Table 4 below |
| SEQ ID NO: 128 | shows the DNA sequence encoding the amino acid sequence of the signal peptide (DNA sequence encoding amino acid sequence of SEQ ID NO: 31) |

An overview of the SEQ ID NOs and detailed sequences, as used in the context of the present invention, is given in the following Table **1.** The sequences in table 1 prevail.

**Table 1**

| **SE Q ID NO** | **Designation, notes** | **Sequence** |
|---|---|---|
| 1 | AA sequence of wildtype PedH with the signal peptide (underlined) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAFLGAKNWMPMAYNK DTGLFYVPSNEWGMDIWNEGIAYKKGAAFLGAGFTIKPLNEDYIGVLRAIDPVSGKEVWRHKNYAPLWGGVL TTKGNLVFTGTPEGFLQAFNAKTGDKVWEFQTGSGVLGSPVTWEMDGEQYVSVVSGWGGAVPLWGGEV AKRVKDFNQGGMLWTFKLPKQLQQTASVKP |
| 2 | AA sequence of wildtype PedH without the signal peptide | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAFLGAKNWMPMAYNKDTGLFYVPSNEWGMDIWNEGIAYKK GAAFLGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPLWGGEVAKRVKDFNQGGMLWTFKLPKQLQQ TASVKP |
| 3 | PedH F412V, F459W, W561A mutant without the signal | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT |
| | peptide (AA seq.) | IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGWTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPLAGGEVAKRVKDFNQGGMLWTFKLPKQLQQ TASVKP |
| 4 | PedH F412V, F459W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGWTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPLAGGEV AKRVKDFNQGGMLWTFKLPKQLQQTASVKP |
| 5 | PedH F412V, F459R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGRTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPLAGGEVAKRVKDFNQGGMLWTFKLPKQLQQT ASVKP |
| 6 | PedH F412V, F459R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGRTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPLAGGEV AKRVKDFNQGGMLWTFKLPKQLQQTASVKP |
| 7 | PedH F412V, L560R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPRAGGEVAKRVKDFNQGGMLWTFKLPKQLQQT ASVKP |
| 8 | PedH F412V, L560R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGFTIKPLNEDYIGVLRAIDPVSGKEVWRHKNYAPLWGGVL TTKGNLVFTGTPEGFLQAFNAKTGDKVWEFQTGSGVLGSPVTWEMDGEQYVSVVSGWGGAVPRAGGEVA KRVKDFNQGGMLWTFKLPKQLQQTASVKP |
| 9 | PedH F412V, L560W, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPWAGGEVAKRVKDFNQGGMLWTFKLPKQLQQ TASVKP |
| 10 | PedH F412V, L560W, W561A mutant with the signal peptide | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF |
| | (underlined) (AA seq.) | YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGFTIKPLNEDYIGVLRAIDPVSGKEVWRHKNYAPLWGGVL TTKGNLVFTGTPEGFLQAFNAKTGDKVWEFQTGSGVLGSPVTWEMDGEQYVSVVSGWGGAVPWAGGEV AKRVKDFNQGGMLWTFKLPKQLQQTASVKP |
| 11 | PedH F412V, L455W, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFWGAGFTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPLAGGEVAKRVKDFNQGGMLWTFKLPKQLQQ TASVKP |
| 12 | PedH F412V, L455W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFWGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPLAGGEV AKRVKDFNQGGMLWTFKLPKQLQQTASVKP |
| 13 | PedH F412V, L455R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFRGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPLAGGEVAKRVKDFNQGGMLWTFKLPKQLQQT ASVKP |
| 14 | PedH F412V, L455R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFRGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPLAGGEV AKRVKDFNQGGMLWTFKLPKQLQQTASVKP |
| 15 | PedH F412V, F459W, L560R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGWTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPRAGGEVAKRVKDFNQGGMLWTFKLPKQLQ QTASVKP |
| 16 | PedH F412V, F459W, L560R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGWTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPRAGGEV AKRVKDFNQGGMLWTFKLPKQLQQTASVKP |
| 17 | PedH F412V, F459R, L560W, W561A mutant without the | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL |
| | signal peptide (AA seq.) | DKDGRPIYNDASRPGAPGSEAKGSSVFVAPAVLGAKNWMPMAYNKDTGLFYVPSNEWGMDIWNEGIAYKK GAAFLGAGRTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPWAGGEVAKRVKDFNQGGMLWTFKLPKQLQQ TASVKP |
| 18 | PedH F412V, F459R, L560W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGRTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPWAGGE VAKRVKDFNQGGMLWTFKLPKQLQQTASVKP |
| 19 | PedH F412V, F459W, L560W, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGWTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPWAGGEVAKRVKDFNQGGMLWTFKLPKQLQ QTASVKP |
| 20 | PedH F412V, F459W, L560W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGWTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPWAGGE VAKRVKDFNQGGMLWTFKLPKQLQQTASVKP |
| 21 | PedH F412V, F459R, L560R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGRTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPRAGGEVAKRVKDFNQGGMLWTFKLPKQLQQT ASVKP |
| 22 | PedH F412V, F459R, L560R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFLGAGRTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPRAGGEV AKRVKDFNQGGMLWTFKLPKQLQQTASVKP |
| 23 | PedH F412V, L455W, L560R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFWGAGFTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPRAGGEVAKRVKDFNQGGMLWTFKLPKQLQ QTASVKP |
| 24 | PedH F412V, L455W, L560R, W561A mutant with the signal peptide | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF |
| | (underlined) (AA seq.) | YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFWGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPRAGGEV AKRVKDFNQGGMLWTFKLPKQLQQTASVKP |
| 25 | PedH F412V, L455R, L560W, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFRGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPWAGGEVAKRVKDFNQGGMLWTFKLPKQLQQ TASVKP |
| 26 | PedH F412V, L455R, L560W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFRGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPWAGGE VAKRVKDFNQGGMLWTFKLPKQLQQTASVKP |
| 27 | PedH F412V, L455R, L560W, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFWGAGFTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPWAGGEVAKRVKDFNQGGMLWTFKLPKQLQ QTASVKP |
| 28 | PedH F412V, L455R, L560W, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFWGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPWAGGE VAKRVKDFNQGGMLWTFKLPKQLQQTASVKP |
| 29 | PedH F412V, L455R, L560R, W561A mutant without the signal peptide (AA seq.) | AVSNEEILQDPKNPQQIVTNGLGVQGQRYSPLDLLNVNNVKELRPVWAFSFGGEKQRGQQAQPLIKDGVM YLTGSYSRVFAVDARTGKKLWQYDARLPDDIRPCCDVINRGVALYGNLVFFGTLDAKLVALNKDTGKVVWS KKVADHKEGYSISAAPMIVNGKLITGVAGGEFGVVGKIQAYNPENGELLWMRPTVEGHMGYVYKDGKAIEN GISGGEAGKTWPGDLWKTGGAAPWLGGYYDPETNLILFGTGNPAPWNSHLRPGDNLYSSSRLALNPDDGT IKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFRGAGFTIKPLNEDYIGVLRAIDPVSGKEVWRHKNYAPLWGGVLTTKGNLVFTGTPEGFLQAFNAKTGD KVWEFQTGSGVLGSPVTWEMDGEQYVSVVSGWGGAVPRAGGEVAKRVKDFNQGGMLWTFKLPKQLQQT ASVKP |
| 30 | PedH F412V, L455R, L560R, W561A mutant with the signal peptide (underlined) (AA seq.) | MTRSPRRPLFAVSLVLSAMLLAGAAHAAVSNEEILQDPKNPQQIVTNGLGVQGQRYSPLDLLNVNNVKELRP VWAFSFGGEKQRGQQAQPLIKDGVMYLTGSYSRVFAVDARTGKKLWQYDARLPDDIRPCCDVINRGVALY GNLVFFGTLDAKLVALNKDTGKVVWSKKVADHKEGYSISAAPMIVNGKLITGVAGGEFGVVGKIQAYNPENG ELLWMRPTVEGHMGYVYKDGKAIENGISGGEAGKTWPGDLWKTGGAAPWLGGYYDPETNLILFGTGNPAP WNSHLRPGDNLYSSSRLALNPDDGTIKWHFQSTPHDGWDFDGVNELISFNYKDGGKEVKAAATADRNGFF YVLDRTNGKFIRGFPFVDKITWATGLDKDGRPIYNDASRPGAPGSEAKGSSVFVAPAVLGAKNWMPMAYNK DTGLFYVPSNEWGMDIWNEGIAYKKGAAFRGAGFTIKPLNEDYIGVLRAIDPVSGKEVWRHKNYAPLWGGV LTTKGNLVFTGTPEGFLQAFNAKTGDKVWEFQTGSGVLGSPVTWEMDGEQYVSVVSGWGGAVPRAGGEV AKRVKDFNQGGMLWTFKLPKQLQQTASVKP |
| 31 | secretion signal peptide for targeting the periplasm | MTRSPRRPLFAVSLVLSAMLLAGAAHA |
| 32 | PedH F412V, F459W, W561A mutant (=SEQ ID NO: 3), with an N-terminal Met and a C-terminal 6xHis-tag (each underlined), without the signal peptide | MAVSNEEILQDPKNPQQIVTNGLGVQGQRYSPLDLLNVNNVKELRPVWAFSFGGEKQRGQQAQPLIKDGV MYLTGSYSRVFAVDARTGKKLWQYDARLPDDIRPCCDVINRGVALYGNLVFFGTLDAKLVALNKDTGKVVW SKKVADHKEGYSISAAPMIVNGKLITGVAGGEFGVVGKIQAYNPENGELLWMRPTVEGHMGYVYKDGKAIE NGISGGEAGKTWPGDLWKTGGAAPWLGGYYDPETNLILFGTGNPAPWNSHLRPGDNLYSSSRLALNPDDG TIKWHFQSTPHDGWDFDGVNELISFNYKDGGKEVKAAATADRNGFFYVLDRTNGKFIRGFPFVDKITWATGL DKDGRPIYNDASRPGAPGSEAKGSSVFVAPAVLGAKNWMPMAYNKDTGLFYVPSNEWGMDIWNEGIAYKK GAAFLGAGWTIKPLNEDYIGVLRAIDPVSGKEVWRHKNYAPLWGGVLTTKGNLVFTGTPEGFLQAFNAKTG DKVWEFQTGSGVLGSPVTWEMDGEQYVSVVSGWGGAVPLAGGEVAKRVKDFNQGGMLWTFKLPKQLQQ TASVKPHHHHHH |
| 33 | DNA seq. of wild type PedH (DNA seq. encoding AA seq. of SEQ ID NO: 1) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCTTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTTCACCATCAAGCCGCTCAATGAAGACTACATCGGCGTGC |
| | | TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGCTGTG GGGCGGCGAAGTGGCCAAACGGGTCAAGGACTTCAACCAGGGCGGCATGCTCTGGACCTTCAAGTTG CCCAAGCAGTTGCAGCAAACGGCAAGCGTCAAGCCATAA |
| 34 | DNA seq. of wild type PedH (DNA seq. encoding AA seq. of SEQ ID NO: 2) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCTTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCTGTGGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 35 | DNA seq. of PedH F412V, F459W, W561A mutant (DNA seq. encoding AA seq. of SEQ ID NO: 3) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGCTGT TCTACGTGCCGTCCAACGAGTGGGGCATGGACATCTGGAACGAAGGCATCGCCTATAAGAAAGGTGCG GCGTTCCTCGGTGCCGGCTGGACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCTGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 36 | DNA seq. of PedH F412V, F459W, W561A mutant (DNA seq. encoding | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT |
| | AA seq. of SEQ ID NO: 4) | CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTGGACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCTGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 37 | DNA seq. of PedH F412V, F459R, W561A mutant (DNA seq. encoding AA seq. of SEQ ID NO: 5) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC |
| | | CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCCGCACCATCAAGCCGCTCAATGAAGACTACATCGGCGTGCTGCGCGCCA TCGACCCGGTCAGCGGCAAGGAAGTGTGGCGCCACAAGAACTATGCGCCGCTGTGGGGCGGTGTGCT GACCACCAAGGGCAACCTGGTGTTCACGGGCACGCCAGAGGGCTTCCTGCAGGCATTCAACGCCAAG ACCGGCGACAAGGTCTGGGAATTCCAGACCGGCTCGGGCGTGCTCGGCTCGCCCGTCACCTGGGAAA TGGACGGCGAGCAATACGTTTCGGTAGTCTCCGGCTGGGGCGGCGCGGTGCCGCTGGCGGGCGGCG AAGTGGCCAAACGGGTCAAGGACTTCAACCAGGGCGGCATGCTCTGGACCTTCAAGTTGCCCAAGCAG TTGCAGCAAACGGCAAGCGTCAAGCCATAA |
| 38 | DNA seq. of PedH F412V, F459R, W561A mutant (DNA seq. encoding AA seq. of SEQ ID NO: 6) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA |
| | | GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCCGCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCTGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 39 | DNA seq. of PedH F412V, L560R, W561A mutant (DNA seq. encoding AA seq. of SEQ ID NO: 7) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA |
| | | CCGGCGACAAGGTCTGGGAATTCCAGACCGGCTCGGGCGTGCTCGGCTCGCCCGTCACCTGGAAAT GGACGGCGAGCAATACGTTTCGGTAGTCTCCGGCTGGGGCGGCGCGGTGCCGCGCGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 40 | DNA seq. of PedH F412V, L560R, W561A mutant (DNA seq. encoding AA seq. of SEQ ID NO: 8) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTTCACCATCAAGCCGCTCAATGAAGACTACATCGGCGTGC TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGCGCG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 41 | DNA seq. of PedH F412V, L560W, W561A mutant (DNA seq. encoding AA seq. of SEQ ID NO: 9) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGTGGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 42 | DNA seq. of PedH F412V, L560W, W561A mutant (DNA seq. encoding | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT |
| | AA seq. of SEQ ID NO: 10) | CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTTCACCATCAAGCCGCTCAATGAAGACTACATCGGCGTGC TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGTGGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 43 | DNA seq. of PedH F412V, L455W, W561A mutant (DNA seq. encoding AA seq. of SEQ ID NO: 11) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC |
| | | CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCTGGGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCTGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 44 | DNA seq. of PedH F412V, L455W, W561A mutant (DNA seq. encoding AA seq. of SEQ ID NO: 12) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA |
| | | GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCTGGGGTGCCGGCTTCACCATCAAGCCGCTCAATGAAGACTACATCGGCGTGC TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGCTGGC GGGCGGCGAAGTGGCCAAACGGGTCAAGGACTTCAACCAGGGCGGCATGCTCTGGACCTTCAAGTTG CCCAAGCAGTTGCAGCAAACGGCAAGCGTCAAGCCATAA |
| 45 | DNA seq. of PedH F412V, L455R, W561A mutant (DNA seq. encoding AA seq. of SEQ ID NO: 13) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCGCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGCGGTGTGCTG ACCACCAAGGGCAACCTGGTGTTCACGGGCACGCCAGAGGGCTTCCTGCAGGCATTCAACGCCAAGA |
| | | CCGGCGACAAGGTCTGGGAATTCCAGACCGGCTCGGGCGTGCTCGGCTCGCCCGTCACCTGGAAAT GGACGGCGAGCAATACGTTTCGGTAGTCTCCGGCTGGGGCGGCGCGGTGCCGCTGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 46 | DNA seq. of PedH F412V, L455R, W561A mutant (DNA seq. encoding AA seq. of SEQ ID NO: 14) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCGCGGTGCCGGCTTCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCTGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 47 | DNA seq. of PedH F412V, F459W, L560R, W561A mutant (DNA seq. encoding AA seq. of SEQ ID NO: 15) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGCTGT TCTACGTGCCGTCCAACGAGTGGGGCATGGACATCTGGAACGAAGGCATCGCCTATAAGAAAGGTGCG GCGTTCCTCGGTGCCGGCTGGACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCGCGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 48 | DNA seq. of PedH F412V, F459W, L560R, W561A mutant (DNA seq. encoding AA | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT |
| | seq. of SEQ ID NO: 16) | CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTGGACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCGC GCGGGCGGCGAAGTGGCCAAACGGGTCAAGGACTTCAACCAGGGCGGCATGCTCTGGACCTTCAAGT TGCCCAAGCAGTTGCAGCAAACGGCAAGCGTCAAGCCATAA |
| 49 | DNA seq. of PedH F412V, F459R, L560W, W561A mutant (DNA seq. encoding AA seq. of SEQ ID NO: 17) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC |
| | | CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCCGCACCATCAAGCCGCTCAATGAAGACTACATCGGCGTGCTGCGCGCCA TCGACCCGGTCAGCGGCAAGGAAGTGTGGCGCCACAAGAACTATGCGCCGCTGTGGGGCGGTGTGCT GACCACCAAGGGCAACCTGGTGTTCACGGGCACGCCAGAGGGCTTCCTGCAGGCATTCAACGCCAAG ACCGGCGACAAGGTCTGGGAATTCCAGACCGGCTCGGGCGTGCTCGGCTCGCCCGTCACCTGGGAAA TGGACGGCGAGCAATACGTTTCGGTAGTCTCCGGCTGGGGCGGCGCGGTGCCGTGGGCGGGCGGCG AAGTGGCCAAACGGGTCAAGGACTTCAACCAGGGCGGCATGCTCTGGACCTTCAAGTTGCCCAAGCAG TTGCAGCAAACGGCAAGCGTCAAGCCATAA |
| 50 | DNA seq. of PedH F412V, F459R, L560W, W561A mutant (DNA seq. encoding AA seq. of SEQ ID NO: 18) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA |
| | | GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCCGCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGTGGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 51 | DNA seq. of PedH F412V, F459W, L560W, W561A mutant (DNA seq. encoding AA seq. of SEQ ID NO: 19 | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCTCGGTGCCGGCTGGACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA |
| | | CCGGCGACAAGGTCTGGGAATTCCAGACCGGCTCGGGCGTGCTCGGCTCGCCCGTCACCTGGAAAT GGACGGCGAGCAATACGTTTCGGTAGTCTCCGGCTGGGGCGGCGCGGTGCCGTGGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 52 | DNA seq. of PedH F412V, F459W, L560W, W561A mutant (DNA seq. encoding AA seq. of SEQ ID NO: 20) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCTGGACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGTGGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 53 | DNA seq. of PedH F412V, F459R, L560R, W561A mutant (DNA seq. encoding AA seq. of SEQ ID NO: 21) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGCTGT TCTACGTGCCGTCCAACGAGTGGGGCATGGACATCTGGAACGAAGGCATCGCCTATAAGAAAGGTGCG GCGTTCCTCGGTGCCGGCCGCACCATCAAGCCGCTCAATGAAGACTACATCGGCGTGCTGCGCGCCA TCGACCCGGTCAGCGGCAAGGAAGTGTGGCGCCACAAGAACTATGCGCCGCTGTGGGCGGTGTGCT GACCACCAAGGGCAACCTGGTGTTCACGGGCACGCCAGAGGGCTTCCTGCAGGCATTCAACGCCAAG ACCGGCGACAAGGTCTGGGAATTCCAGACCGGCTCGGGCGTGCTCGGCTCGCCCGTCACCTGGGAAA TGGACGGCGAGCAATACGTTTCGGTAGTCTCCGGCTGGGGCGGCGCGGTGCCGCGCGCGGGCGGCG AAGTGGCCAAACGGGTCAAGGACTTCAACCAGGGCGGCATGCTCTGGACCTTCAAGTTGCCCAAGCAG TTGCAGCAAACGGCAAGCGTCAAGCCATAA |
| 54 | DNA seq. of PedH F412V, F459R, L560R, W561A mutant (DNA seq. encoding AA | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT |
| | seq. of SEQ ID NO: 22) | CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCTCGGTGCCGGCCGCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCGC GCGGGCGGCGAAGTGGCCAAACGGGTCAAGGACTTCAACCAGGGCGGCATGCTCTGGACCTTCAAGT TGCCCAAGCAGTTGCAGCAAACGGCAAGCGTCAAGCCATAA |
| 55 | DNA seq. of PedH F412V, L455W, L560R, W561A mutant (DNA seq. encoding AA seq. of SEQ ID NO: 23) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC |
| | | CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCTGGGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCGCGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 56 | DNA seq. of PedH F412V, L455W, L560R, W561A mutant (DNA seq. encoding AA seq. of SEQ ID NO: 24) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA |
| | | GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCTGGGGTGCCGGCTTCACCATCAAGCCGCTCAATGAAGACTACATCGGCGTGC TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGCGCG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 57 | DNA seq. of PedH F412V, L455R, L560W, W561A mutant (DNA seq. encoding AA seq. of SEQ ID NO: 25) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCGCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA |
| | | CCGGCGACAAGGTCTGGGAATTCCAGACCGGCTCGGGCGTGCTCGGCTCGCCCGTCACCTGGAAAT GGACGGCGAGCAATACGTTTCGGTAGTCTCCGGCTGGGGCGGCGCGGTGCCGTGGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 58 | DNA seq. of PedH F412V, L455R, L560W, W561A mutant (DNA seq. encoding AA seq. of SEQ ID NO: 26) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCGCGGTGCCGGCTTCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGTGGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 59 | DNA seq. of PedH F412V, L455W, L560W, W561A mutant (DNA seq. encoding AA seq. of SEQ ID NO: 27) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGCTGT TCTACGTGCCGTCCAACGAGTGGGGCATGGACATCTGGAACGAAGGCATCGCCTATAAGAAGGTGCG GCGTTCTGGGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGTGGGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 60 | DNA seq. of PedH F412V, L455W, L560W, W561A mutant (DNA seq. encoding AA | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT |
| | seq. of SEQ ID NO: 28) | CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCTGGGGTGCCGGCTTCACCATCAAGCCGCTCAATGAAGACTACATCGGCGTGC TGCGCGCCATCGACCCGGTCAGCGGCAAGGAAGTGTGGCGCCACAAGAACTATGCGCCGCTGTGGGG CGGTGTGCTGACCACCAAGGGCAACCTGGTGTTCACGGGCACGCCAGAGGGCTTCCTGCAGGCATTC AACGCCAAGACCGGCGACAAGGTCTGGGAATTCCAGACCGGCTCGGGCGTGCTCGGCTCGCCCGTCA CCTGGGAAATGGACGGCGAGCAATACGTTTCGGTAGTCTCCGGCTGGGGCGGCGCGGTGCCGTGGG CGGGCGGCGAAGTGGCCAAACGGGTCAAGGACTTCAACCAGGGCGGCATGCTCTGGACCTTCAAGTT GCCCAAGCAGTTGCAGCAAACGGCAAGCGTCAAGCCATAA |
| 61 | DNA seq. of PedH F412V, L455R, L560R, W561A mutant (DNA seq. encoding AA seq. of SEQ ID NO: 29) | GCTGTCAGCAATGAAGAAATCCTCCAGGACCCGAAGAACCCGCAGCAGATCGTGACCAATGGCCTGGG CGTGCAGGGCCAGCGCTACAGCCCGCTGGACCTGCTCAATGTCAATAACGTCAAGGAGCTGCGCCCG GTCTGGGCGTTCTCCTTCGGCGGGGAGAAGCAGCGCGGCCAGCAGGCCCAGCCGCTGATCAAGGAC GGGGTGATGTACCTGACCGGCTCCTACTCGCGGGTGTTCGCCGTGGATGCCCGCACCGGCAAGAAAC TGTGGCAATACGATGCACGTCTGCCGGATGACATCCGCCCCTGCTGCGACGTAATCAACCGCGGCGTC GCGCTGTACGGCAACCTGGTGTTCTTCGGCACGCTGGACGCCAAGCTGGTGGCCCTGAACAAGGACA CCGGCAAGGTGGTCTGGAGCAAGAAGGTCGCCGACCACAAAGAAGGCTACTCCATCAGCGCCGCGCC GATGATCGTCAATGGCAAGCTGATCACAGGCGTTGCCGGCGGCGAGTTCGGCGTGGTGGGCAAGATC CAGGCGTACAACCCGGAGAACGGCGAACTGCTGTGGATGCGCCCCACCGTGGAAGGGCACATGGGCT ATGTGTACAAGGATGGCAAGGCGATCGAGAACGGTATTTCCGGCGGTGAGGCGGGCAAGACCTGGCC TGGCGACCTGTGGAAGACCGGCGGCGCCGCGCCGTGGCTGGGGGGTTACTACGACCCTGAAACCAAC |
| | | CTGATCCTGTTTGGTACCGGTAACCCGGCGCCGTGGAACTCGCACCTGCGCCCCGGTGACAACCTGTA CTCCTCCTCACGCCTGGCACTGAACCCGGACGACGGCACCATCAAGTGGCACTTCCAGAGCACGCCG CATGACGGCTGGGACTTCGACGGCGTCAACGAGCTGATCTCGTTCAACTACAAGGACGGCGGCAAGG AGGTCAAGGCTGCCGCCACGGCAGACCGCAACGGTTTCTTCTACGTGCTCGACCGCACCAACGGCAA GTTCATCCGCGGCTTCCCCTTCGTGGACAAGATCACCTGGGCCACTGGCCTGGACAAGGACGGCCGG CCGATCTACAACGACGCCAGCCGCCCGGGCGCACCCGGCAGCGAGGCCAAGGGCAGCTCGGTGTTC GTCGCGCCGGCCGTCCTCGGCGCCAAGAACTGGATGCCGATGGCCTACAACAAGGACACAGGGCTGT TCTACGTGCCGTCCAACGAGTGGGGCATGGACATCTGGAACGAAGGCATCGCCTATAAGAAAGGTGCG GCGTTCCGCGGTGCCGGCTTCACCATCAAGCCGCTCAATGAAGACTACATCGGCGTGCTGCGCGCCAT CGACCCGGTCAGCGGCAAGGAAGTGTGGCGCCACAAGAACTATGCGCCGCTGTGGGGCGGTGTGCTG ACCACCAAGGGCAACCTGGTGTTCACGGGCACGCCAGAGGGCTTCCTGCAGGCATTCAACGCCAAGA CCGGCGACAAGGTCTGGGAATTCCAGACCGGCTCGGGCGTGCTCGGCTCGCCCGTCACCTGGGAAAT GGACGGCGAGCAATACGTTTCGGTAGTCTCCGGCTGGGGCGGCGCGGTGCCGCGCGCGGGCGGCGA AGTGGCCAAACGGGTCAAGGACTTCAACCAGGGCGGCATGCTCTGGACCTTCAAGTTGCCCAAGCAGT TGCAGCAAACGGCAAGCGTCAAGCCATAA |
| 62 | DNA seq. of PedH F412V, L455R, L560R, W561A mutant (DNA seq. encoding AA seq. of SEQ ID NO: 30) | ATGACCCGATCCCCACGTCGCCCCTTGTTCGCCGTGAGCCTGGTGCTCAGCGCCATGCTGCTTGCCGG CGCGGCTCACGCCGCTGTCAGCAATGAAGAAATCCTCCAGGACCCGAAGAACCCGCAGCAGATCGTG ACCAATGGCCTGGGCGTGCAGGGCCAGCGCTACAGCCCGCTGGACCTGCTCAATGTCAATAACGTCAA GGAGCTGCGCCCGGTCTGGGCGTTCTCCTTCGGCGGGGAGAAGCAGCGCGGCCAGCAGGCCCAGCC GCTGATCAAGGACGGGGTGATGTACCTGACCGGCTCCTACTCGCGGGTGTTCGCCGTGGATGCCCGC ACCGGCAAGAAACTGTGGCAATACGATGCACGTCTGCCGGATGACATCCGCCCCTGCTGCGACGTAAT CAACCGCGGCGTCGCGCTGTACGGCAACCTGGTGTTCTTCGGCACGCTGGACGCCAAGCTGGTGGCC CTGAACAAGGACACCGGCAAGGTGGTCTGGAGCAAGAAGGTCGCCGACCACAAAGAAGGCTACTCCAT CAGCGCCGCGCCGATGATCGTCAATGGCAAGCTGATCACAGGCGTTGCCGGCGGCGAGTTCGGCGTG GTGGGCAAGATCCAGGCGTACAACCCGGAGAACGGCGAACTGCTGTGGATGCGCCCCACCGTGGAAG GGCACATGGGCTATGTGTACAAGGATGGCAAGGCGATCGAGAACGGTATTTCCGGCGGTGAGGCGGG CAAGACCTGGCCTGGCGACCTGTGGAAGACCGGCGGCGCCGCGCCGTGGCTGGGGGGTTACTACGA CCCTGAAACCAACCTGATCCTGTTTGGTACCGGTAACCCGGCGCCGTGGAACTCGCACCTGCGCCCCG GTGACAACCTGTACTCCTCCTCACGCCTGGCACTGAACCCGGACGACGGCACCATCAAGTGGCACTTC CAGAGCACGCCGCATGACGGCTGGGACTTCGACGGCGTCAACGAGCTGATCTCGTTCAACTACAAGGA CGGCGGCAAGGAGGTCAAGGCTGCCGCCACGGCAGACCGCAACGGTTTCTTCTACGTGCTCGACCGC ACCAACGGCAAGTTCATCCGCGGCTTCCCCTTCGTGGACAAGATCACCTGGGCCACTGGCCTGGACAA |
| | | GGACGGCCGGCCGATCTACAACGACGCCAGCCGCCCGGGCGCACCCGGCAGCGAGGCCAAGGGCAG CTCGGTGTTCGTCGCGCCGGCCGTCCTCGGCGCCAAGAACTGGATGCCGATGGCCTACAACAAGGAC ACAGGGCTGTTCTACGTGCCGTCCAACGAGTGGGGCATGGACATCTGGAACGAAGGCATCGCCTATAA GAAAGGTGCGGCGTTCCGCGGTGCCGGCTTCACCATCAAGCCGCTCAATGAAGACTACATCGGCGTG CTGCGCGCCATCGACCCGGTCAGCGGCAAGGAAGTGTGGCGCCACAAGAACTATGCGCCGCTGTGGG GCGGTGTGCTGACCACCAAGGGCAACCTGGTGTTCACGGGCACGCCAGAGGGCTTCCTGCAGGCATT CAACGCCAAGACCGGCGACAAGGTCTGGGAATTCCAGACCGGCTCGGGCGTGCTCGGCTCGCCCGTC ACCTGGGAAATGGACGGCGAGCAATACGTTTCGGTAGTCTCCGGCTGGGGCGGCGCGGTGCCGCGC GCGGGCGGCGAAGTGGCCAAACGGGTCAAGGACTTCAACCAGGGCGGCATGCTCTGGACCTTCAAGT TGCCCAAGCAGTTGCAGCAAACGGCAAGCGTCAAGCCATAA |
| 63 | DNA seq. of PedH F412V, F459W, W561A mutant (DNA seq. encoding AA seq. of SEQ ID NO: 32) (seq coding for N-terminal Met and C-terminal 6xHis are underlinded) | ATGGCTGTCAGCAATGAAGAAATCCTCCAGGACCCGAAGAACCCGCAGCAGATCGTGACCAATGGCCT GGGCGTGCAGGGCCAGCGCTACAGCCCGCTGGACCTGCTCAATGTCAATAACGTCAAGGAGCTGCGC CCGGTCTGGGCGTTCTCCTTCGGCGGGGAGAAGCAGCGCGGCCAGCAGGCCCAGCCGCTGATCAAG GACGGGGTGATGTACCTGACCGGCTCCTACTCGCGGGTGTTCGCCGTGGATGCCCGCACCGGCAAGA AACTGTGGCAATACGATGCACGTCTGCCGGATGACATCCGCCCCTGCTGCGACGTAATCAACCGCGGC GTCGCGCTGTACGGCAACCTGGTGTTCTTCGGCACGCTGGACGCCAAGCTGGTGGCCCTGAACAAGG ACACCGGCAAGGTGGTCTGGAGCAAGAAGGTCGCCGACCACAAAGAAGGCTACTCCATCAGCGCCGC GCCGATGATCGTCAATGGCAAGCTGATCACAGGCGTTGCCGGCGGCGAGTTCGGCGTGGTGGGCAAG ATCCAGGCGTACAACCCGGAGAACGGCGAACTGCTGTGGATGCGCCCCACCGTGGAAGGGCACATGG GCTATGTGTACAAGGATGGCAAGGCGATCGAGAACGGTATTTCCGGCGGTGAGGCGGGCAAGACCTG GCCTGGCGACCTGTGGAAGACCGGCGGCGCCGCGCCGTGGCTGGGGGGTTACTACGACCCTGAAAC CAACCTGATCCTGTTTGGTACCGGTAACCCGGCGCCGTGGAACTCGCACCTGCGCCCCGGTGACAACC TGTACTCCTCCTCACGCCTGGCACTGAACCCGGACGACGGCACCATCAAGTGGCACTTCCAGAGCACG CCGCATGACGGCTGGGACTTCGACGGCGTCAACGAGCTGATCTCGTTCAACTACAAGGACGGCGGCA AGGAGGTCAAGGCTGCCGCCACGGCAGACCGCAACGGTTTCTTCTACGTGCTCGACCGCACCAACGG CAAGTTCATCCGCGGCTTCCCCTTCGTGGACAAGATCACCTGGGCCACTGGCCTGGACAAGGACGGCC GGCCGATCTACAACGACGCCAGCCGCCCGGGCGCACCCGGCAGCGAGGCCAAGGGCAGCTCGGTGT TCGTCGCGCCGGCCGTCCTCGGCGCCAAGAACTGGATGCCGATGGCCTACAACAAGGACACAGGGCT GTTCTACGTGCCGTCCAACGAGTGGGGCATGGACATCTGGAACGAAGGCATCGCCTATAAGAAAGGTG CGGCGTTCCTCGGTGCCGGCTGGACCATCAAGCCGCTCAATGAAGACTACATCGGCGTGCTGCGCGC CATCGACCCGGTCAGCGGCAAGGAAGTGTGGCGCCACAAGAACTATGCGCCGCTGTGGGCGGTGTG CTGACCACCAAGGGCAACCTGGTGTTCACGGGCACGCCAGAGGGCTTCCTGCAGGCATTCAACGCCA |
| | | AGACCGGCGACAAGGTCTGGGAATTCCAGACCGGCTCGGGCGTGCTCGGCTCGCCCGTCACCTGGGA AATGGACGGCGAGCAATACGTTTCGGTAGTCTCCGGCTGGGGCGGCGCGGTGCCGCTGGCGGGCGG CGAAGTGGCCAAACGGGTCAAGGACTTCAACCAGGGCGGCATGCTCTGGACCTTCAAGTTGCCCAAGC AGTTGCAGCAAACGGCAAGCGTCAAGCCACATCATCACCATCACCACTAA |
| 64 | RNA seq. of wild type PedH (RNA seq. encoding AA seq. of SEQ ID NO: 1) | AUGACCCGAUCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCUUCCUCGGCGCCAA GAACUGGAUGCCGAUGGCCUACAACAAGGACACAGGGCUGUUCUACGUGCCGUCCAACGAGUGGG CAUGGACAUCUGGAACGAAGGCAUCGCCUAUAAGAAAGGUGCGGCGUUCCUCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCUGUGGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 65 | RNA seq. of wild type PedH (RNA seq. encoding AA seq. of SEQ ID NO: 2) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCUUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCUGUGGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 66 | RNA seq. of PedH F412V, F459W, W561A mutant (RNA seq. encoding | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC |
| | AA seq. of SEQ ID NO: 3) | GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCUGGACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCUGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 67 | RNA seq. of PedH F412V, F459W, W561A mutant (RNA seq. encoding AA seq. of SEQ ID NO: 4) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA |
| | | UGCGCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCUGGAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCUGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 68 | RNA seq. of PedH F412V, F459R, W561A mutant (RNA seq. encoding AA seq. of SEQ ID NO: 5) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU |
| | | UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCCGCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCUGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 69 | RNA seq. of PedH F412V, F459R, W561A mutant (RNA seq. encoding AA seq. of SEQ ID NO: 6) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA |
| | | GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCCGCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCUGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 70 | RNA seq. of PedH F412V, L560R, W561A mutant (RNA seq. encoding AA seq. of SEQ ID NO: 7) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG |
| | | CUCGGGCGUGCUCGGCUCGCCCGUCACCUGGGAAAUGGACGGCGAGCAAUACGUUUCGGUAGUCU CCGGCUGGGGCGGCGCGGUGCCGCGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 71 | RNA seq. of PedH F412V, L560R, W561A mutant (RNA seq. encoding AA seq. of SEQ ID NO: 8) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCGCGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 72 | RNA seq. of PedH F412V, L560W, W561A mutant (RNA seq. encoding AA seq. of SEQ ID NO: 9) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGCAUGGACAUCUGG AACGAAGGCAUCGCCUAUAAGAAAGGUGCGGCGUUCCUCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGUGGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 73 | RNA seq. of PedH F412V, L560W, W561A mutant (RNA seq. encoding | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG |
| | AA seq. of SEQ ID NO: 10) | GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGAAAUGGACGGCGAGCAAUAC GUUUCGGUAGUCUCCGGCUGGGGCGGCGCGGUGCCGUGGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 74 | RNA seq. of PedH F412V, L455W, W561A mutant (RNA seq. encoding AA seq. of SEQ ID NO: 11) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG |
| | | AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCUGGGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCUGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 75 | RNA seq. of PedH F412V, L455W, W561A mutant (RNA seq. encoding AA seq. of SEQ ID NO: 12) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC |
| | | GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCUGGGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCUGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 76 | RNA seq. of PedH F412V, L455R, W561A mutant (RNA seq. encoding AA seq. of SEQ ID NO: 13) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG |
| | | AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCGCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCUGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 77 | RNA seq. of PedH F412V, L455R, W561A mutant (RNA seq. encoding AA seq. of SEQ ID NO: 14) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGCUGUUCUACGUGCCGUCCAACGAGUGGGG CAUGGACAUCUGGAACGAAGGCAUCGCCUAUAAGAAAGGUGCGGCGUUCCGCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG |
| | | UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGAAAUGGACGGCGAGCAAUAC GUUUCGGUAGUCUCCGGCUGGGGCGGCGCGGUGCCGCUGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 78 | RNA seq. of PedH F412V, F459W, L560R, W561A mutant (RNA seq. encoding AA seq. of SEQ ID NO: 15) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCUGGACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCGCGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 79 | RNA seq. of PedH F412V, F459W, L560R, W561A mutant (RNA seq. encoding AA seq. of SEQ ID NO: 16) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCUGGAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCGCGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 80 | RNA seq. of PedH F412V, F459R, L560W, W561A mutant | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA |
| | (RNA seq. encoding AA seq. of SEQ ID NO: 17) | AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGCAUGGACAUCUGG AACGAAGGCAUCGCCUAUAAGAAAGGUGCGGCGUUCCUCGGUGCCGGCCGCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGUGGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 81 | RNA seq. of PedH F412V, F459R, L560W, W561A mutant (RNA seq. encoding AA seq. of SEQ ID NO: 18) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG |
| | | CGGCGAGUUCGGCGUGGUGGGCAAGAUCCAGGCGUACAACCCGGAGAACGGCGAACUGCUGUGGA UGCGCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCCGCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGUGGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 82 | RNA seq. of PedH F412V, F459W, L560W, W561A mutant (RNA seq. encoding AA seq. of SEQ ID NO: 19 | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA |
| | | AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCUGGACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGUGGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 83 | RNA seq. of PedH F412V, F459W, L560W, W561A mutant (RNA seq. encoding AA seq. of SEQ ID NO: 20) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG |
| | | GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCUCGGUGCCGGCUGGAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGUGGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 84 | RNA seq. of PedH F412V, F459R, L560R, W561A mutant (RNA seq. encoding AA seq. of SEQ ID NO: 21) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCUCGGUGCCGGCCGCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC |
| | | GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGAAAUGGACGGCGAGCAAUACGUUUCGGUAGUCU CCGGCUGGGGCGGCGCGGUGCCGCGCGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 85 | RNA seq. of PedH F412V, F459R, L560R, W561A mutant (RNA seq. encoding AA seq. of SEQ ID NO: 22) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGG CAUGGACAUCUGGAACGAAGGCAUCGCCUAUAAGAAAGGUGCGGCGUUCCUCGGUGCCGGCCGCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCGCGCGGGCGGCGAAGUGGCCAAACGGG |
| | | UCAAGGACUUCAACCAGGGCGGCAUGCUCUGGACCUUCAAGUUGCCCAAGCAGUUGCAGCAAACGG CAAGCGUCAAGCCAUAA |
| 86 | RNA seq. of PedH F412V, L455W, L560R, W561A mutant (RNA seq. encoding AA seq. of SEQ ID NO: 23) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGCAUGGACAUCUGG AACGAAGGCAUCGCCUAUAAGAAAGGUGCGGCGUUCUGGGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCGCGCGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 87 | RNA seq. of PedH F412V, L455W, L560R, | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA |
| | W561A mutant (RNA seq. encoding AA seq. of SEQ ID NO: 24) | CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCUGGGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCGCGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 88 | RNA seq. of PedH F412V, L455R, L560W, W561A mutant (RNA seq. encoding AA | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC |
| | seq. of SEQ ID NO: 25) | AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCGCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGUGGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 89 | RNA seq. of PedH F412V, L455R, L560W, W561A mutant (RNA seq. encoding AA seq. of SEQ ID NO: 26) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG |
| | | UGGCUGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCGCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGUGGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 90 | RNA seq. of PedH F412V, L455W, L560W, W561A mutant (RNA seq. encoding AA seq. of SEQ ID NO: 27) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG |
| | | CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCUGGGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGUGGGCGGGCGGCGAAGUGGCCAAACGGGUCAAGGACUUCAAC CAGGGCGGCAUGCUCUGGACCUUCAAGUUGCCCAAGCAGUUGCAGCAAACGGCAAGCGUCAAGCCA UAA |
| 91 | RNA seq. of PedH F412V, L455W, L560W, W561A mutant (RNA seq. encoding AA seq. of SEQ ID NO: 28) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCUGGGGUGCCGGCUUCAC |
| | | CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGUGGGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 92 | RNA seq. of PedH F412V, L455R, L560R, W561A mutant (RNA seq. encoding AA seq. of SEQ ID NO: 29) | GCUGUCAGCAAUGAAGAAAUCCUCCAGGACCCGAAGAACCCGCAGCAGAUCGUGACCAAUGGCCUG GGCGUGCAGGGCCAGCGCUACAGCCCGCUGGACCUGCUCAAUGUCAAUAACGUCAAGGAGCUGCGC CCGGUCUGGGCGUUCUCCUUCGGCGGGGAGAAGCAGCGCGGCCAGCAGGCCCAGCCGCUGAUCAA GGACGGGGUGAUGUACCUGACCGGCUCCUACUCGCGGGUGUUCGCCGUGGAUGCCCGCACCGGCA AGAAACUGUGGCAAUACGAUGCACGUCUGCCGGAUGACAUCCGCCCCUGCUGCGACGUAAUCAACC GCGGCGUCGCGCUGUACGGCAACCUGGUGUUCUUCGGCACGCUGGACGCCAAGCUGGUGGCCCUG AACAAGGACACCGGCAAGGUGGUCUGGAGCAAGAAGGUCGCCGACCACAAAGAAGGCUACUCCAUC AGCGCCGCGCCGAUGAUCGUCAAUGGCAAGCUGAUCACAGGCGUUGCCGGCGGCGAGUUCGGCGU GGUGGGCAAGAUCCAGGCGUACAACCCGGAGAACGGCGAACUGCUGUGGAUGCGCCCCACCGUGG AAGGGCACAUGGGCUAUGUGUACAAGGAUGGCAAGGCGAUCGAGAACGGUAUUUCCGGCGGUGAG GCGGGCAAGACCUGGCCUGGCGACCUGUGGAAGACCGGCGGCGCCGCGCCGUGGCUGGGGGGUU ACUACGACCCUGAAACCAACCUGAUCCUGUUUGGUACCGGUAACCCGGCGCCGUGGAACUCGCACC UGCGCCCCGGUGACAACCUGUACUCCUCCUCACGCCUGGCACUGAACCCGGACGACGGCACCAUCA AGUGGCACUUCCAGAGCACGCCGCAUGACGGCUGGGACUUCGACGGCGUCAACGAGCUGAUCUCGU UCAACUACAAGGACGGCGGCAAGGAGGUCAAGGCUGCCGCCACGGCAGACCGCAACGGUUUCUUCU ACGUGCUCGACCGCACCAACGGCAAGUUCAUCCGCGGCUUCCCCUUCGUGGACAAGAUCACCUGGG CCACUGGCCUGGACAAGGACGGCCGGCCGAUCUACAACGACGCCAGCCGCCCGGGCGCACCCGGCA GCGAGGCCAAGGGCAGCUCGGUGUUCGUCGCGCCGGCCGUCCUCGGCGCCAAGAACUGGAUGCCG AUGGCCUACAACAAGGACACAGGGCUGUUCUACGUGCCGUCCAACGAGUGGGGCAUGGACAUCUGG AACGAAGGCAUCGCCUAUAAGAAAGGUGCGGCGUUCCGCGGUGCCGGCUUCACCAUCAAGCCGCUC AAUGAAGACUACAUCGGCGUGCUGCGCGCCAUCGACCCGGUCAGCGGCAAGGAAGUGUGGCGCCAC AAGAACUAUGCGCCGCUGUGGGGCGGUGUGCUGACCACCAAGGGCAACCUGGUGUUCACGGGCAC GCCAGAGGGCUUCCUGCAGGCAUUCAACGCCAAGACCGGCGACAAGGUCUGGGAAUUCCAGACCGG CUCGGGCGUGCUCGGCUCGCCCGUCACCUGGGAAAUGGACGGCGAGCAAUACGUUUCGGUAGUCU CCGGCUGGGGCGGCGCGGUGCCGCGCGCGGCGGCGAAGUGGCCAAACGGGUCAAGGACUUCAAC |
| | | CAGGGCGGCAUGCUCUGGACCUUCAAGUUGCCCAAGCAGUUGCAGCAAACGGCAAGCGUCAAGCCA UAA |
| 93 | RNA seq. of PedH F412V, L455R, L560R, W561A mutant (RNA seq. encoding AA seq. of SEQ ID NO: 30) | AUGACCCGAUCCCCACGUCGCCCCUUGUUCGCCGUGAGCCUGGUGCUCAGCGCCAUGCUGCUUGC CGGCGCGGCUCACGCCGCUGUCAGCAAUGAAGAAAUCCUCCAGGACCCGAAGAACCCGCAGCAGAU CGUGACCAAUGGCCUGGGCGUGCAGGGCCAGCGCUACAGCCCGCUGGACCUGCUCAAUGUCAAUAA CGUCAAGGAGCUGCGCCCGGUCUGGGCGUUCUCCUUCGGCGGGGAGAAGCAGCGCGGCCAGCAGG CCCAGCCGCUGAUCAAGGACGGGGUGAUGUACCUGACCGGCUCCUACUCGCGGGUGUUCGCCGUG GAUGCCCGCACCGGCAAGAAACUGUGGCAAUACGAUGCACGUCUGCCGGAUGACAUCCGCCCCUGC UGCGACGUAAUCAACCGCGGCGUCGCGCUGUACGGCAACCUGGUGUUCUUCGGCACGCUGGACGC CAAGCUGGUGGCCCUGAACAAGGACACCGGCAAGGUGGUCUGGAGCAAGAAGGUCGCCGACCACAA AGAAGGCUACUCCAUCAGCGCCGCGCCGAUGAUCGUCAAUGGCAAGCUGAUCACAGGCGUUGCCGG CGGCGAGUUCGGCGUGGUGGGCAAGAUCCAGGCGUACAACCCGGAGAACGGCGAACUGCUGUGGA UGCGCCCCACCGUGGAAGGGCACAUGGGCUAUGUGUACAAGGAUGGCAAGGCGAUCGAGAACGGUA UUUCCGGCGGUGAGGCGGGCAAGACCUGGCCUGGCGACCUGUGGAAGACCGGCGGCGCCGCGCCG UGGCUGGGGGGUUACUACGACCCUGAAACCAACCUGAUCCUGUUUGGUACCGGUAACCCGGCGCCG UGGAACUCGCACCUGCGCCCCGGUGACAACCUGUACUCCUCCUCACGCCUGGCACUGAACCCGGAC GACGGCACCAUCAAGUGGCACUUCCAGAGCACGCCGCAUGACGGCUGGGACUUCGACGGCGUCAAC GAGCUGAUCUCGUUCAACUACAAGGACGGCGGCAAGGAGGUCAAGGCUGCCGCCACGGCAGACCGC AACGGUUUCUUCUACGUGCUCGACCGCACCAACGGCAAGUUCAUCCGCGGCUUCCCCUUCGUGGAC AAGAUCACCUGGGCCACUGGCCUGGACAAGGACGGCCGGCCGAUCUACAACGACGCCAGCCGCCCG GGCGCACCCGGCAGCGAGGCCAAGGGCAGCUCGGUGUUCGUCGCGCCGGCCGUCCUCGGCGCCAA GAACUGGAUGCCGAUGGCCUACAACAAGGACACAGGGCUGUUCUACGUGCCGUCCAACGAGUGGGG CAUGGACAUCUGGAACGAAGGCAUCGCCUAUAAGAAAGGUGCGGCGUUCCGCGGUGCCGGCUUCAC CAUCAAGCCGCUCAAUGAAGACUACAUCGGCGUGCUGCGCGCCAUCGACCCGGUCAGCGGCAAGGA AGUGUGGCGCCACAAGAACUAUGCGCCGCUGUGGGGCGGUGUGCUGACCACCAAGGGCAACCUGG UGUUCACGGGCACGCCAGAGGGCUUCCUGCAGGCAUUCAACGCCAAGACCGGCGACAAGGUCUGGG AAUUCCAGACCGGCUCGGGCGUGCUCGGCUCGCCCGUCACCUGGGAAAUGGACGGCGAGCAAUAC GUUUCGGUAGUCUCCGGCUGGGGCGGCGCGGUGCCGCGCGCGGGCGGCGAAGUGGCCAAACGGG UCAAGGACUUCAACCAGGGCGGCAUGCUCUGGACCUUCAAGUUGCCCAAGCAGUUGCAGCAAACGG CAAGCGUCAAGCCAUAA |
| 94 | DNA seq. of oligonucleotide TL_016 | AGTATAGGGATAACAGGGTAATCTGGGCAAGAAACTGTGGCAATAC |
| 95 | DNA seq. of oligonucleotide TL_017 | CTTGCCGTTGGTGCGGTCGAGCACGTAGAAG |
| 96 | DNA seq. of oligonucleotide TL_018 | CGTGCTCGACCGCACCAACGGCAAGTTCATCC |
| 97 | DNA seq. of oligonucleotide TL_019 | TGAAGGTCCAGAGCATGCCGCCCTGGTTGAAG |
| 98 | DNA seq. of oligonucleotide TL_020 | CAGGGCGGCATGCTCTGGACCTTCAAGTTG |
| 99 | DNA seq. of oligonucleotide TL_021 | CCCTAGAAGCTTGCATGCCTGCAGGGACGATGTTCAGTACGCC |
| 100 | DNA seq. of oligonucleotide TL_023 | CGTGCTCGACCGCACCAAC |
| 101 | DNA seq. of oligonucleotide TL_022 | TTTGGCCACTTCGCCGCCCGCGC |
| 102 | DNA seq. of oligonucleotide TL_024 | CCGCAACGGTTTCTTCTAC |
| 103 | DNA seq. of oligonucleotide TL_025 | AAGTCCTTGACCCGTTTGG |
| 104 | DNA seq. of oligonucleotide TL_001 | ACCGAATTCCTCGAGTCTAG |
| 105 | DNA seq. of oligonucleotide TL_002 | ACCTCCTGTTTCCTGTGTG |
| 106 | DNA seq. of oligonucleotide TL_028 | TCACACAGGAAACAGGAGGTTAGCGGATGACCCGATCCCCACGTC |
| 107 | DNA seq. of oligonucleotide TL_027 | CTAGACTCGAGGAATTCGGTTTATGGCTTGACGCTTGCC |
| 108 | DNA seq. of oligonucleotide oES_92 | TTAAGGTCTCCCATGGCTGTCAGCAATGAAGAAATCCTCCA |
| 109 | DNA seq. of oligonucleotide oES_93 | TTAAGGTCTCCTCGAGTTAGTGGTGATGGTGATGATGTGGCTTGACGCTTGCCG |
| 110 | DNA seq. of oligonucleotide oES_110 | TTAAGGTCTCGTCCTCGGCGCCAAGAACTGGATG |
| 111 | DNA seq. of oligonucleotide oES_111 | TTAAGGTCTCGAGGACGGCCGGCGCGACGAA |
| 112 | DNA seq. of oligonucleotide oES_114 | TTAAGGTCTCGCGGGCGGCGAAGTGGCCAAACGG |
| 113 | DNA seq. of oligonucleotide oES_115 | TTAA GGTCTCGCCCGCCAGCGGCACCGCGCCGCCC |
| 114 | DNA seq. of oligonucleotide oES_134 | TTAAGGTCTCCGGAACTCGCACCTGCGCCCCGGT |
| 115 | DNA seq. of oligonucleotide oES_135 | TTAAGGTCTCGTTGCGCGGCGCCGGGTTACCG |
| 116 | DNA seq. of oligonucleotide oES_136 | TTAAGGTCTCTGGGCGGGCGGCGAAGTGGCC |
| 117 | DNA seq. of oligonucleotide oES_137 | TTAAGGTCTCCGCCCACGGCACCGCGCCGCCCC |
| 118 | DNA seq. of oligonucleotide oES_138 | TTAAGGTCTCCGCGCGGGCGGCGAAGTGGCC |
| 119 | DNA seq. of oligonucleotide oES_139 | TTAAGGTCTCCGCGCGCGGCACCGCGCCGCCCC |
| 120 | DNA seq. of oligonucleotide oES_140 | TTAAGGTCTCTGGACCATCAAGCCGCTCAATGAAGAC |
| 121 | DNA seq. of oligonucleotide oES_141 | TTAAGGTCTCGGTCCAGCCGGCACCGAGGAACGCCGC |
| 122 | DNA seq. of oligonucleotide oES_142 | TTAAGGTCTCCGCACCATCAAGCCGCTCAATGAAGAC |
| 123 | DNA seq. of oligonucleotide oES_143 | TTAAGGTCTCGGTGCGGCCGGCACCGAGGAACGCCGC |
| 124 | DNA seq. of oligonucleotide oES_144 | TTAAGGTCTCCGCGGTGCCGGCTTCACCATCAAGCC |
| 125 | DNA seq. of oligonucleotide oES_145 | TTAAGGTCTCACCGCGGAACGCCGCACCTTTCTTATAGGCG |
| 126 | DNA seq. of oligonucleotide oES_146 | TTAAGGTCTCTGGGGTGCCGGCTTCACCATCAAGCC |
| 127 | DNA seq. of oligonucleotide oES_147 | TTAAGGTCTCACCCCAGAACGCCGCACCTTTCTTATAGGCG |
| 128 | DNA seq. encoding the AA seq. of the signal peptide (DNA seq. encoding AA seq. of SEQ ID NO: 31) | ATGACCCGATCCCCACGTCGCCCCTTGTTCGCCGTGAGCCTGGTGCTCAGCGCCATGCTGCTTGCCGG CGCGGCTCACGCC |

| | | |
|---|---|---|
| Notes: Seq. = sequence; AA = amino acid; mutated amino acids and respective triplets are marked; in the RNA sequences the uracil is in the attached sequence listing written as thymidine according to WIPO ST. 26 | | |

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "and/or", wherever used herein, includes the meaning of "and", "or" and "all or any other combination of the elements connected by said term".

The term "less than" or in turn "more than" does not include the concrete number. For example, "less than 20" means less than the number indicated. Similarly, "more than" or "greater than" means more than or greater than the indicated number, e.g. "more than 80 %" means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps, but not the exclusion of any other integer or step or group of integer or step. When used herein the term "comprising" can be substituted with the term "containing" or "including" or sometimes, when used herein, with the term "having". When used herein, "consisting of" excludes any element, step, or ingredient not specified.

The term "including" means "including but not limited to", "Including" and "including but not limited to" are used interchangeably.

It should be understood that this invention is not limited to the particular methodology, protocols, material, reagents, and substances, etc., described herein and as such can vary. The terminology used herein is for the purpose of describing particular embodiments, only, and is not intended to limit the scope of the present invention, which is defined solely by the claims.

All publications cited throughout the text of this specification (including all patents, patent application, scientific publications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

A better understanding of the present invention and of its advantages will be had from the following examples, offered for illustrative purposes only. The examples are not intended to limit the scope of the present invention in any way.

The invention is further characterized by the following items:
1. A alcohol oxidoreductase polypeptide converting 5-hydroxymethyl-2-furoic acid (HMFA) to 5-formyl-2-furoic acid (FFA) with a Km value of less than 15 mM selected from the group consisting of
   (a) a polypeptide having the amino acid sequence shown in SEQ ID NO: 1, wherein in said polypeptide amino acids at positions corresponding to positions F412, F459 and W561 are V, W or R and A, respectively;
   (b) a polypeptide fragment of the polypeptide of (a), wherein in said polypeptide fragment amino acids at positions corresponding to positions F412, F459 and W561 are V, W or R and A, respectively;
   (c) a polypeptide having an amino acid sequence which is at least 70% identical to the amino acid sequence of the polypeptide of (a) or (b), wherein in said polypeptide amino acids at positions corresponding to positions F412, F459 and W561 are V, W or R and A, respectively;
   (d) a polypeptide having the amino acid sequence shown in SEQ ID NO: 1, wherein in said polypeptide amino acids at positions corresponding to positions F412, L560 and W561 are V, R or W and A, respectively;
   (e) a polypeptide fragment of the polypeptide of (d), wherein in said polypeptide fragment amino acids at positions corresponding to positions F412, L560 and W561 are V, R or W and A, respectively;
   (f) a polypeptide having an amino acid sequence which is at least 70% identical to the amino acid sequence of the polypeptide of (d) or (e), wherein in said polypeptide amino acids at positions corresponding to positions F412, L560 and W561 are V, R or W and A, respectively;
   (g) a polypeptide having the amino acid sequence shown in SEQ ID NO: 1, wherein in said polypeptide amino acids at positions corresponding to positions F412, L455 and W561 are V, W or R and A, respectively;
   (h) a polypeptide fragment of the polypeptide of (g), wherein in said polypeptide fragment amino acids at positions corresponding to positions F412, L455 and W561 are V, W or R and A, respectively;
   (i) a polypeptide having an amino acid sequence which is at least 70% identical to the amino acid sequence of the polypeptide of (g) or (h), wherein in said polypeptide amino acids at positions corresponding to positions F412, L455 and W561 are V, W or R and A, respectively.
2. The alcohol oxidoreductase of claim 1, wherein said alcohol oxidoreductase is a alcohol dehydrogenase, preferably the alcohol dehydrogenase is a co factor dependent alcohol dehydrogenase, wherein the co factor is a co factor located in the periplasm, preferably the co factor is cytochrome C, pyrroloquinoline quinone (PQQ), or a quinone similar to PQQ, more preferably the co factor dependent alcohol dehydrogenase is the pyrroloquinoline quinone (PQQ) dependent alcohol dehydrogenase, most preferably the co factor dependent alcohol dehydrogenase is PedE or PedH, exceptionally preferred is PedH.
3. The polypeptide of any one of items 1(a) to (f), wherein in said polypeptide or polypeptide fragment amino acids at positions corresponding to positions F412, F459, L560 and W561 are V, W or R, R or W and A, respectively, said polypeptide or polypeptide fragment further converting FFA to 2,5 furandicarboxylic acid (FDCA).
4. The polypeptide of item 3, wherein said polypeptide is selected from the group consisting of
   (a) a polypeptide having the amino acid sequence shown in SEQ ID NO: 1, wherein in said polypeptide amino acids at positions corresponding to positions F412, F459, L560 and W561 are V, W or R, R or W and A, respectively;
   (b) a polypeptide fragment of the polypeptide of (a), wherein in said polypeptide fragment amino acids at positions corresponding to positions F412, F459, L560 and W561 are V, W or R, R or W, and A, respectively;
   (c) a polypeptide having an amino acid sequence which is at least 70% identical to the amino acid sequence of the polypeptide of (a) or (b), wherein in said polypeptide amino acids at positions corresponding to positions F412, F459, L560 and W561 are V, W or R, R or W and A, respectively.
5. The polypeptide of any one of items 1(g) to (i), wherein in said polypeptide or polypeptide fragment amino acids at positions corresponding to positions F412, L455, L560 and W561 are V, W or R, R or W and A, respectively, said polypeptide or polypeptide fragment further converting FFA to FDCA.
6. The polypeptide of item 5, wherein said polypeptide is selected from the group consisting of
   (a) a polypeptide having the amino acid sequence shown in SEQ ID NO: 1, wherein in said polypeptide amino acids at positions corresponding to positions F412, L455, L560 and W561 are V, W or R, R or W and A, respectively;
   (b) a polypeptide fragment of the polypeptide of (a), wherein in said polypeptide fragment amino acids at positions corresponding to positions F412, L455, L560 and W561 are V, W or R, R or W, and A, respectively;
   (c) a polypeptide having an amino acid sequence which is at least 70% identical to the amino acid sequence of the polypeptide of (a) or b), wherein in said polypeptide amino acids at positions corresponding to positions F412, L455, L560 and W561 are V, W or R, R or W and A, respectively.
7. The polypeptide of item 1(a) having the amino acid sequence shown in SEQ ID NO: 3, 4, 5 or 6.
8. The polypeptide of item 1(d) having the amino acid sequence shown in SEQ ID NO: 7, 8, 9, or 10.
9. The polypeptide of item 1(g) having the amino acid sequence shown in SEQ ID NO: 11, 12, 13, or 14.
10. The polypeptide of item 3(a) having the amino acid sequence shown in SEQ ID NO: 15, 16, 17, 18, 19, 20, 21, or 22.
11. The polypeptide of item 5(a) having the amino acid sequence shown in SEQ ID NO: 23, 24, 25, 26, 27, 28, 29, and 30.
12. A polynucleotide encoding the polypeptide of any one of items 1 to 11.
13. The polynucleotide of item 12 having the nucleotide sequence shown in SEQ ID NO: 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 67, 68, 69, 70, 71, 72, 73, 74, 75, 76, 77, 78, 79, 80, 81, 82, 83, 84, 85, 86, 87, 88, 89, 90, 91, 92 or 93.
14. A vector comprising the polynucleotide of item 12 or 13.
15. The vector of item 14, wherein said vector is an expression vector.
16. A host cell comprising the polynucleotide of item 12 or 13 or the vector of item 14 or 15.
17. The host cell of item 16, wherein said host cell is a gram-negative bacterial host cell.
18. The host cell of item 17, wherein said gram-negative bacterial host cell is a Pseudomonas species, preferably Pseudomonas putida.
19. The host cell of item 17 or 18, wherein said host cell lacks a functional hmft1 gene (HMF transporter associated with hmf clusters) and /or a functional hmfH gene.
20. A method for converting HMFA to FFA, comprising culturing the host cell of any of items 16 to 19 expressing the polypeptide as defined in any one of items 1 to 11.
21. The method of item 20, wherein FFA is further converted to FDCA by the host cell of any one of items 16 to 19 expressing the polypeptide as defined in any one of items 3 to 6, 10 or 11.
22. The method of item 20 or 21, wherein said host cell is further capable of converting 5-(hydroxymethyl)furfural (HMF) to HMFA.
23. A method for the production of FDCA, comprising culturing a host cell expressing a polypeptide as defined in any one of items 3 to 6, 10 or 11.
24. The method of any one of items 20 to 23, wherein the conversion takes place in the periplasm of said host cell.
25. Use of the host cell of any one of items 16 to 19 for converting HMFA to FFA, said host cell expressing the polypeptide as defined in any one of items 1 to 11.
26. Use of the host cell of any one of items 16 to 19 for converting FFA to FDCA, said host cell expressing the polypeptide as defined in any one of items 3 to 6, 10 or 11.
27. The use of item 25 or 26, wherein said host cell is further capable of converting HMF to HMFA.
28. Use of the host cell of any one of items 16 to 19 for expressing the polypeptide as defined in any one of items 3 to 6, 10 or 11 for the production of FDCA.
29. Crystal structure of the polypeptide having the amino acid shown in SEQ ID NO: 15 characterized as having (a) a space group of P4₃2₁2 and (b) unit cell dimensions of a = 105.23 Å, b = 105.23 A and c = 186.52 Å, α, β, γ = 90°.
30. Use of the crystal structure as defined in item 29 for obtaining atomic spatial relationship data.
31. Use of the crystal structure of item 29 for identifying amino acids which aid in the conversion of FFA to FDCA.

### EXAMPLES

### Material and Methods

### Strains and culture conditions

Bacterial cells (a summary of all strains used in this study is provided in **Table 2)** were routinely cultivated in LB medium (5 g L⁻¹ sodium chloride) at 37°C (*E*. *coli*) or 30°C (*P*. *putida* KT2440) and 200 rpm in a rotary shaker (Kuhner Shaker, Herzogenrath, Germany) with controlled humidity of 80% and a throw of 50 mm. For the maintenance of plasmids and the selection of genomic recombination events, kanamycin sulfate (50 mg L⁻¹) and gentamycin sulfate (20 mg L⁻¹) were utilized. Selection for *Pseudomonas* after mating procedures was achieved by supplementation of irgasan (25 mg L⁻¹) to the medium. HMF conversion assays were carried out in buffer-adjusted MSM (one-fold concentrations: 22.3 mM K₂HPO₄ and 13.6 mM NaH₂PO₄) according to Hartmans *et al.* (25). Carbon sources and lanthanides salts were added as indicated for the respective experiment.

### Plasmid cloning and strain engineering

All newly generated plasmids **(Table 3)** were constructed using NEBuilder HiFi DNA Assembly (New England Biolabs, Ipswich, MA, USA) and confirmed by Sanger sequencing. DNA amplicons utilized in cloning were obtained by PCR with Q5 High-Fidelity Polymerase (New England Biolabs, Ipswich, MA, USA). Primers **(Table 4)** were purchased from Eurofins Genomics (Ebersberg, Germany) as unmodified DNA oligonucleotides. *Pseudomonas* was transformed with purified plasmids by electroporation using a GenePulser Xcell (BioRad, Hercules, CA, USA) (settings: 2 mm cuvette gap, 2.5 kV, 200 Ω, 25 µF) or through conjugation via triparental patch matings (26).

The gene encoding PedH (PP_2679) was PCR amplified from genomic DNA of *Pseudomonas putida* KT2440 without the signal peptide (SEQ ID NO: 31) (PedH28-595 = (SEQ ID NO: 2) using the forward primer oES_92 (SEQ ID NO: 78) and the reverse primer oES_93 (SEQ ID NO: 79) containing a Ncol and Xhol restriction site and the coding sequence for a c-terminal hexa-histidine tag. The fragment was digested with Ncol and Xhol and cloned into pET24d(+) (Novagen) yielding in pES_12 (pET24d-PedH).

Variants have been cloned using Golden Gate Mutagenesis. The mutant fragments were amplified from pES12 using forward and reverse primers having the desired mutation and Bsal restriction site. All Golden Gate reactions were performed in a total volume of 15 µl. The final reaction volume contained 1-fold concentrated T4 ligase buffer (Promega, Madison, US). Prepared reaction mixtures (ligase buffer, acceptor plasmid, insert(s)) were adjusted to 13.5 µl with ddH2O. In a final step, the corresponding enzymes were quickly added. First, a volume of 0.5 µl of Bsal-HF^{®}v2 (10 units; New England Biolabs, Ipswich, US) and then 1 µl (1-3 units) of T4 ligase (Promega, Madison, US) was added. Golden Gate reactions were carried out by default under following conditions: a) Enzymatic restriction 37 °C (2 min); b) Ligation 16 °C (5 min) [10 passes each] and c) enzyme inactivation: 80 °C (20 min).

For genomic replacement of *pedH* by *pedH*^{F412V, F459W, L560R, W561A}, a 667 bp DNA fragment within the *pedH* gene (PP_2679) of *P. putida* KT2440 including the codons to be mutated was replaced by the homologous sequence containing the desired modifications using the I-Scel-based system (27). The mutated gene section was amplified from the expression vector pES88 and cloned into pEMG linearized with Sall-HF and EcoRI-HF (New England Biolabs, Ipswich, MA, USA) alongside 700-900 bp upstream and downstream flanking regions (TS1/TS2). The resulting integrative plasmid was transferred from *E*. *coli* PIR2 into *P. putida* KT2440. Integration clones were subsequently transformed with pSW-2 (no induction of *I-scel* expression by 3-methylbenzoate). Successfully engineered clones were identified by colony PCR (preceded by alkaline cell lysis) (28) with a reverse primer specifically binding to the mutated sequence coding for L560R and W561A, cured of pSW-2, and confirmed by Sanger sequencing.

### Protein production and purification.

For enzyme kinetics and crystallography protein production and purification was performed as follows (29). Briefly, constructs were transformed in E. *coli* BL21 (DE3) (Novagen) for overexpression. Therefore, constructs were created expressing the PedH amino acid sequence with the desired amino acid mutation without the signal peptide, but with one methionine added at the N-terminus and a 6xHis-tag added at the C-terminus. For example, the amino acid sequences of the PedH^{F412V, F459W, W561A} with and without the signal peptide are shown in SEQ ID NOs: 3 and 4. The amino acid sequence of PedH^{F412V, F459W, W561A} used for expression in *E*. *coli* is shown in SEQ ID NO: 32 (DNA sequence shown in SEQ ID NO 63), without the signal peptide, but with one methionine added at the **N**-terminus and a 6xHis-tag added at the C-terminus. The further used constructs were created identical, all having one methionine added at the **N**-terminus and a 6xHis-tag added at the C-terminus, but lacks the signal peptide. Cells were grown in lysogeny-broth (LB) medium, supplemented with 1 % lactose and kanamycin (50 mg/l). Cells were incubated at 30 °C overnight under rigorous shaking (180 rpm). Cells were harvested by centrifugation (3,500 x g, 20 min, 4 °C) and resuspended in 20 ml buffer A (20 mM HEPES-Na, pH 8.0, 500 mM NaCl, 20 mM KCI, 20 mM MgCl₂, 40 mM imidazole) before lysis in a M-110L Microfluidizer (Microfluidics). The lysate was cleared at 47,850 x g for 20 min at 4 °C and the supernatant was applied onto 5 ml HisTrap FF columns (GE Healthcare) for NiNTA affinity chromatography. After a wash step with 15 column volumes (CV) of buffer A, proteins were eluted with three CV of buffer B (20 mM HEPES-Na, pH 8.0, 500 mM NaCl, 20 mM KCI, 20 mM MgCl₂, 500 mM imidazole). Protein eluted fractions were pooled and treated with a final concentration of 100 mM EDTA prior to concentration in Amicon Ultra-10K centrifugal filters to 2.5 ml and further purified by size-exclusion chromatography (SEC). PedH and the variants were purified using a HiLoad 26/600 Superdex 200 column (GE Healthcare) equilibrated in buffer SEC (20 mM HEPES-Na, pH 7.5, 500 mM NaCl). Fractions containing the protein were pooled and concentrated for crystallization experiments.

### Enzyme kinetics measurements.

To be able to reach saturating substrate concentrations, a modified protocol was used to determine specific enzyme activities for the analysis of kinetic enzyme parameters (30, 31). Shortly, 1 ml of assay solution containing 20 mM HEPES-Na (pH 8), 500 µM phenazine methosulfate (PMS), 150 µM 2,6-dichlorophenol indophenol (DCPIP), 25 mM imidazole, 50 µM PrCl₃, 0.1 mM PQQ, and 0.2 µM of enzyme. Addition of the enzyme to the reaction mixture started the measurement. Enzyme activity was calculated based on the change in OD₆₀₀ within the first minute upon substrate addition. Assays were performed in 3 replicates and data are presented as the mean value with error bars representing the corresponding standard deviation. Kinetic parameters were obtained by fitting the enzyme activities at different substrate concentrations to the Michaelis-Menten equation. Kinetic values are presented as mean values with corresponding standard error.

### Crystallization and structure determination.

Crystallization was performed by the sitting-drop method at 20 °C in 0.5 µl drops consisting of equal parts of protein and precipitation solutions. PedH^{F412V/F459W/L560R/W561A} was co-crystallized at 250 µM concentration with equimolar concentration of PQQ and metal of interest within 24 h in 0.2 M Lithium sulfate, 0.1 M sodium cacodylate pH = 6.5, 30 % PEG 400. Prior to data collection, crystals were flash-frozen in liquid nitrogen employing a cryo-solution that consisted of mother-liquor supplemented with 30 % (v/v) glycerol. Data were collected under cryogenic conditions at the BESSY II beamlines. Data were integrated and scaled with XDS and merged with XSCALE. Structures were determined by molecular replacement with PHASER, manually built in COOT (32), and refined with PHENIX (33). The structure of the PedH^{F412V/F459W/L560R/W561A} -PQQ-Metal complex was determined by molecular replacement using the crystal structures of PedH^{F412V/W561A} of *Pseudomonas putida* (PDB-ID: 6ZCV) (24) as search models. Figures were prepared with PYMoL.

### Whole-cell HMF conversion assays in shake flasks and consecutive HPLC analysis

HMF conversion assays in shake flasks were performed as described previously (35). Culture supernatants were analyzed at 40 °C and a flow rate of 0.8 mL min⁻¹ using a 1260 Infinity II HPLC system (Agilent, Santa Clara, CA, USA) equipped with an ISAsphere 100-3 C18 BDS (3.0 x 250 mm, 3.0 µm) column. Chromatography was performed with a solvent mixture of potassium acetate buffer (10 mM, pH = 5.5) and acetonitrile for 19 min. Solvent composition was varied as indicated hereafter: 0 min: 10% acetonitrile; 0-10 min: linear increase to 40% acetonitrile; 10-13 min: 40% acetonitrile; 13-15 min: linear decrease to 10% acetonitrile; 15-19 min: 10% acetonitrile. UV detection was conducted using different wavelengths specific to each compound: HMF at 280 nm, HMFA at 250 nm, FFA at 280 nm and FDCA at 260 nm. Retention times were 6.23 min, 2.66 min, 3.00 min, and 2.23 min for HMF, HMFA, FFA, and FDCA respectively. Analytical standards used for quantification were purchased from Biosynth.

### Verification of in vitro oxidation of HMFA.

To verify the ability of the PedH variants produced in *E*. *coli* the inventors used the PedH variants *in-vitro* for producing FFA and analyzing the results by HPLC as mentioned before.

Shortly, 200µL of assay solution for HPLC (= Culture supernatants, mentioned before) were prepared containing 10 mM HMFA, 0.4 µM of enzyme (meaning one of the PedH variants produced herein), 20 mM HEPES-Na (pH 8), 500 µM phenazine methosulfate (PMS), 150 µM 2,6-dichlorophenol indophenol (DCPIP), 25 mM imidazole, 50 µM PrCl3, and 5µM PQQ. This mix was incubated at 30°C in a microtiter plate for 6 hours. The plate was covered with a lid to minimize evaporation. After that the solution was analyzed via HPLC as mentioned above in "Whole-cell HMF conversion assays in shake flasks and consecutive HPLC analysis" using the solution instead of the culture supernatants.

The graphs show HPLC analysis of A: analytical standards and a control reaction without added enzyme, and B: reactions with to different enzyme mutants. All four tested mutants convert HMFA into FFA, confirming their activity.

**Table 2**

| Bacterial strains used in the examples | | |
|---|---|---|
| **Strain** | **relevant characteristics** | **reference** |
| ***E. coli*** | | |
| PIR2 | F⁻, Δ*lac169*, *rpoS(Am), robA1, creC510*, *hsdR514, endA, recA1*, *uidA(*Δ*Mlul)*::*pir*; host for *oriV*(R6K) vectors in low copy number | Thermo Fisher Scientific |
| HB101 pRK2013 | Sm^{R}, *hsdR-M⁺*, *proA2, leuB6, thi-1, recA;* bears plasmid pRK2013 | (34) |
| DH5α | F Φ80 *lacZ*ΔM15 Δ(*lac*ZYA-argF)U169 *recA1 endA1 hsdR17* (rₖ⁻, mₖ⁺) *phoA supE44 thi-1 gyrA96 rel*A1 λ⁻ | Thermo Fisher Scientific |
| DH5α pSW-2 | DH5α bearing pSW-2 | (27) |
| DH5α pES12 | DH5α bearing pES12 | (24) |
| DH5α pES67 | DH5α bearing pES67 | (24) |
| DH5α pES72 | DH5α bearing pES72 | this work |
| DH5α pES73 | DH5α bearing pES73 | this work |
| DH5α pES74 | DH5α bearing pES74 | this work |
| DH5α pES75 | DH5α bearing pES75 | this work |
| DH5α pES76 | DH5α bearing pES76 | this work |
| DH5α pES77 | DH5α bearing pES77 | this work |
| DH5α pES78 | DH5α bearing pES78 | this work |
| DH5α pES88 | DH5α bearing pES88 | this work |

| **Strain** | **relevant characteristics** | **reference** |
|---|---|---|
| ***E. coli*** | | |
| BL21 (DE3) | F - ompT hsdSB (rB- mB-) gal dcm (DE3) | Thermo Fisher Scientific |
| BL21 (DE3) pES12 | BL21 (DE3) bearing pES12 | (24) |
| BL21 (DE3) pES67 | BL21 (DE3) bearing pES67 | (24) |
| BL21 (DE3) pES72 | BL21 (DE3) bearing pES72 | this work |
| BL21 (DE3) pES73 | BL21 (DE3) bearing pES73 | this work |
| BL21 (DE3) pES74 | BL21 (DE3) bearing pES74 | this work |
| BL21 (DE3) pES75 | BL21 (DE3) bearing pES75 | this work |
| BL21 (DE3) pES76 | BL21 (DE3) bearing pES76 | this work |
| BL21 (DE3) pES77 | BL21 (DE3) bearing pES77 | this work |
| BL21 (DE3) pES78 | BL21 (DE3) bearing pES78 | this work |
| BL21 (DE3) pES88 | BL21 (DE3) bearing pES88 | this work |

| ***P. putida*** | | |
|---|---|---|
| KT2440 | mt-2 derivative, cured from the TOL plasmid pWW0 | (35) |
| KT2440 *pedH*^{F412V/F459W/L560R/W561A} | native *pedH* exchanged by the rationally engineered quadruple mutant *pedH*^{F412V/F459W/L560R/W561A} | this work |
| KT2440 pBT'T_*pedH*^{F412V/F459W/L560R/W561A} | KT2440 harboring pBT'T_*pedH*^{F412V/F459W/L560R/W561A} | this work |
| KT2440 pBT'T_*pedH* | KT2440 harboring pBT'T_*pedH* | this work |

**Table 3**

| Plasmids used in the examples | | |
|---|---|---|
| **plasmid** | **relevant characteristics** | **reference** |
| pRK2013 | Km^{R}, *oriV*(RK2/ColE1), mob⁺ tra⁺ | (36) |
| pSW-2 | Gm^{R}, *oriV*(RK2), mob⁺, *xylS, Pₘ* → *I-scel* | (27) |

| **pEMG and derivatives** | | |
|---|---|---|
| pEMG | Km^{R}, *oriV(R6K),* lacZα-MCS flanked by two I-Scel sites | (27) |
| pEMG-*pedH*^{F412V/F459W/L560R/W561A} | pEMG bearing quadruple mutant *pedH*^{F412V/F459W/L560R/W561A} flanked by adequate homology regions for genomic substitution of native *pedH* (PP_2679) | this work |

| **Expression plasmids** | | |
|---|---|---|
| pBT'Tmcs | pBT'mcs with RBS, Km^{R}, oriV(pBBR1), expression vector containing the constitutive *P_{tac}* promoter, no terminator | (37) |
| pBT'T_*pedH*^{F412V/F459W/L560R/W561A} | pBT'T harboring engineered quadruple mutant *pedH*^{F412V/F459W/L560R/W561A} from *P*. *putida* KT2440, 5'UTR designed such as translation initiation rate was predicted to be 5000 (RBS calculator, Salis Lab) (38) | this work |
| pBT'T_*pedH* | pBT'T harboring wild type *pedH* from *P. putida* KT2440, 5'UTR designed such as translation initiation rate was predicted to be 5000 (RBS calculator, Salis Lab) (38) | this work |
| pES12 | pET24d(+) (Novagen) expressing PedH without the signal peptide (PedH28-595) | (24) |
| pES67 | pET24d(+) (Novagen) expressing PedH^{F412V/W561A} | (24) |
| pES72 | pET24d(+) (Novagen) expressing PedH^{F412V/W285R/W561A} | this work |
| pES73 | pET24d(+) (Novagen) expressing PedH^{F412V/L560W/W561A} | this work |
| pES74 | pET24d(+) (Novagen) expressing PedH^{F412V/L560R/W561A} | this work |
| pES75 | pET24d(+) (Novagen) expressing PedH^{F41e2V/F459W/W561A} | this work |
| pES76 | pET24d(+) (Novagen) expressing PedH^{F412V/F459R/W561A} | this work |

| **plasmid** | **relevant characteristics** | **reference** |
|---|---|---|
| **Expression plasmids** | | |
| pES77 | pET24d(+) (Novagen) expressing PedH^{F412V/L455R/W561A} | this work |
| pES78 | pET24d(+) (Novagen) expressing PedH^{F412V/L455W/W561A} | this work |
| pES88 | pET24d(+) (Novagen) expressing PedH^{F412V/F459W/L560R/W561A} | this work |
| pES89 | pET24d(+) (Novagen) expressing PedH^{F412V/L455R/L560R/W561A} | this work |
| pES90 | pET24d(+) (Novagen) expressing PedH^{F412V/L455W/L560R/W561A} | this work |

**Table 4**

| Oligonucleotides (name, sequence, and description) used as PCR primers for cloning procedures. Lower case letters indicate overhangs, letters representing the binding sequence are capitalized, customly added spacers are italicized, and restriction sites are underlined. | | | |
|---|---|---|---|
| **name** | **SEQ ID NO** | **Sequence (5' → 3')** | **description** |
| **TL_016** | 94 | agtatagggataacagggtaatctgGGCAA GAAACTGTGGCAATAC | TS1*-pedH* forward primer |
| **TL_017** | 95 | cttgccgttggtGCGGTCGAGCACGTA GAAG | TS1*-pedH* reverse primer |
| **TL_018** | 96 | cgtgctcgaccgcACCAACGGCAAGTT CATCC | *pedH*^{F412V/F459W/L560R/W561 A} forward primer |
| **TL_019** | 97 | tgaaggtccagagCATGCCGCCCTGG TTGAAG | *pedH*^{*F412V*/*F459W*/*L560R*/}*^{W561} A reverse primer* |
| **TL_020** | 98 | caggcggcatgCTCTGGACCTTCAA GTTG | *TS2-pedH forward primer* |
| **TL_021** | 99 | ccctagaagcttgcatgcctgcaggGACGA TGTTCAGTACGCC | *TS2-pedH reverse primer* |

| **name** | **SEQ ID NO** | **Sequence (5' → 3')** | **description** |
|---|---|---|---|
| **TL_023** | 100 | CGTGCTCGACCGCACCAAC | Mapping of mutated *pedH* forward primer |
| **TL_022** | 101 | TTTGGCCACTTCGCCGCCCGCG C | Mapping of mutated *pedH* reverse primer, anneals specifically to the sequence coding for L560R and W561A |
| **TL_024** | 102 | CCGCAACGGTTTCTTCTAC | Sanger sequencing of mutated pedH forward primer |
| **TL_025** | 103 | AAGTCCTTGACCCGTTTGG | Sanger sequencing of mutated pedH reverse primer |
| **TL_001** | 104 | ACCGAATTCCTCGAGTCTAG | pBT'Tmcs forward primer |
| **TL_002** | 105 | ACCTCCTGTTTCCTGTGTG | pBT'Tmcs reverse primer |
| **oES_92** | 106 | ttaaggtctcccatgGCTGTCAGCAATG AAGAAATCCTCCA | pES12_PedH_ forward primer |
| **TL_028** | 107 | tcacacaggaaacaggaggttagcggATGA CCCGATCCCCACGTC | *pedH*^{(F412V/F459W/L560R/W56 1A)} from KT2440 (*pedH*^{F412V/F459W/L560R/W56 1A}) for pBT'T_*pedH*^{(F412V/F459W/L 560R/W561A)} forward primer |
| **TL_027** | 108 | ctagactcgaggaattcggtTTATGGCTT GACGCTTGCC | *pedH*^{(F412V/F459W/L560R/W56 1A)} from KT2440 (*pedH*^{F412V/F459W/L560R/W56 1A}) for pBT'T_*pedH*^{(F412V/F459W/L 560R/W561A)} reverse primer |
| **oES_93** | 109 | ttaaggtctcctcgagTTAgtggtgatggtgatg atgTGGCTTGACGCTTGCCG | *pES12*_*PedH*_reverse *primer* |
| **oES_110** | 110 | ttaaggtctcgTCCTCGGCGCCAAGAA CTGGATG | *pES67_F412V_forward primer* |
| **oES_111** | 111 | ttaaggtctcGAGGAcGGCCGGCGCG ACGAA | *pES67_F412V_reverse primer* |
| **oES_114** | 112 | ttaaggtctcgcGGGCGGCGAAGTGG CCAAACGG | *pES67_W561A_forward primer* |
| **oES_115** | 113 | ttaa ggtctcGCCCgcCAGCGGCACCGCG CCGCCC | *pES67_ W561A _reverse primer* |
| **oES_134** | 114 | ttaaggtctccGGAACTCGCACCTGC GCCCCGGT | *pES72_W285R_forward primer* |
| **oES_135** | 115 | ttaaggtctcGTTgCgCGGCGCCGGG TTACCG | *pES72_ W285R _reverse primer* |
| **oES_136** | 116 | ttaaggtctcTGGGCGGGCGGCGAAG TGGCC | *pES73_L560W_ forward primer* |
| **oES_137** | 117 | ttaaggtctcCGCCCACGGCACCGCG CCGCCCC | *PES73_L560W_ reverse primer* |
| **oES_138** | 118 | ttaaggtctcCGCGCGGCGGCGAA GTGGCC | *pES74_L560R_forward primer* |
| **oES_139** | 119 | ttaaggtctcCGCGCGCGGCACCGCG CCGCCCC | *pES74_L560R_ reverse primer* |
| **oES_140** | 120 | ttaaggtctcTGGACCATCAAGCCGC TCAATGAAGAC | *pES75_F459W_ forward primer* |
| **oES_141** | 121 | ttaaggtctcGGTCCAGCCGGCACCG AGGAACGCCGC | *pES75_F459W_*reverse *primer* |
| **oES_142** | 122 | ttaaggtctcCGCACCATCAAGCCGC TCAATGAAGAC | *pES76_F459R_ forward primer* |
| **oES_143** | 123 | ttaaggtctcGGTGCGGCCGGCACCG AGGAACGCCGC | *pES76_F459R_reverse primer* |
| **oES_144** | 124 | ttaaggtctcCGCGGTGCCGGCTTCA CCATCAAGCC | *pES77_L455R_forward primer* |
| **oES_145** | 125 | ttaaggtctcACCGCGGAACGCCGCA CCTTTCTTATAGGCG | *pES77_L455R_reverse primer* |
| **oES_146** | 126 | ttaaggtctcTGGGGTGCCGGCTTCA CCATCAAGCC | *pES78_L455W_ forward primer* |
| **oES_147** | 127 | ttaaggtctcACCCCAGAACGCCGCA CCTTTCTTATAGGCG | *pES78_L455W_ reverse primer* |

### Example 1 Optimization of the PQQ-dependent alcohol dehydrogenase PedH for carboxylated furanic substrates

The previous findings of the inventors showed that the double mutant PedH^{F412V/W561A} variant is capable of oxidizing HMF and HMFA. It was interesting why it showed higher Km value towards HMFA compared to HMF, however HMFA is not that much different in size. Moreover, it is not capable of oxidising FFA, the last aldhyde in the pathway to produce FDCA as the target product for establishing the future green chemistry(24). This may be attributed to various factors, including the chemical nature of the substrates and their interaction with the enzyme (10, 11, 25, 26). It was reported that PQQ-dependent dehydrogenases can perform aldehyde oxidation through its hydrated form (5). Therefore, the formation and stability of the gem-diols are essential for the oxidation process. The gem-diol formation of HMFA and FFA is likely only very little due to their stable conjugated system through their carboxylic acid group (11). As previously shown, FFA has shown significantly lower hydration degree in aqueous solution compare to HMF and 5-formylfurfural (FFF) (10, 25). This could, in theory, impact the binding kinetics and contribute to the observed low or no activity of the quinoprotein towards HMFA and FFA.

Depending on analysis of the previously solved structures of wild-type PedH (PDB ID: 6ZCW) and the double mutant PedH^{F412V/W561A} variant (PDB ID: 6ZCV) (24), we aimed to fine-tune the activity towards HMFA and FFA as substrates to establish the first periplasmic pathway to produce FDCA. The sidechains of C131, E199, W285, V412, L413, A453, L455, F459, A557 and L560 residues of the double mutant PedH^{F412V/W561A} variant are actively involved in forming a substrate channel, which is around 9 Å long and 4 Å width **(****Fig. 2A and 2B****).** This represents the shortest distance through which the substrate could reach the active site, where PQQ and metal cofactors are located. The inventors solved an electron density of a glycerol molecule which clearly defines the binding pocket for the substrate **(****Fig. 2C****).** The widened pocket of the double mutant PedH^{F412V/W561A} perfectly fits the studied furans. By superimposing a carboxylated furanic substrate (HMFA or FFA) onto the glycerol, the inventors further define L455, F459, and L560 as the closest residues pointing to the carboxylic group of the HMFA or FFA **(****Fig. 2D****,** **Fig. 3****).** Therefore, these amino acids represented attractive targets of great interest for further engineering.

The inventors hypothesized that creating an electrostatic microenvironment could not only contribute to improve the binding kinetics of the carboxylated furanic substrates, but also enhance the transfer of the electrons for better redox activity of the enzyme. The inventors have constructed a set of variants **(Table 5)** replacing these residues of interest into ones, which could potentially behave a better electrostatic interactions with the carboxyl group of HMFA and FFA. The inventors chose arginine as a positively charged amino acid with a guanidinium side chain. The guanidinium group contains three nitrogen atoms, allowing arginine to engage in multiple hydrogen bonds with carboxyl groups, which could theortically enhance the substrate binding. In addition, the inventors included tryptophan for its signifcant role in the process of the electron transfer in redox reactions.

**Table 5**

| Composition of the designed PedH variants. | | |
|---|---|---|
| **Variant** | **Mutated residues** | **Mutation variant** |
| 1 | PedH^{F412V/L455W/W561A} | 3^{rd} triple mutation variant |
| 2 | PedH^{F412V/L455R/W561A} | 3^{rd} triple mutation variant |
| 3 | PedH^{F412V/F459W/W561A} | 1^{st} triple mutation variant |
| 4 | PedH^{F412V/F459R/W561A} | 1^{st} triple mutation variant |
| 5 | PedH^{F412V/L560W/W561A} | 2^{nd} triple mutation variant |
| 6 | PedH^{F412V/L560R/W561A} | 2^{nd} triple mutation variant |
| 7 | PedH^{F412V/F459W/L560R/W561A} | 1^{st} quadruple mutation variant |
| 8 | PedH^{F412V/L455R/L560R/W561A} | 2^{nd} quadruple mutatition variant |
| 9 | PedH^{F412V/L455W/L560R/W561A} | 2^{nd} quadruple mutation variant |

### Example 2 Kinetic parameters determination of the designed variants

The variants have been purified using His affinity followed by size exclusion chromatography. Then, the kinetic parameters have been determined **(****Fig. 4****).** Of the tested variants, the triple mutant PedH^{F412V/F459W/W561A} variant and PedH^{F412V/L560R/W561A} variant showed improved kinetic parameters with HMFA **(****Fig. 4B and 4C****, respectively).** The Km value has significantly reduced from 18.8 ± 2.6 mM in case of double mutant PedH^{F412V/W561A} variant to 11.4 ± 1.2 mM and 7.5 ± 0.8 mM in case of the newly designed triple mutants PedH^{F412V/F459WW561A} and PedH^{F412V/L560R/W561A} respectively **(Table 6).** This noticeably indicates the enhancement of the binding affinity of HMFA to the triple mutant variants by approximately 2 to 3 folds. In addition, this was accompained with increasing the catalytic efficiency (kcat = 1.64 ± 0.05 s⁻¹, kcat/Km= 0.14 ± 0.02 mM⁻¹ s⁻¹ in case of PedH^{F412V/F459W/W561A}, and kcat = 2.01 ± 0.07 s⁻¹, kcat/Km= 0.28 ± 0.03 mM⁻¹ s⁻¹ in case of PedH^{F412V/L560R/W561A}) compared to our previously published double mutant PedH^{F412V/W561A} (24) (**Table 6**).

The inventors then tested if they could acquire a synergistic effect by combining the residues involved in enhancing the kinetic parameters in the previous round. Therefore, a quadruple mutant PedH^{F412V/F459W/L560R/W561A} variant **(****Fig. 4D****, 4E and Table 6)** has been constructed and purified as previously mentioned. Interestingly, the quadruple mutant PedH^{F412V/F459W/L560R/W561A} variant showed around 5 fold decrease in the Km value for HMFA (4.1 ± 0.3 mM). At the same time, it exhibited the highest catalytic efficiency (kcat = 3.21 ± 0.06 s⁻¹, kcat/Km= 0.78 ± 0.06 mM⁻¹ s⁻¹) compared to the first generation engineered double mutant PedH^{F412V/W561A} variant, the triple mutant PedH^{F412V/F459W/W561A}, PedH^{F412V/L560R/W561A} variants, and other previously characterized enyzmes, for example HmfO of *Methylovorus* sp. MP688 (16) (kcat/KM = 0.12 mM⁻¹ s⁻¹) and the aryl alcohol oxidase AAO of *Pleurotus eryngii* (27) (kcat/KM = 0.017 ± 0.002 mM⁻¹ s⁻¹). In addition, it displayed for the first time activity towards FFA (Km= 10.2 ± 2.2 mM, kcat = 1.35 ± 0.06 s⁻¹, kcat/Km= 0.13 ± 0.08 mM⁻¹ s⁻¹), the limiting step for the double mutant PedH^{F412V/W561A} variant to produce FDCA **(Fig. 4E and Table 6).**

**Table 6**

| Kinetic parameters of the variants derived by fitting specific enzyme activities at different substrates to the Michaelis-Menten model of single-substrate enzyme kinetics | | | |
|---|---|---|---|
| **Variant** | **K_{cat} [s⁻¹]** | **K_{M} [mM]** | **K_{cat}/ K_{M} [mM⁻¹ s⁻¹]** |
| **PedH** | n.d. | n.d. | n.d. |
| **PedH^{F412V/W561A}** | 1.17 ± 0.05 | 18.8 ± 0.05 | 0.06 ± 0.01 |
| **PedH^{F412V/F459W/W561A}** | 1.64 ± 0.05 | 11.4 ± 1.2 | 1.14 ± 0.02 |
| **PedH^{F412V/L560R/W561A}** | 2.01 ± 0.07 | 7.5 ± 0.8 | 0.28 ± 0.03 |
| **PedH^{F412V/F459W/L560R/W561A}** | 3.21 ± 0.06 | 4.1 ± 03 | 0.78 ± 0.06 |
| **PedH^{F412V/F459W/L560R/W561A} (using FFA as input)** | 1.35 ± 0.06 | 10.2 ± 2.2 | 0.13 ± 0.08 |

### Example 3 Resolution of the crystal structure of quadruple mutant PedH^{F412V/F459W/L560R/W561A} variant

To get insights into the molecular basis for the improved kinetic parameters of the (first) quadruple mutant PedH^{F412V/F459W/L560R/W561A} variant, we have then solved its crystal structure in presence of the cofactors PQQ and Pr⁺³ to resolution of 2 Å **(Table 7).** By generating the electrostatic charge of the determined structure, we could observe the clear difference between the quadruple variant **(****Fig. 5B****)** and the previously solved structure of the double variant **(****Fig. 5A****)** on the surface where the entrance towards the substrate channel is located. In case of the quadruple variant, the substrate channel entrance is sandwiched between the positively charged guanidino group of the polar arginine residue R560 and the higher electron cloud of W459. Such designed electrostatic microenvironment which could efficiently participate in improving the kinetic parameters of a second generation of the periplasmic PQQ-dependent alcohol dehydrogenase PedH towards the carboxylated substrates of HMFA and FFA.

**Table 7**

| Crystallographic data collection and refinement statistics for PedH^{F412V/F459W/L560R/W561A}. | |
|---|---|
| | **PedH^{F412V/F459W/L560R/W561A}** |
| **Data collection** | |
| Space group | P4₃2₁2 |
| Resolution range (Å) | 47.06 - 2.0 (2.071 - 2.0) |

| **Unit cell parameters** | |
|---|---|
| a, *b,* c (Å) | 105.23, 105.23, 186.52 |
| *α,* β, *γ* (°) | 90, 90, 90 |
| *R*_{merge} | 0.2459 (1.785) |
| Average //*σ*(*l*) | 11.25 (1.79) |
| No. of total reflections | 1054668 (107140) |
| Completeness (%) | 99.74 (99.46) |
| CC_{1/2}(%) | 0.997 (0.682) |

| ***Refinement*** | |
|---|---|
| *R*_{work} | 0.1654 (0.2303) |
| *R*_{free} | 0.1902 (0.2600) |

| **No. of atoms** | |
|---|---|
| Overall | 4926 |
| Protein | 4348 |
| Ligands | 25 |
| Water | 553 |

**Table 7 (continued)**

| | **PedH^{F412V/F459W/L560R/W561A}** |
|---|---|
| **Average *B*-factor(Å²)** | |
| Overall | 27.49 |
| Protein | 26.33 |
| Ligands | 23.32 |
| Water | 36.87 |

| **Root-mean-square deviation** | |
|---|---|
| Bond lengths (Å) | 0.017 |
| Bond angles (°) | 2.09 |

| **Ramachandran plot (%)** | |
|---|---|
| Favored | 95.89 |
| Allowed | 3.57 |
| Outliers | 0.54 |

### Example 4 In vivo application of first quadruple mutant PedH^{F412V/F459W/L560R/W561A}

Ultimately, the applicability of the improved enzyme variant for whole-cell biocatalysis was tested. For this, the respective mutations were introduced into the genome of P. *putida* KT2440 and the resulting strain was analyzed for FDCA production from HMF in shake flasks as described previously (14). In accordance with prior results, the wild type and the mutant strain KT2440 *pedH*^{F412V/F459W/L560R/W561A} rapidly oxidized HMF to the corresponding acid HMFA in 3 h through the action of PaoEFG and AldB-I(I) **(****Fig. 6****)** (14,28). Since native PedH does not accept HMFA as a substrate the wild type strain could not further process this molecule **(****Fig. 6A** and **6B****)** (24). However, in the presence of LaCl₃, the strain harboring the quadruple mutant produced FDCA, whereas in the control experiment without lanthanum ions no further oxidation of HMFA could be detected **(****Fig. 6C** and **6D****).** This proved that the rationally designed mutations in *pedH* enable entirely periplasmic oxidation of HMF to FDCA with *P. putida* KT2440 as whole-cell catalyst.

### Example 5: Verification of in vitro oxidation of HMFA.

To verify the activity on HMFA of the PedH variants produced in E. *coli* the inventors used the PedH variants *in-vitro* for producing FFA and analyzing the results by HPLC as mentioned before.

As shown in the methods section above, HMFA was mixed with one of the PedH variants PedH^{F412V/F459W/L560R/W561A}, PedH^{F412V/L455W/W561A}, PedH^{F412V/L455R/L560R/W561A}, or PedH^{F412V/L455W/L560R/W561A}. After 6 hours incubation the content of HMFA and FFA, which results by converting HMFA to FFA by the PedH variant added, in the solution was analyzed by HPLC. As shown in **Figure 7B****,** all of the tested variants effectively convert HMFA to FFA. **Figure 7A** shows analytical stanrds of HMF and FFA and a negative control reaction without any PedH variant (=enzyme).

Thus, all four tested mutants convert HMFA into FFA, confirming their activity.

This is the first proof-of-principle of such a periplasmic FDCA production route, but it currently still suffers from modest efficiency. A cultivation over about 5 days yielded around 3 mM FDCA **(****Fig. 8****).** As aspects such as selection and concentration of the lanthanide salt, the choice of the carbon source, and expression strength of *pedH* could be ruled out as limiting factors for efficient conversion **(****Figs. 9, 10****,** **11****),** further improvement of the enzyme is probably necessary to render it competitive with HmfH or other GMC-type oxidoreductases. Nonetheless, the first observation of *in vivo* activity of PedH^{F412V/F459W/L560R/W561A} to HMFA opens up new possibilities for whole-cell bioproduction of FDCA in the periplasm. Amongst others, it gives access to alternative engineering strategies like growth-coupled enzyme evolution (29).

### References

1 Bachmann, M.; Zibunas, C.; Hartmann, J.; Tulus, V.; Suh, S.; Guillén-Gosálbez, G.; Bardow, A. Towars circular plastics within planetary boundaries. Nat Sustain 2023, 6 (5), 599-610. DOI: 10.1038/s41893-022-01054-9. Rosenboom, J. G.; Langer, R.; Traverso, G. Bioplastics for a circular economy. Nat Rev Mater 2022, 7 (2), 117-137. DOI: 10.1038/s41578-021-00407-8.
2 Zhu, Y. Q.; Romain, C.; Williams, C. K. Sustainable polymers from renewable resources. Nature 2016, 540 (7633), 354-362. DOI: 10.1038/nature21001.
3 Salusjärvi, L.; Havukainen, S.; Koivistoinen, O.; Toivari, M. Biotechnological production of glycolic acid and ethylene glycol: current state and perspectives. Appl Microbiol Biot 2019, 103 (6), 2525-2535. DOI: 10.1007/s00253-019-09640-2.
   Wong, M. K.; Lock, S. S. M.; Chan, Y. H.; Yeoh, S. J.; Tan, I. S. Towards sustainable production of bio-based ethylene glycol: Progress, perspective and challenges in catalytic conversion and purification. Chem Eng J 2023, 468. DOI: 10.1016/j.cej.2023.143699.
4 Loos, K.; Zhang, R.; Pereira, I.; Agostinho, B.; Hu, H.; Maniar, D.; Sbirrazzuoli, N.; Silvestre, A. J. D.; Guigo, N.; Sousa, A. F. A Perspective on PEF Synthesis, Properties, and End-Life. Front Chem 2020, 8, 585. DOI: 10.3389/fchem.2020.00585.
5 Bozell, J. J.; Petersen, G. R. Technology development for the production of biobased products from biorefinery carbohydrates-the US Department of Energy's "Top 10" revisited. Green Chemistry 2010, 12 (4). DOI: 10.1039/b922014c.
6 Totaro, G.; Sisti, L.; Marchese, P.; Colonna, M.; Romano, A.; Gioia, C.; Vannini, M.; Celli, A. Current Advances in the Sustainable Conversion of 5-Hydroxymethylfurfural into 2,5-Furandicarboxylic Acid. Chemsuschem 2022, 15 (13). DOI: 10.1002/cssc.202200501.
7 Delidovich, I.; Hausoul, P. J. C.; Deng, L.; Pfützenreuter, R.; Rose, M.; Palkovits, R. Alternative Monomers Based on Lignocellulose and Their Use for Polymer Production. Chem Rev 2016, 116 (3), 1540-1599. DOI: 10.1021/acs.chemrev.5b00354.
   Bell, E. L.; Finnigan, W.; France, S. P.; Green, A. P.; Hayes, M. A.; Hepworth, L. J.; Lovelock, S. L.; Niikura, H.; Osuna, S.; Romero, E.; et al. Biocatalysis. Nat Rev Method Prime 2021, 1 (1). DOI: 10.1038/s43586-021-00044-z.
8 Hsu, C. T.; Kuo, Y. C.; Liu, Y. C.; Tsai, S. L. Green conversion of 5-hydroxymethylfurfural to furan-2,5-dicarboxylic acid by heterogeneous expression of 5-hydroxymethylfurfural oxidase in Pseudomonas putida S12. Microb Biotechnol 2020, 13 (4), 1094-1102. DOI: 10.1111/1751-7915.13564. Yuan, H. B.; Li, J. H.; Shin, H. D.; Du, G. C.; Chen, J.; Shi, Z. P.; Liu, L. Improved production of 2,5-furandicarboxylic acid by overexpression of 5-hydroxymethylfurfural oxidase and 5-hydroxymethylfurfural/furfural oxidoreductase in Raoultella ornithinolytica BF60. Bioresource Technol 2018, 247, 1184-1188. DOI: 10.1016/j.biortech.2017.08.166. Sayed, M.; Gaber, Y.; Junghus, F.; Martin, E. V.; Pyo, S. H.; Hatti-Kaul, R. Oxidation of 5-hydroxymethylfurfural with a novel aryl alcohol oxidase from Mycobacterium sp. MS1601. Microbial Biotechnology 2022, 15 (8), 2176-2190. DOI: 10.1111/1751-7915.14052. McKenna, S. M.; Mines, P.; Law, P.; Kovacs-Schreiner, K.; Birmingham, W. R.; Turner, N. J.; Leimkuhler, S.; Carnell, A. J. The continuous oxidation of HMF to FDCA and the immobilisation and stabilisation of periplasmic aldehyde oxidase (PaoABC). Green Chemistry 2017, 19 (19), 4660-4665. DOI: 10.1039/c7gc01696d.
   Milic, M.; Byström, E.; de Maria, P. D.; Kara, S. Enzymatic Cascade for the Synthesis of 2,5-Furandicarboxylic Acid in Biphasic and Microaqueous Conditions: 'Media-Agnostic' Biocatalysts for Biorefineries. Chemsuschem 2022, 15 (9). DOI: 10.1002/cssc.202102704.
9 Pham, N. N.; Chen, C. Y.; Li, H.; Nguyen, M. T. T.; Nguyen, P. K. P.; Tsai, S. L.; Chou, J. Y.; Ramli, T. C.; Hu, Y. C. Engineering Stable Pseudomonas putida S12 by CRISPR for 2,5-Furandicarboxylic Acid (FDCA) Production. Acs Synth Biol 2020, 9 (5), 1138-1149. DOI: 10.1021/acssynbio.0c00006.
10 Dijkman, W. P.; Groothuis, D. E.; Fraaije, M. W. Enzyme-Catalyzed Oxidation of 5-Hydroxymethylfurfural to Furan-2,5-dicarboxylic Acid. Angew Chem Int Edit 2014, 53 (25), 6515-6518. DOI: 10.1002/anie.201402904.
11 Dijkman, W. P.; Binda, C.; Fraaije, M. W.; Mattevi, A. Structure-Based Enzyme Tailoring of 5-Hydroxymethylfurfural Oxidase. Acs Catal 2015, 5 (3), 1833-1839. DOI: 10.1021/acscatal.5b00031.
12 de Carvalho, C. C. C. R. Whole cell biocatalysts: essential workers from Nature to the industry. Microbial Biotechnology 2017, 10 (2), 250-263. DOI: 10.1111/1751-7915.12363.
13 LoPachin, R. M.; Gavin, T. Molecular mechanisms of aldehyde toxicity: a chemical perspective. Chem Res Toxicol 2014, 27 (7), 1081-1091. DOI: 10.1021/tx5001046.
   Bitzenhofer, N. L.; Kruse, L.; Thies, S.; Wynands, B.; Lechtenberg, T.; Rönitz, J.; Kozaeva, E.; Wirth, N. T.; Eberlein, C.; Jaeger, K. E.; et al. Towards robust Pseudomonas cell factories to harbour novel biosynthetic pathways. Essays Biochem 2021, 65 (2), 319-336. DOI: 10.1042/EBC20200173.
14 Lechtenberg, T.; Wynands, B.; Wierckx, N. Engineering 5-hydroxymethylfurfural (HMF) oxidation in Pseudomonas boosts tolerance and accelerates 2,5-furandicarboxylic acid (FDCA) production. Metabolic Engineering 2024, 81, 262-272. DOI: https://doi.org/10.1016/j.ymben.2023.12.010.
15 Koopman, F.; Wierckx, N.; de Winde, J. H.; Ruijssenaars, H. J. Efficient whole-cell biotransformation of 5-(hydroxymethyl)furfural into FDCA, 2,5-furandicarboxylic acid. Bioresource Technol 2010, 101 (16), 6291-6296. DOI: 10.1016/j.biortech.2010.03.050.
16 Dijkman, W. P.; Fraaije, M. W. Discovery and Characterization of a 5-Hydroxymethylfurfural Oxidase from Methylovorus sp Strain MP688. Appl Environ Microb 2014, 80 (3), 1082-1090. DOI: 10.1128/Aem.03740-13.
17 Imlay, J. A. Pathways of oxidative damage. Annu Rev Microbiol 2003, 57, 395-418. DOI: 10.1146/annurev.micro.57.030502.090938. Zhao, X. L.; Drlica, K. Reactive oxygen species and the bacterial response to lethal stress. Curr Opin Microbiol 2014, 21, 1-6. DOI: 10.1016/j.mib.2014.06.008.
18 Kim, J.; Park, W. Oxidative stress response in Pseudomonas putida. Appl Microbiol Biot 2014, 98 (16), 6933-6946. DOI: 10.1007/s00253-014-5883-4. Nikel, P. I.; Fuhrer, T.; Chavarría, M.; Sánchez-Pascuala, A.; Sauer, U.; de Lorenzo, V. Reconfiguration of metabolic fluxes in as a response to sub-lethal oxidative stress. Isme J 2021, 15 (6), 1751-1766. DOI: 10.1038/s41396-020-00884-9.
19 Baldwin, C. V. F.; Woodley, J. M. On oxygen limitation in a whole cell biocatalytic Baeyer-Villiger oxidation process. Biotechnol Bioeng 2006, 95 (3), 362-369. DOI: 10.1002/bit.20869.
20 Lin, B.; Tao, Y. Whole-cell biocatalysts by design. Microb Cell Fact 2017, 16 (1), 106. DOI: 10.1186/s12934-017-0724-7.
21 Wehrmann, M.; Billard, P.; Martin-Meriadec, A.; Zegeye, A.; Klebensberger, J. Functional Role of Lanthanides in Enzymatic Activity and Transcriptional Regulation of Pyrroloquinoline Quinone-Dependent Alcohol Dehydrogenases in Pseudomonas putida KT2440. Mbio 2017, 8 (3). DOI: 10.1128/mBio.00570-17.
22 Wehrmann, M.; Berthelot, C.; Billard, P.; Klebensberger, J. Rare Earth Element (REE)-Dependent Growth of Pseudomonas putida KT2440 Relies on the ABC-Transporter PedA1A2BC and Is Influenced by Iron Availability. Front Microbiol 2019, 10. DOI: 10.3389/fmicb.2019.02494.
23 Zheng, Y.; Huang, J.; Zhao, F.; Chistoserdova, L. Physiological Effect of XoxG(4) on Lanthanide-Dependent Methanotrophy. Mbio 2018, 9 (2). DOI: 10.1128/mBio.02430-17.
   Versantvoort, W.; Pol, A.; Daumann, L. J.; Larrabee, J. A.; Strayer, A. H.; Jetten, M. S. M.; van Niftrik, L.; Reimann, J.; Op den Camp, H. J. M. Characterization of a novel cytochrome as the electron acceptor of XoxF-MDH in the thermoacidophilic methanotroph Methylacidiphilum fumariolicum SolV. Bba-Proteins Proteom 2019, 1867 (6), 595-603. DOI: 10.1016/j.bbapap.2019.04.001. Hardy, G. P. M. A.; Demattos, M. J. T.; Neijssel, O. M. Energy-Conservation by pyrroloquinoline quinol-linked xylose oxidation in Pseudomonas putida NCTC 10936 during carbon-limited growth in chemostat culture. Fems Microbiol Lett 1993, 107 (1), 107-110. DOI: DOI 10.1111/j.1574-6968.1993.tb06012.x.
24 Wehrmann, M.; Elsayed, E. M.; Köbbing, S.; Bendz, L.; Lepak, A.; Schwabe, J.; Wierckx, N.; Bange, G.; Klebensberger, J. Engineered PQQ-dependent alcohol dehydrogenase for the oxidation of 5-(hydroxymethyl)furoic acid. Acs Catal 2020. DOI: 10.1021/acscatal.0c01789.
25 Hartmans, S., Smits, J. P., van der Werf, M. J., Volkering, F. & de Bont, J. A. Metabolism of Styrene Oxide and 2-Phenylethanol in the Styrene-Degrading Xanthobacter Strain 124X. Appl. Environ. Microbiol. 55, 2850-2855 (1989).
26 Wynands, B. et al. Metabolic engineering of Pseudomonas taiwanensis VLB120 with minimal genomic modifications for high-yield phenol production. Metab. Eng. 47, 121-133 (2018).
27 Martinez-Garcia, E. & de Lorenzo, V. Engineering multiple genomic deletions in Gram-negative bacteria: analysis of the multi-resistant antibiotic profile of Pseudomonas putida KT2440. Environ. Microbiol. 13, 2702-2716 (2011).
28 Chomczynski, P. & Rymaszewski, M. Alkaline polyethylene glycol-based method for direct PCR from bacteria, eukaryotic tissue samples, and whole blood. Biotechniques 40, 454,456,458 (2006).
29 Elsayed, E. M. A. G. Studies on the rare earth elements-and calcium-dependent quinoproteins in Pseudomonas putida KT2440.
30 Anthony, C. & Zatman, L. J. The microbial oxidation of methanol. 2. The methanol-oxidizing enzyme of Pseudomonas sp. M27. Biochem. J. 92, 614 (1964).
31 Chattopadhyay, A., Förster-Fromme, K. & Jendrossek, D. PQQ-dependent alcohol dehydrogenase (QEDH) of Pseudomonas aeruginosa is involved in catabolism of acyclic terpenes. J. Basic Microbiol. 50, 119-124 (2010).
32 Emsley, P. & Cowtan, K. Coot: model-building tools for molecular graphics. Acta Crystallogr. Sect. D Biol. Crystallogr. 60, 2126-2132 (2004).
33 Adams, P. D. et al. PHENIX: a comprehensive Python-based system for macromolecular structure solution. Acta Crystallogr. Sect. D Biol. Crystallogr. 66, 213-221 (2010).
34 Ditta, G., Stanfield, S., Corbin, D. & Helinski, D. R. Broad host range DNA cloning system for gram-negative bacteria: construction of a gene bank of Rhizobium meliloti. Proc. Natl. Acad. Sci. 77, 7347-7351 (1980).
35 Bagdasarian, M. al et al. Specific-purpose plasmid cloning vectors II. Broad host range, high copy number, RSF 1010-derived vectors, and a host-vector system for gene cloning in Pseudomonas. Gene 16, 237-247 (1981).
36 Figurski, D. H. & Helinski, D. R. Replication of an origin-containing derivative of plasmid RK2 dependent on a plasmid function provided in trans. Proc. Natl. Acad. Sci. 76, 1648-1652 (1979).
37 Koopman, F., Wierckx, N., de Winde, J. H. & Ruijssenaars, H. J. Identification and characterization of the furfural and 5-(hydroxymethyl) furfural degradation pathways of Cupriavidus basilensis HMF14. Proc. Natl. Acad. Sci. 107, 4919-4924 (2010).
38 Salis, H. M., Mirsky, E. A. & Voigt, C. A. Automated design of synthetic ribosome binding sites to control protein expression. Nat. Biotechnol. 27, 946-950 (2009).
39 Li, W.-J. et al. Unraveling 1,4-Butanediol Metabolism in Pseudomonas putida KT2440. Front. Microbiol. 11, 382 (2020).

## Claims

1. A alcohol oxidoreductase polypeptide converting 5-hydroxymethyl-2-furoic acid (HMFA) to 5-formyl-2-furoic acid (FFA) with a Km value of less than 15 mM selected from the group consisting of
(a) a polypeptide having the amino acid sequence shown in SEQ ID NO: 1, wherein in said polypeptide amino acids at positions corresponding to positions F412, F459 and W561 are V, W or R and A, respectively; optionally having the amino acid sequence shown in SEQ ID NO: 3, 4, 5 or 6;
(b) a polypeptide fragment of the polypeptide of (a), wherein in said polypeptide fragment amino acids at positions corresponding to positions F412, F459 and W561 are V, W or R and A, respectively;
(c) a polypeptide having an amino acid sequence which is at least 70% identical to the amino acid sequence of the polypeptide of (a) or (b), wherein in said polypeptide amino acids at positions corresponding to positions F412, F459 and W561 are V, W or R and A, respectively;
(d) a polypeptide having the amino acid sequence shown in SEQ ID NO: 1, wherein in said polypeptide amino acids at positions corresponding to positions F412, L560 and W561 are V, R or W and A, respectively; optionally having the amino acid sequence shown in SEQ ID NO: 7, 8, 9, or 10;
(e) a polypeptide fragment of the polypeptide of (d), wherein in said polypeptide fragment amino acids at positions corresponding to positions F412, L560 and W561 are V, R or W and A, respectively;
(f) a polypeptide having an amino acid sequence which is at least 70% identical to the amino acid sequence of the polypeptide of (d) or (e), wherein in said polypeptide amino acids at positions corresponding to positions F412, L560 and W561 are V, R or W and A, respectively;
(g) a polypeptide having the amino acid sequence shown in SEQ ID NO: 1, wherein in said polypeptide amino acids at positions corresponding to positions F412, L455 and W561 are V, W or R and A, respectively; optionally having the amino acid sequence shown in SEQ ID NO: 11, 12, 13, or 14;
(h) a polypeptide fragment of the polypeptide of (g), wherein in said polypeptide fragment amino acids at positions corresponding to positions F412, L455 and W561 are V, W or R and A, respectively;
(i) a polypeptide having an amino acid sequence which is at least 70% identical to the amino acid sequence of the polypeptide of (g) or (h), wherein in said polypeptide amino acids at positions corresponding to positions F412, L455 and W561 are V, W or R and A, respectively;
wherein said alcohol oxidoreductase is a alcohol dehydrogenase, preferably the alcohol dehydrogenase is a co factor dependent alcohol dehydrogenase, wherein the co factor is a co factor located in the periplasm, preferably the co factor is cytochrome C, pyrroloquinoline quinone (PQQ), or a quinone similar to PQQ, more preferably the co factor dependent alcohol dehydrogenase is the pyrroloquinoline quinone (PQQ) dependent alcohol dehydrogenase, most preferably the co factor dependent alcohol dehydrogenase is PedE or PedH, exceptionally preferred is PedH.

2. The polypeptide of any one of claims 1(a) to (f), wherein in said polypeptide or polypeptide fragment amino acids at positions corresponding to positions F412, F459, L560 and W561 are V, W or R, R or W and A, respectively, said polypeptide or polypeptide fragment further converting FFA to 2,5 furandicarboxylic acid (FDCA), optional said polypeptide is selected from the group consisting of
(a) a polypeptide having the amino acid sequence shown in SEQ ID NO: 1, wherein in said polypeptide amino acids at positions corresponding to positions F412, F459, L560 and W561 are V, W or R, R or W and A, respectively; optionally having the amino acid sequence shown in SEQ ID NO: 15, 16, 17, 18, 19, 20, 21, or 22;
(b) a polypeptide fragment of the polypeptide of (a), wherein in said polypeptide fragment amino acids at positions corresponding to positions F412, F459, L560 and W561 are V, W or R, R or W, and A, respectively;
(c) a polypeptide having an amino acid sequence which is at least 70% identical to the amino acid sequence of the polypeptide of (a) or (b), wherein in said polypeptide amino acids at positions corresponding to positions F412, F459, L560 and W561 are V, W or R, R or W and A, respectively.

3. The polypeptide of any one of claims 1(g) to (i), wherein in said polypeptide or polypeptide fragment amino acids at positions corresponding to positions F412, L455, L560 and W561 are V, W or R, R or W and A, respectively, said polypeptide or polypeptide fragment further converting FFA to FDCA, optional said polypeptide is selected from the group consisting of
(a) a polypeptide having the amino acid sequence shown in SEQ ID NO: 1, wherein in said polypeptide amino acids at positions corresponding to positions F412, L455, L560 and W561 are V, W or R, R or W and A, respectively; optionally having the amino acid sequence shown in SEQ ID NO: 23, 24, 25, 26, 27, 28, 29, and 30;
(b) a polypeptide fragment of the polypeptide of (a), wherein in said polypeptide fragment amino acids at positions corresponding to positions F412, L455, L560 and W561 are V, W or R, R or W, and A, respectively;
(c) a polypeptide having an amino acid sequence which is at least 70% identical to the amino acid sequence of the polypeptide of (a) or b), wherein in said polypeptide amino acids at positions corresponding to positions F412, L455, L560 and W561 are V, W or R, R or W and A, respectively.

4. A polynucleotide encoding the polypeptide of any one of claims 1 to 3, optionally the polynucleotide has the nucleotide sequence shown in SEQ ID NO: 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 67, 68, 69, 70, 71, 72, 73, 74, 75, 76, 77, 78, 79, 80, 81, 82, 83, 84, 85, 86, 87, 88, 89, 90, 91, 92 or 93.

5. A vector comprising the polynucleotide of claim 4, optionally said vector is an expression vector.

6. A host cell comprising the polynucleotide of claim 4 or the vector of claim 5.

7. The host cell of claim 6, wherein said host cell is a gram-negative bacterial host cell, optionally said gram-negative bacterial host cell is a Pseudomonas species, preferably Pseudomonas putida and further optionally said host cell lacks a functional hmft1 gene and /or a functional hmfH gene.

8. A method for converting HMFA to FFA, comprising culturing the host cell of any of claims 6 or 7 expressing the polypeptide as defined in any one of claims 1 to 3, optionally FFA is further converted to FDCA by the host cell of any one of claims 6 or 7 expressing the polypeptide as defined in any one of claims 2 or 3, further optionally wherein said host cell is further capable of converting 5-(hydroxymethyl)furfural (HMF) to HMFA; preferably the conversion(s) takes place in the periplasm of said host cell.

9. A method for the production of FDCA, comprising culturing a host cell expressing a polypeptide as defined in any one of claims 2 or 3, preferably the conversion takes place in the periplasm of said host cell.

10. Use of the host cell of any one of claims 6 or 7 for converting HMFA to FFA, said host cell expressing the polypeptide as defined in any one of claims 1 to 3, optionally said host cell is further capable of converting HMF to HMFA.

11. Use of the host cell of any one of claims 6 or 7 for converting FFA to FDCA, said host cell expressing the polypeptide as defined in any one of claims 2 or 3, optionally said host cell is further capable of converting HMF to HMFA.

12. Use of the host cell of any one of claims 6 or 7 for expressing the polypeptide as defined in any one of claims 2 or 3 for the production of FDCA.

13. Crystal structure of the polypeptide having the amino acid shown in SEQ ID NO: 15 characterized as having (a) a space group of P4₃2₁2 and (b) unit cell dimensions of a = 105.23 Å, b = 105.23 Å and c = 186.52 Å, α, β, γ = 90°.

14. Use of the crystal structure as defined in claim 13 for obtaining atomic spatial relationship data.

15. Use of the crystal structure of claim 13 for identifying amino acids which aid in the conversion of FFA to FDCA
